(19) Europäisches Patentamt
European Patent Office
Office européen des brevets

(11) EP 3 301 098 B1

(12) **EUROPEAN PATENT SPECIFICATION**

(45) Date of publication and mention of the grant of the patent:
**19.02.2020 Bulletin 2020/08**

(21) Application number: **17195336.7**

(22) Date of filing: **19.12.2013**

(51) Int Cl.:
*C07F 7/08* (2006.01) *C07C 13/465* (2006.01)
*C07F 17/00* (2006.01) *C08F 10/06* (2006.01)
*C08F 4/6592* (2006.01) *C07C 1/32* (2006.01)
*C07C 25/18* (2006.01)

(54) **PROCESS FOR THE PREPARATION OF A LIGAND FOR A CATALYST**

VERFAHREN ZUR HERSTELLUNG EINES LIGANDEN FÜR EIN KATALYSATOR

PROCÉDÉ DE PREPARATION D'UN LIGAND POUR UN CATALYSEUR

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **21.12.2012 EP 12199251**

(43) Date of publication of application:
**04.04.2018 Bulletin 2018/14**

(62) Document number(s) of the earlier application(s) in accordance with Art. 76 EPC:
**13811231.3 / 2 935 296**

(73) Proprietor: **Borealis AG**
**1220 Vienna (AT)**

(72) Inventors:
- **RESCONI, Luigi**
  **4501 Neuhofen an der Krems (AT)**
- **VIRKKUNEN, Ville**
  **FI-00790 Helsinki (FI)**
- **AJELLAL, Noureddine**
  **00970 Helsinki (FI)**
- **CASTRO, Pascal**
  **FI-00150 Helsinki (FI)**
- **IZMER, Vyatcheslav**
  **117186 Moscow (RU)**
- **KONONOVICH, Dmitry**
  **119234 Moscow (RU)**
- **VOSKOBOYNIKOV, Alexander**
  **129515 Moscow (RU)**

(74) Representative: **Dehns**
**St. Bride's House**
**10 Salisbury Square**
**London EC4Y 8JD (GB)**

(56) References cited:
**EP-A1- 2 935 295** **EP-A2- 2 532 687**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 3 301 098 B1

## Description

## Field of invention

This invention relates to a process for preparing ligands suitable for use in bisindenyl catalysts, in particular, solid particulate racemic symmetrical metallocene catalysts containing such bisindenyl ligands. Bisindenyl metallocene catalysts containing the ligands can be used for the production of polypropylene at excellent catalyst activities to give polypropylene with high molecular weight, and high melting point even at industrially relevant polymerization temperatures.

## Background of Invention

Metallocene catalysts have been used to manufacture polyolefins for many years. Countless academic and patent publications describe the use of these catalysts in olefin polymerisation. Metallocenes are now used industrially and polyethylenes and polypropylenes in particular are often produced using cyclopentadienyl based catalyst systems with different substitution patterns.

The two most important physical properties of isotactic polypropylene (iPP) are its average molecular weight and its melting point (Tm), the latter being mostly determined by the degree of stereoregularity (isotacticity) of the polypropylene chains.

The Ziegler-Natta catalyst systems known in the literature can produce iPP with high molecular weights together with moderate to high isotacticities and melting temperatures (Tm). The Tm (measured by standard DSC methods) of non-nucleated iPPs are in the range of 160 to 165 °C.

In the case of metallocenes, there are very few examples which can produce iPP having both very high molecular weights and high melting points. For example rac-Et(2,4,7-$Me_3$Ind)$_2$Zr$Cl_2$ can produce isotactic polypropylene with a molecular weight of 1,900,000 g/mol and a melting point of 168 °C (Deng Macromolecules, 1996, 29, 6371).

In order to achieve such high values, a polymerization temperature of -30 °C is necessary. When the polymerization temperature is increased to 30 °C, the melting point of the resulting polypropylene decreases to 158 °C. A polymerization temperature of -30 °C is however, far too low for polypropylene manufacturing in commercial plants, which need to be operated above 60 °C. When used at industrially useful polymerization temperatures, this same metallocene yields low molecular weight polypropylenes with relatively low melting point. For example at 70 °C, rac-Et(2,4,7-$Me_3$Ind)$_2$Zr$Cl_2$ /MAO yields a polypropylene of molecular weight of only 30,600 with a melting point of only 145 °C (EP537686).

Higher melting point polymers can be made using dimethylsilyl bis (2-methyl-4-(1-naphthyl)indenyl Zr$Cl_2$ (Spaleck Organometallics, 1994, 13, 954). EP-A-1070729 exemplifies a variety of 2,4-disubstituted metallocenes although melting points are not discussed.

A melting point of 162 °C and of 165 °C have also been reported for the simple $Me_2$Si(2-Me-4-Ph-Indenyl)$_2$ metallocenes, (J. Ewen et al. Macromol. Rapid Commun. 1998, 19, 71).

The metallocenes above have been used in non-supported form however, i.e. typically in solution polymerisation. When used in solid form and in particular when used on a catalyst support such as silica, the melting point of the polymer reduces.

Some supported metallocenes have however been found to give high melting points. In US7,405,261, rac-Et[2,7-$Me_2$-4-(4-tBuPh)Ind]$_2$Zr$Cl_2$ is reported to produce iPP with a melting point of 156 °C, by polymerizing liquid propylene at 65 °C.

WO2009/054831 describes zirconocenes with a 2-methyl-4,7-aryl substitution pattern, such as rac-$Me_2$Si[2-Me-4,7-(4-tBuPh)$_2$Ind]$_2$Zr$Cl_2$. The melting points of the homopolymers are still quite low, being in all cases below 150 °C despite the relatively low polymerization temperature of 65 °C.

The present applicant has developed an alternative to conventional inorganic supports. In WO03/051934, the inventors proposed an alternative form of catalyst which is provided in solid form but does not require a conventional external carrier material such as silica. The invention is based on the finding that a homogeneous catalyst system containing an organometallic compound of a transition metal can be converted, in a controlled way, to solid, uniform catalyst particles by first forming a liquid/liquid emulsion system, which comprises as the dispersed phase, said solution of the homogeneous catalyst system, and as the continuous phase a solvent immiscible therewith, and then solidifying said dispersed droplets to form solid particles comprising the said catalyst.

The invention described in WO03/051934 enabled the formation of solid spherical catalyst particles of said organotransition metal catalyst without using e.g. external porous carrier particles, such as silica, normally required in the art. Thus, problems relating to catalyst silica residues can be solved by this type of catalyst. Further, it could be seen that catalyst particles having improved morphology, will give, due to the replica effect, polymer particles having improved morphology as well.

Some supported metallocene or metallocenes in solid form made using the techniques of WO03/051934 are also known. Metallocenes with a 3,5-di-tert-butylphenyl substituent on the 4-position of indene are known. WO02/02576 describes conventionally supported metallocenes such as rac-$Me_2Si[2\text{-}Me\text{-}4\text{-}(3,5\text{-}tBu_2Ph)Ind]_2ZrCl_2$. These metallocene catalysts, activated with MAO or a borate, on a silica support, at a polymerisation temperature of 60 or 70 °C, give iPP with Tm between 156 and 159 °C.

The metallocene rac-9-silafluorenyl-9,9-$[2\text{-}Me\text{-}4\text{-}(3,5\text{-}tBu_2Ph)Ind]_2ZrCl_2$ also gives high melting point iPP and are described in WO02/02575.

In EP2532687, a catalyst based on rac-Me2Si$[2\text{-}Me\text{-}4\text{-}(3,5\text{-}tBu_2Ph)\text{-}7\text{-}OMe\text{-}Ind]_2ZrCl_2$/MAO is described with relatively low activity.

In general however, metallocene catalysts, when used under industrially relevant polymerization conditions, produce iPP having melting points which are lower than the melting points of Ziegler Natta iPP, and even the best metallocene catalysts produce iPP with melting points of less than 160 °C. In addition, few metallocene catalysts can produce iPP having both high melting point and high molecular weight at polymerisation temperatures above 60 °C with good catalyst activity.

In order to overcome this inherent limitation of metallocene catalysts, and in order to produce polypropylenes having both high melting points and high molecular weights with good activity, we have developed a new family of catalysts comprising substituted bis-indenyl complexes.

We preferably employ these complexes in solid particulate, yet unsupported, form to make a new family of catalysts with interesting properties. These metallocenes have been found to produce isotactic polypropylenes with surprisingly high melting points and very high molecular weights at good catalyst activities.

The catalysts comprise a bridged bisindenyl metallocene complex with a substituted aryl group at the 4-position of an indenyl ligand, a non hydrogen substituent at the 2-position of the ring and a substituent at the 6-position of the ring. The 3, 5 and 7 positions are unsubstituted.

One of the limitations of metallocene catalysts for polypropylene is the relative low melting of the homopolymer. Several families of bridged bisindenyl metallocene catalysts with the proper substitution pattern have been developed to produce high molecular weight, highly isotactic polypropylene. However, often the increased melting point is obtained at the expense of activity, or catalyst cost, or both.

The present inventors sought a new catalyst system capable of producing, *inter alia,* isotactic polypropylene with high melting points, e.g. 152 °C or more, high isotacticity and high molecular weights without compromising catalyst activity at commercially relevant temperatures. In particular, catalysts should offer high activities and high molecular weight at high melting points. The inventors have devised a new process for making ligands for use in such a catalyst.

**Summary of Invention**

Thus, viewed from one aspect the invention provides a process for the preparation of a compound of formula (A'):

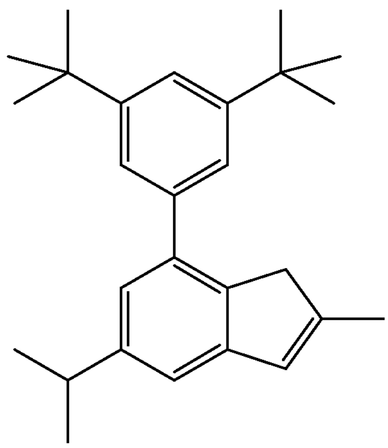

(A')

comprising at least the steps of:

a) reacting a compound of formula (B")

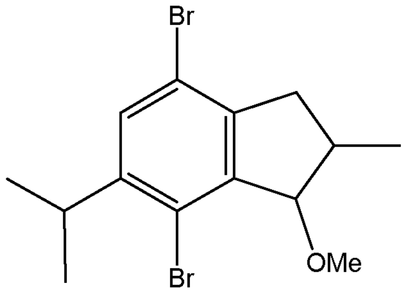

(B'')

with nBuLi to obtain a compound of formula (C")

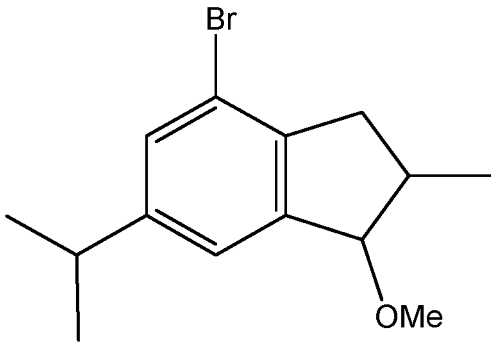

(C'')

b) reacting the compound of formula (C") with a compound (D')

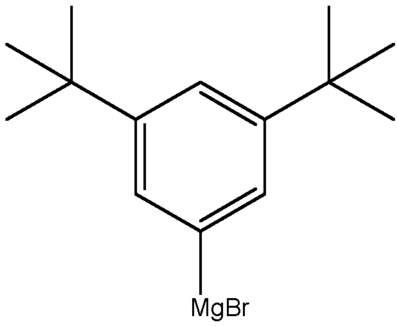

(D')

in the presence of $NiCl_2(PPh_3)$IPr wherein IPr represents 1,3-bis(2,6-diisopropylphenyl)imidazolidin-2-ylidene to form compound (E");

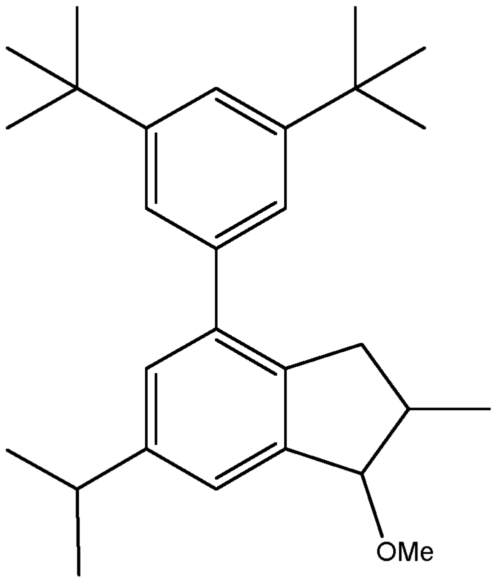

(E'')

and reducing compound (E") in the presence of TsOH.

**Definitions**

Throughout the description the following definitions are employed.

By free from an external carrier is meant that the catalyst does not contain an external support, such as an inorganic support, for example, silica or alumina, or an organic polymeric support material, onto which catalyst components are loaded.

The term symmetrical is used herein to imply that both ligands are identical, i.e. there is C2 symmetry.

**Detailed Description of invention**

A compound of formula (A') can be used to form bis indenyl complexes. Complexes can comprise zirconium or hafnium as the metal centre. Highly preferred complexes are:
*Rac*-dimethylsilanediylbis[2-methyl-4-(3,5-di-*tert*-butylphenyl)-6-isopropyl-inden-1-yl]zirconium dichloride (I-mc3)
or their Hf analogues.

**Synthesis**

The present inventors have devised a new procedure for the formation of formula (A') .

The new procedure is shown in Scheme 2:

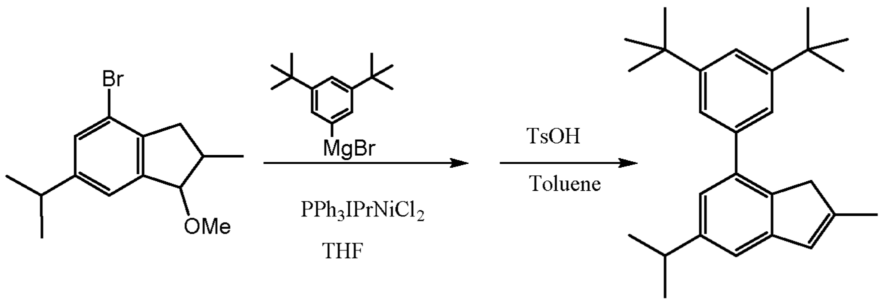


Scheme 2

The first step of this "one-pot" sequence is a Ni-catalyzed Kumada coupling, where the bromine atom in the indene 6-membered ring gets substituted with a di(tert-butyl)phenyl moiety). In order to obtain an indene i.e. formally eliminate MeOH and form a carbon-carbon double bond, an acid-catalyzed elimination using a dean-stark apparatus is

used. TsOH can be used as an acid catalyst and toluene can be employed to remove water/methanol azeotropically.

**Cocatalyst**

To form an active catalytic species it is normally necessary to employ a cocatalyst as is well known in the art. Cocatalysts comprising an organometallic compound of Group 13 metal, like organoaluminium compounds used to activate metallocene catalysts are suitable for use in this invention.

Alternatively, however, the catalysts may be used with other cocatalysts, e.g. boron compounds. It will be appreciated by the skilled man that where boron based cocatalysts are employed, it is normal to preactivate the complex by reaction thereof with an aluminium alkyl compound, such as TIBA. This procedure is well known and any suitable aluminium alkyl, e.g. $Al(C_{1-6}\text{-alkyl})_3$. can be used.

The use of aluminoxanes, especially MAO, is highly preferred.

**Manufacture**

The metallocene complex can be used in combination with a suitable cocatalyst as a catalyst for the polymerization of olefins, e.g. in a solvent such as toluene or an aliphatic hydrocarbon, (i.e. for polymerization in solution), as it is well known in the art. Preferably, polymerization of olefins, especially propylene, takes place in the condensed phase or in gas phase.

The catalyst is preferably in solid particulate form but unsupported, i.e. no external carrier is used. In order to provide the catalyst in solid form but without using an external carrier, it is preferred if a liquid liquid emulsion system is used. The process involves forming dispersing catalyst components (i) and (ii) in a solvent, and solidifying said dispersed droplets to form solid particles.

In particular, the method involves preparing a solution of one or more catalyst components; dispersing said solution in an solvent to form an emulsion in which said one or more catalyst components are present in the droplets of the dispersed phase; immobilising the catalyst components in the dispersed droplets, in the absence of an external particulate porous support, to form solid particles comprising the said catalyst, and optionally recovering said particles.

This process enables the manufacture of active catalyst particles with improved morphology, e.g. with a predetermined spherical shape and particle size and without using any added external porous support material, such as an inorganic oxide, e.g. silica. Also desirable surface properties can be obtained.

By the term "preparing a solution of one or more catalyst components" is meant that the catalyst forming compounds may be combined in one solution which is dispersed to the immiscible solvent, or, alternatively, at least two separate catalyst solutions for each part of the catalyst forming compounds may be prepared, which are then dispersed successively to the solvent.

In a preferred method for forming the catalyst at least two separate solutions for each or part of said catalyst may be prepared, which are then dispersed successively to the immiscible solvent.

More preferably, a solution of the complex comprising the transition metal compound and the cocatalyst is combined with the solvent to form an emulsion wherein that inert solvent forms the continuous liquid phase and the solution comprising the catalyst components forms the dispersed phase (discontinuous phase) in the form of dispersed droplets. The droplets are then solidified to form solid catalyst particles, and the solid particles are separated from the liquid and optionally washed and/or dried. The solvent forming the continuous phase may be immiscible to the catalyst solution at least at the conditions (e.g. temperatures) used during the dispersing step.

The term "immiscible with the catalyst solution" means that the solvent (continuous phase) is fully immiscible or partly immiscible i.e. not fully miscible with the dispersed phase solution.

Preferably said solvent is inert in relation to the compounds of the catalyst system to be produced. Full disclosure of the necessary process can be found in WO03/051934 which is herein incorporated by reference.

The inert solvent must be chemically inert at least at the conditions (e.g. temperature) used during the dispersing step. Preferably, the solvent of said continuous phase does not contain dissolved therein any significant amounts of catalyst forming compounds. Thus, the solid particles of the catalyst are formed in the droplets from the compounds which originate from the dispersed phase (i.e. are provided to the emulsion in a solution dispersed into the continuous phase).

The terms "immobilisation" and "solidification" are used herein interchangeably for the same purpose, i.e. for forming free flowing solid catalyst particles in the absence of an external porous particulate carrier, such as silica. The solidification happens thus within the droplets. Said step can be effected in various ways as disclosed in said WO03/051934 Preferably solidification is caused by an external stimulus to the emulsion system such as a temperature change to cause the solidification. Thus in said step the catalyst component(s) remain "fixed" within the formed solid particles. It is also possible that one or more of the catalyst components may take part in the solidification/immobilisation reaction.

Accordingly, solid, compositionally uniform particles having a predetermined particle size range can be obtained.
Furthermore, the particle size of the catalyst particles of the invention can be controlled by the size of the droplets in the solution, and spherical particles with a uniform particle size distribution can be obtained.
The invention is also industrially advantageous, since it enables the preparation of the solid particles to be carried out as a one-pot procedure. Continuous or semicontinuous processes are also possible for producing the catalyst.

**Polymerisation**

The olefin polymerized using the catalyst is preferably propylene or a higher alpha-olefin or a mixture of ethylene and an $\alpha$-olefin or a mixture of alpha olefins, for example $C_{2-20}$ olefins, e.g. ethylene, propylene, 1-butene, 1-hexene, 4-methyl-1-pentene, 1-octene etc. The olefins polymerized in the method of the invention may include any compound which includes unsaturated polymerizable groups. Thus, for example unsaturated compounds, such as $C_{6-20}$ olefins (including cyclic and polycyclic olefins (e.g. norbornene)), and polyenes, especially $C_{4-20}$ dienes, may be included in a comonomer mixture with lower olefins, e.g. $C_{2-5}$ $\alpha$-olefins. Diolefins (i.e. dienes) are suitably used for introducing long chain branching into the resultant polymer. Examples of such dienes include $\alpha,\omega$ linear dienes such as 1,5-hexadiene, 1,6-heptadiene, 1,8-nonadiene, 1,9-decadiene, etc.
The catalysts are particularly suited for use in the manufacture of polypropylene polymers, especially isotactic polypropylene.
It is a feature of the invention that the catalysts enable the formation of polymers with remarkably high melting temperatures, Tm and with remarkably high molecular weight. These features can be achieved at commercially interesting polymerisation temperatures, e.g. 60 °C or more. It is a preferred feature of the invention that the catalysts of the invention are used to polymerise propylene at a temperature of at least 60 °C, preferably at least 65 °C, such as at least 70 °C. It is also notable that catalysts of the present invention produce polymers with high melting temperatures, such as above 156 °C with clearly higher activity of the catalyst compared to catalysts of the prior art.
The catalysts of the invention enable the formation of high molecular weight polypropylene which also possess high isotacticity. Isotacticity is measured by 13C NMR or also by DSC. Thus, in the case of polypropylene homopolymers, isotacticity can be higher than 99.1 % mm when measured by 13C NMR. When measured by standard DSC, the high isotacticity of the polypropylene homopolymers means a melting point (Tm) higher than 150 °C, preferably higher than 152 °C, even more preferably higher than 155 °C.
The molecular weight of the polypropylene can be at least 300,000, preferably at least 400,000, especially at least 500,000. However, the molecular weight of the formed polymer is dependent on the amount of hydrogen employed, as is well known in the art.
The polymers made by the catalysts are useful in all kinds of end articles such as pipes, films (cast, blown and BOPP films), fibers, moulded articles (e.g. injection moulded, blow moulded, rotomoulded articles), extrusion coatings and so on. Film applications, such as those requiring BOPP (bi-oriented polypropylene) film, especially for capacitors are favoured.
The invention will now be illustrated by reference to the following nonlimiting Examples.

Chemicals

All the chemicals and chemical reactions were handled under an inert gas atmosphere using Schlenk and glovebox techniques, with oven-dried glassware, syringes, needles or cannulas.
MAO was purchased from Albermarle and used as a 30 wt-% solution in toluene.
The mixture of perfluoroalkylethyl acrylate esters (CAS 65605-70-1) used as the surfactant was purchased from the Cytonix corporation, dried over activated molecular sieves (2 times) and degassed by argon bubbling prior to use.
Perfluoro-1,3-dimethylcyclohexane (PFC, CAS 335-27-3) was dried over activated molecular sieves (2 times) and degassed by argon bubbling prior to use.
Triethylaluminum was purchased from Crompton and used in pure form. Hydrogen is provided by AGA and purified before use.
Propylene is provided by Borealis and adequately purified before use.
2 M HCl, 12 M HCl (Reachim, Russia), silica gel 60 (40-63 um, Merck), $K_2CO_3$ (Merck), $ZrCl_4(THF)_2$ magnesium turnings (Acros), TsOH (Aldrich), nBuLi (Chemetall), n-hexane (Merck), methyl iodide (Acros), 2-Bromo-4-chloro-toluene (Aldrich), 1-bromo-3,5-di-*tert*-butylbenzene (Aldrich), (3,5-di-tert-butylphenyl)boronic acid (Aldrich), Pd(OAc)2 (Strem), PPh3 (Acros), and DME (Merck) were used as received. Toluene (Merck), THF (Merck), dichloromethane (Merck), were kept and distilled over Na/K alloy. Dichlorodimethylsilane (Merck) was distilled before use. $CDCl_3$, DMSO-$d_6$ and $CD_2Cl_2$ (Deutero GmbH) for NMR experiments were dried and kept over $CaH_2$.
*Bis*(2,6-diisopropylphenyl)imidazolium chloride, i.e. IPr(HCl), and $(IPr)NiCl_2(PPh_3)$ were synthesized as described in [Hintermann, L. Beilstein J. Org. Chem. 2007, 3, 1] and [Matsubara, K.; Ueno, K.; Shibata, Y. Organometallics

2006, 25, 3422], respectively.

4-Bromo-1-methoxy-2-methylindane was obtained as described in [Izmer, V.V.; Lebedev, A.Y.; Nikulin, M.V.; Ryabov, A.N.; Asachenko, A.F.; Lygin, A.V.; Sorokin, D.A.; Voskoboynikov, A.Z. Organometallics 2006, 25, 1217].

## Test methods

### ICP analysis

The elemental analysis of a catalyst was performed by taking a solid sample of mass, M, cooling over dry ice. Samples were diluted up to a known volume, V, by dissolving in nitric acid (HNO3, 65 %, 5 % of V) and freshly deionised (DI) water (5 % of V). The solution was then added to hydrofluoric acid (HF, 40 %, 3 % of V), diluted with DI water up to the final volume, V, and left to stabilise for two hours.

The analysis was run at room temperature using a Thermo Elemental iCAP 6300 Inductively Coupled Plasma - Optical Emmision Spectrometer (ICP-OES) which was calibrated using a blank (a solution of 5 % HNO3, 3 % HF in DI water), and 6 standards of 0.5 ppm, 1 ppm, 10 ppm, 50 ppm, 100 ppm and 300 ppm of Al, with 0.5 ppm, 1 ppm, 5 ppm, 20 ppm, 50 ppm and 100 ppm of Hf and Zr in solutions of 5 % HNO3, 3 % HF in DI water.
Immediately before analysis the calibration is 'resloped' using the blank and 100 ppm Al, 50 ppm Hf, Zr standard, a quality control sample (20 ppm Al, 5 ppm Hf, Zr in a solution of 5 % HNO3, 3 % HF in DI water) is run to confirm the reslope. The QC sample is also run after every 5th sample and at the end of a scheduled analysis set.

The content of hafnium was monitored using the 282.022 nm and 339.980 nm lines and the content for zirconium using 339.198 nm line. The content of aluminium was monitored via the 167.079 nm line, when Al concentration in ICP sample was between 0-10 ppm (calibrated only to 100 ppm) and via the 396.152 nm line for Al concentrations above 10 ppm.

The reported values are an average of three successive aliquots taken from the same sample and are related back to the original catalyst by inputting the original mass of sample and the dilution volume into the software.

### DSC analysis

Melting temperature $T_m$ and crystallization temperature $T_c$ were measured on approx. 5 mg samples with a Mettler-Toledo 822e differential scanning calorimeter (DSC), according to ISO 11357-3 in a heat/cool/heat cycle with a scan rate of 10 °C/min in the temperature range of +23 to +225 °C under a nitrogen flow rate of 50 ml $min^{-1}$. Melting and crystallization temperatures were taken as the endotherm and exotherm peaks, respectively in the second heating and in the cooling step. Calibration of the instrument was performed with $H_20$, Lead, Tin, Indium, according to ISO 11357-1.

### Melt Flow Rate

The melt flow rate (MFR) is determined according to ISO 1133 and is indicated in g/10 min. The MFR is an indication of the flowability, and hence the processability, of the molten polymer. The higher the melt flow rate, the lower the viscosity of the polymer. The MFR is determined at 230 °C and may be determined at different loadings such as 2.16 kg ($MFR_2$) or 21.6 kg ($MFR_{21}$).

### Molecular weight averages, molecular weight distribution (Mn, Mw, Mz, MWD)

Molecular weight averages (Mz, Mw and Mn), Molecular weight distribution (MWD) and its broadness, described by polydispersity index, PDI= Mw/Mn (wherein Mn is the number average molecular weight and Mw is the weight average molecular weight) were determined by Gel Permeation Chromatography (GPC) according to ISO 16014-1:2003, ISO 16014-2:2003, ISO 16014-4:2003 and ASTM D 6474-12 using the following formulas:

$$M_n = \frac{\sum_{i=1}^{N} A_i}{\sum_{i=1}^{N} (A_i / M_i)} \quad (1)$$

$$M_w = \frac{\sum_{i=1}^{N} (A_i x\, M_i)}{\sum_{i=1}^{N} A_i} \quad (2)$$

$$M_z = \frac{\sum_{i=1}^{N}(A_i \, x \, M_i^2)}{\sum_{i=1}^{N}(A_i/M_i)} \quad (3)$$

For a constant elution volume interval $\Delta V_i$, where $A_i$, and $M_i$ are the chromatographic peak slice area and polyolefin molecular weight (MW), respectively associated with the elution volume, $V_i$, where N is equal to the number of data points obtained from the chromatogram between the integration limits.

A high temperature GPC instrument, equipped with either infrared (IR) detector (IR4 or IR5 from PolymerChar (Valencia, Spain) or differential refractometer (RI) from Agilent Technologies, equipped with 3 x Agilent-PLgel Olexis and 1x Agilent-PLgel Olexis Guard columns was used. As the solvent and mobile phase 1,2,4-trichlorobenzene (TCB) stabilized with 250 mg/L 2,6-Di tert butyl-4-methyl-phenol) was used. The chromatographic system was operated at 160 °C and at a constant flow rate of 1 mL/min. 200 μL of sample solution was injected per analysis. Data collection was performed using either Agilent Cirrus software version 3.3 or PolymerChar GPC-IR control software.

The column set was calibrated using universal calibration (according to ISO 16014-2:2003) with 19 narrow MWD polystyrene (PS) standards in the range of 0,5 kg/mol to 11 500 kg/mol. The PS standards were dissolved at room temperature over several hours. The conversion of the polystyrene peak molecular weight to polyolefin molecular weights is accomplished by using the Mark Houwink equation and the following Mark Houwink constants:

$$K_{PS} = 19 \times 10^{-3} \text{ mL/g}, \alpha_{PS} = 0.655$$

$$K_{PE} = 39 \times 10^{-3} \text{ mL/g}, \qquad \alpha_{PE} = 0.725$$

$$K_{PP} = 19 \times 10^{-3} \text{ mL/g}, \alpha_{PP} = 0.725$$

A third order polynomial fit was used to fit the calibration data.

All samples were prepared in the concentration range of 0,5 -1 mg/ml and dissolved at 160 °C for 2.5 hours for PP or 3 hours for PE under continuous gentle shaking.

**Quantification of polypropylene homopolymer microstructure by NMR spectroscopy**

Quantitative nuclear-magnetic resonance (NMR) spectroscopy was used to quantify the isotacticity and content of regio-defects of the polypropylene homopolymers. Quantitative $^{13}C\{^1H\}$ NMR spectra recorded in the solution-state using a Bruker Advance III 400 NMR spectrometer operating at 400.15 and 100.62 MHz for $^1H$ and $^{13}C$ respectively. All spectra were recorded using a $^{13}C$ optimised 10 mm selective excitation probehead at 125 °C using nitrogen gas for all pneumatics. Approximately 200 mg of material was dissolved in 1,2-tetrachloroethane-$d_2$ (TCE-$d_2$). This setup was chosen primarily for the high resolution needed for tacticity distribution quantification (Busico, V., Cipullo, R., Prog. Polym. Sci. 26 (2001) 443; Busico, V.; Cipullo, R., Monaco, G., Vacatello, M., Segre, A.L., Macromolecules 30 (1997) 6251). Standard single-pulse excitation was employed utilising the NOE and bi-level WALTZ16 decoupling scheme (Zhou, Z., Kuemmerle, R., Qiu, X., Redwine, D., Cong, R., Taha, A., Baugh, D. Winniford, B., J. Mag. Reson. 187 (2007) 225; Busico, V., Carbonniere, P., Cipullo, R., Pellecchia, R., Severn, J., Talarico, G., Macromol. Rapid Commun. 2007, 28, 11289). A total of 8192 (8k) transients were acquired per spectra. Quantitative $^{13}C\{^1H\}$ NMR spectra were processed, integrated and relevant quantitative properties determined from the integrals using proprietary computer programs. All chemical shifts are internally referenced to the methyl signal of the isotactic pentad mmmm at 21.85 ppm.

The tacticity distribution was quantified through integration of the methyl region between 23.6 and 19.7 ppm correcting for any sites not related to the stereo sequences of interest (Busico, V., Cipullo, R., Prog. Polym. Sci. 26 (2001) 443; Busico, V., Cipullo, R., Monaco, G., Vacatello, M., Segre, A.L., Macromolecules 30 (1997) 6251). The pentad isotacticity was determined through direct integration of the methyl region and reported as either the mole fraction or percentage of isotactic pentad mmmm with respect to all steric pentads i.e. [mmmm] = mmmm / sum of all steric pentads. When appropriate integrals were corrected for the presence of sites not directly associated with steric pentads.

Characteristic signals corresponding to regio irregular propene insertion were observed (Resconi, L., Cavallo, L., Fait, A., Piemontesi, F., Chem. Rev. 2000, 100, 1253). The presence of secondary inserted propene in the form of 2,1 erythro regio defects was indicated by the presence of the two methyl signals at 17.7 and 17.2 ppm and confirmed by the presence of other characteristic signals. The amount of 2,1 erythro regio defects was quantified using the average integral (e) of the e6 and e8 sites observed at 17.7 and 17.2 ppm respectively, i.e. e = 0.5 * (e6 + e8). Characteristic signals corresponding to other types of regio irregularity were not observed (Resconi, L., Cavallo, L., Fait, A., Piemontesi,

F., Chem. Rev. 2000, 100, 1253). The amount of primary inserted propene (p) was quantified based on the integral of all signals in the methyl region (CH3) from 23.6 to 19.7 ppm paying attention to correct for other species included in the integral not related to primary insertion and for primary insertion signals excluded from this region such that p = CH3 + 2*e. The relative content of a specific type of regio defect was reported as the mole fraction or percentage of said regio defect with respect all observed forms of propene insertion i.e. sum of all primary (1,2), secondary (2,1) and tertiary (3,1) inserted propene units, e.g. [21e] = e/ (p + e + t + i). The total amount of secondary inserted propene in the form of 2,1-erythro or 2,1-threo regio defects was quantified as sum of all said regio irregular units, i.e. [21] = [21e] + [21t].

*Synthesis of the complexes*

**Comparative complexes:**

**C-mcl:**

The metallocene rac-dimethylsilylenebis(2-methyl-4-(3,5 -di-tert-butylphenyl)-inden-1-yl)zirconium dichloride has been synthesized as described in European Patent Application No. 12199251.5.

**C-mc2:**

The metallocene rac-dimethylsilylenebis(2-methyl-4-(3,5-di-tert-butylphenyl)-7-methoxy-inden-1-yl)zirconium dichloride has been synthesized as described in EP-A-2532687.

**Inventive compl:**

**I-mc3:**

**1-(Chloromethyl)-4-isopropylbenzene**

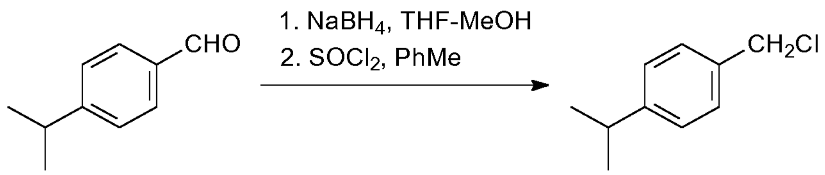


500 ml of methanol was added dropwise by vigorous stirring over 5 h to a mixture of 148 g (1.0 mol) 4-isopropylbenzaldehyde and 37.8 g (1.0 mol) of $NaBH_4$ in 1000 ml of THF at 0-5 °C. This mixture was stirred overnight at room temperature and then evaporated under vacuum. The residue was acidified with 1200 ml of 2 M HCl to pH~1, and the formed (4-isopropylphenyl)methanol was extracted with 3 x 400 ml of dichloromethane. The combined organic extract was dried over $Na_2SO_4$ and evaporated to dryness. To the residue dissolved in 1000 ml of dichloromethane 73 ml (1.0 mol) of thionyl chloride was added dropwise at +5°C. The resulting solution was stirred at room temperature overnight, evaporated to dryness, and then the residue was dissolved in 750 ml dichloromethane. The formed solution was washed by 250 ml of water. The organic layer was separated, the aqueous layer was extracted with 2 x 150 ml of dichloromethane. The combined organic extract was dried over $Na_2SO_4$, passed through a short pad of silica gel 60 (40-63 μm), and evaporated to dryness. Crude product was distilled under vacuum to give 142 g (84%) of a colorless liquid, b.p. 107-112 °C/15 mm Hg.
Anal. calc. for $C_{10}H_{13}Cl$: C, 71.21; H, 7.77. Found: C, 71.25; H, 7.63.
$^1H$ NMR ($CDCl_3$): $\delta$7.31 (d, $J$ = 8.1 Hz, 2H), 7.21 (d, $J$ = 8.1 Hz, 2H), 4.56 (s, 2H), 2.96-2.85 (m, 1H), 1.24 (d, $J$ = 6.8 Hz, 6H).

**3-(4-Isopropylphenyl)-2-methylpropanoyl chloride**

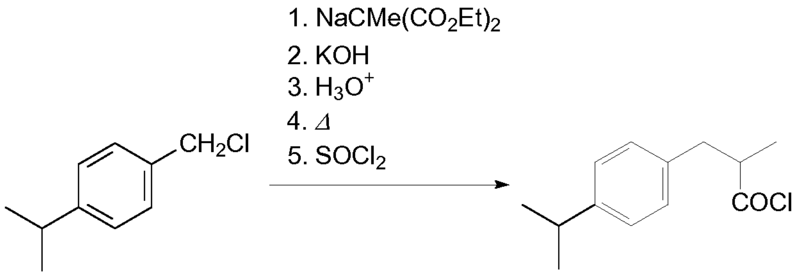


120 g (0.691 mol) of diethyl methylmalonate was added to a solution of sodium ethoxide prepared from 19.0 g (0.827 mol) of sodium metal and 500 ml of anhydrous ethanol. This mixture was stirred for 15 min, then 117 g (0.691 mmol) of 1-(chloromethyl)-4-isopropylbenzene was added at such a rate as to maintain a gentle reflux. This mixture was refluxed for 3 h and then cooled to room temperature. A solution of 138 g of KOH in 370 ml of water was added. The obtained mixture was refluxed for 4 h to saponificate the ester formed. Ethanol and water were distilled off until the temperature reached 95 °C, and 2000 ml of water and then 12 M HCl (to pH~1) were added to the residue. The precipitated substituted methylmalonic acid was filtered off, washed with water, and partially dried on the filter. Crude 3-(4-isopropylphenyl)-2-methylpropanoic acid was obtained after decarboxylation of this substituted methylmalonic acid at 180°C. A mixture of this acid and 175 ml of thionyl chloride was stirred at room temperature for 24 h, and then an excess of thionyl chloride was distilled off. The residue was distilled under vacuum to give *135 g (87%)* of the title compound, b.p. 114-119°C/5 mm Hg.

Anal. calc. for $C_{13}H_{17}ClO$: C, 69.48; H, 7.62. Found: C, 69.63; H, 7.80.

$^1$H NMR ($CDCl_3$): $\delta$ 7.14-7.17 (m, 2H, 2,6-H), 7.08-7.11 (m, 2H, 3,5-H), 3.09-3.17 (m, 2H, ArC*H*H' and C*H*C(O)Cl), 2.87 (sep, *J* = 7.0 Hz, 1H, C*H*Me$_2$), 2.68-2.74 (m, 1H, ArCH*H'*), 1.26 (d, *J* = 7.0 Hz, 3H, CH*Me*), 1.23 (d, *J* = 7.0 Hz, 6H, CH*Me$_2$*). $^{13}$C{$^1$H} NMR ($CDCl_3$): $\delta$ 177.1, 147.4, 134.8, 128.9, 126.6, 53.3, 38.8, 33.7, 23.9, 16.6.

**6-Isopropyl-2-methylindan-1-one**

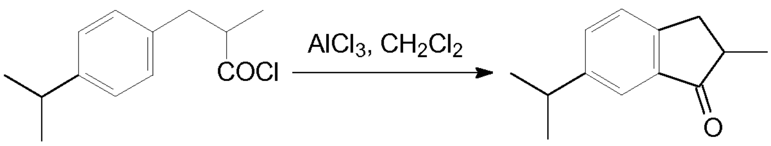


To a suspension of 192 g (1.44 mol, 1.2 eq.) of $AlCl_3$ in 1000 ml of dichloromethane a solution of *270 g (1.2 mol*) of 3-(4-isopropylphenyl)-2-methylpropanoyl chloride in 200 ml of dichloromethane was added dropwise by vigorous stirring at 5°C. The obtained mixture was stirred overnight at room temperature and then poured on 1500 g of the crushed ice. The organic layer was separated, and the aqueous layer was extracted with 3 x 100 ml of dichloromethane. The combined organic extract was washed by aqueous $K_2CO_3$, dried over $K_2CO_3$, passed through a short pad of silica gel 60 (40-63 μm), and then evaporated to dryness. Crude product was distilled under vacuum to give 200 g (89%) of as a colorless liquid, b.p. 125-131 °C/5 mm Hg.

Anal. calc. for $C_{13}H_{16}O$: C, 82.94; H, 8.57. Found: C, 82.92; H, 8.66.

$^1$H NMR ($CDCl_3$): $\delta$ 7.63 (d, *J* = 1.1 Hz, 1H, 7-H), 7.46 (dd, *J* = 7.9 Hz, *J*= 1.8 Hz, 1H, 5-H), 7.36 (d, *J* = 7.9 Hz, 1H, 5-H), 3.35 (dd, *J* = 17.0 Hz, *J* = 7.9 Hz, 1H, 3-C*H*H'), 2.97 (sep, *J* = 7.0 Hz, 1H, C*H*Me$_2$), 2.65-2.74 (m, 2H, 3-CH*H'* and 2-H), 1.30 (d, *J* = 7.3 Hz, 3H, CH*Me*), 1.26 (d, *J* = 7.0 Hz, 6H, CH*Me$_2$*). $^{13}$C{$^1$H} NMR ($CDCl_3$): $\delta$ 209.5, 151.2, 148.4, 136.4, 133.7, 126.2, 121.0, 42.2, 34.5, 33.8, 23.9, 16.2.

**4,7-Dibromo-6-isopropyl-2-methylindan-1-one**

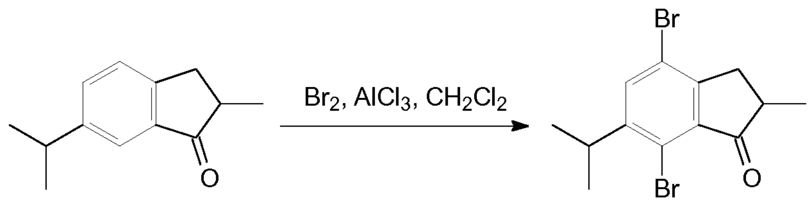


a A solution of 230 g (1.22 mol) of 6-isopropyl-2-methylindan-1-one in 700 ml of dichloromethane was added dropwise with vigorous stirring over 1 h to a suspension of 416 g (3.12 mol, 2.55 eq.) of $AlCl_3$ in 1100 ml of dichloromethane at 0 °C. The reaction mixture was stirred for 10 min at this temperature, then 2 g of iron powder was added. Further on, 402 g (2.52 mol, 2.07 eq.) of bromine was added dropwise for 1 h. The resulting mixture was stirred overnight at room temperature and then poured onto 4000 $cm^3$ of the crushed ice. The organic layer was separated, and the aqueous layer was extracted with 3 x 400 ml of dichloromethane. The combined organic extract was washed with aqueous $K_2CO_3$, dried over $K_2CO_3$, passed through a short pad of silica gel 60 (40-63 μm), and then evaporated to dryness. The resultant dark-red oily liquid was distilled under vacuum: a broad-boiling fraction with b.p. 140-200 °C/6 mm Hg was collected to give 357 g of crude product. This crude product was dissolved in 1200 ml of hot hexane, and the formed solution was kept in a -30 °C freezer over 2 days. A yellowish solid precipitate formed, which was collected and dried in vacuo. This procedure gave 275 g (65%) of 4,7-dibromo-6-isopropyl-2-methylindan-1-one. The mother liquor was evaporated to dryness to give 97.9 g of a mixture (~80% purity) of 4-bromo-6-isopropyl-2-methylindan-1-one and 4,7-dibromo-6-isopropyl-2-methylindan-1-one in ratio equal to ca. 1 to 0.43, i.e. it contains ca. 0.235 mol of 4-bromo-6-isopropyl-2-methylindan-1-one and 0.101 mol of 4,7-dibromo-6-isopropyl-2-methylindan-1-one. This mixture was dissolved in 130 ml of dichloromethane and then brominated via the above-described protocol using 78.5 g of $AlCl_3$ in 200 ml of dichloromethane, 1 g of iron powder and 40.0 g of bromine. This procedure gave 59.3 g of 4,7-dibromo-6-isopropyl-2-methylindan-1-one. Thus, 4,7-dibromo-6-isopropyl-2-methylindan-1-one was obtained in 79% total yield.

Anal. calc. for $C_{13}H_{14}Br_2O$: C, 45.12; H, 4.08. Found: C, 45.42; H, 4.31.

$^1H$ NMR ($CDCl_3$): $\delta$ 7.64 (s, 1H, 5-H), 3.59 (sep, *J* = 6.9 Hz, 1H, C*H*Me$_2$), 3.25 (dd, *J* = 17.4 Hz, *J* = 8.0 Hz, 1H, 3-C*H*H'), 2.69-2.82 (m, 1H, 2-H), 2.57 (dd, *J* = 17.4 Hz, *J* = 4.1 Hz, 1H, 3-CH*H'*), 1.33 (d, *J* = 7.2 Hz, 3H, 2-Me), 1.25 (d, *J* = 6.9 Hz, 6H, CH*Me$_2$*). $^{13}C\{^1H\}$ NMR ($CDCl_3$): $\delta$ 205.7, 153.0, 149.9, 135.6, 135.1, 121.2, 119.9, 42.8, 34.6, 31.4, 22.7, 16.2.

**4,7-Dibromo-6-isopropyl-1-methoxy-2-methylindane**

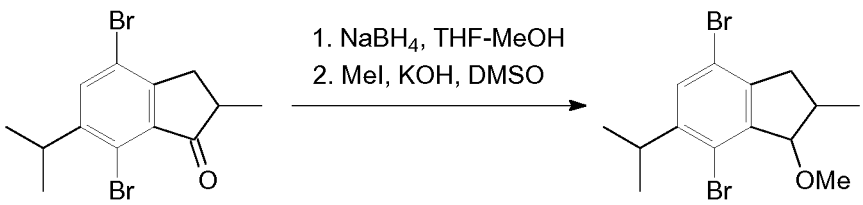


300 ml of methanol was added dropwise over 5 h with vigorous stirring to a mixture of 167 g (0.483 mol) of 4,7-dibromo-6-isopropyl-2-methylindan-1-one and 27.7 g (0.732 mol) of $NaBH_4$ in 600 ml of THF at 0-5 °C. This mixture was stirred overnight at room temperature and then evaporated under vacuum. The residue was acidified by 2 M HCl to pH~5, and the formed 4-bromo-6-isopropyl-2-methylindan-1-ol was extracted with 3 x 300 ml of dichloromethane. The combined organic extract was dried over $Na_2SO_4$ and evaporated to dryness. The residue was immediately dissolved in 1050 ml of DMSO, then 127 g (2.26 mol, 4.7 eq.) of KOH and 157 g (1.1 mol, 2.28 eq.) of iodomethane were added. This mixture was stirred for 5 h at ambient temperature and then poured into 3000 ml of water. Crude product was extracted with 3 x 500 ml of dichloromethane. The combined organic extract was washed with 7 x 1000 ml of water, dried over $Na_2SO_4$, and then evaporated to dryness. Crude product was distilled under vacuum to give 174 g (99%) of a colorless liquid (b.p. 148-162 °C/5 mm Hg) containing a mixture of two stereoisomers.

Anal. calc. for $C_{14}H_{18}Br_2O$: C, 46.44; H, 5.01. Found: C, 46.63; H, 5.18.

*Syn*-isomer: $^1H$ NMR ($CDCl_3$): $\delta$ 7.29 (s, 1H, 5-H), 4.66 (d, *J* = 5.5 Hz, 1H, 1-H), 3.55 (s, 3H, OMe), 3.36 (sep, *J* = 6.9 Hz, 1H, C*H*Me$_2$), 2.95 (dd, *J* = 16.0 Hz, *J* = 7.8 Hz, 1H, 3-C*H*H'), 2.75 (dd, *J* = 16.0 Hz, *J* = 9.6 Hz, 1H, 3-CH*H'*), 2.38-2.53 (m, 1H, 2-H), 1.25 (d, *J* = 7.1 Hz, 3H, 2-Me), 1.22 (d, *J* = 6.9 Hz, 3H, CHMeMe'), 1.20 (d, *J* = 6.9 Hz, 3H, CHMe*Me'*). $^{13}C\{^1H\}$ NMR ($CDCl_3$): $\delta$ 147.7, 145.6, 143.8, 129.7, 121.4, 119.1, 88.0, 59.5, 40.7, 38.7, 32.5, 22.9, 22.8, 13.5.

Anti-isomer: $^1H$ NMR ($CDCl_3$): $\delta$ 7.32 (s, 1H, 5-H), 4.48 (s, 1H, 1-H), 3.46 (s, 3H, OMe), 3.25-3.43 (m, 2H, C*H*Me$_2$ and

3-C*H*H'), 2.52-2.63 (m, 1H, 2-H), 2.49 (dd, *J* = 16.7 Hz, *J* = 1.6 Hz, 1H, 3-CH*H'*), 1.22 (d, 6H, *J* = 6.9 Hz, CH*Me*$_2$), 1.05 (d, *J* = 7.3 Hz, 3H, 2-Me). $^{13}C\{^1H\}$ NMR ($CDCl_3$): $\delta$ 148.0, 143.2, 143.1, 130.1, 122.0, 119.7, 93.4, 57.1, 40.7, 36.1, 32.4, 22.9, 22.8, 19.9.

**1-methoxy-2-methyl-4-bromo-6-isopropylindane**

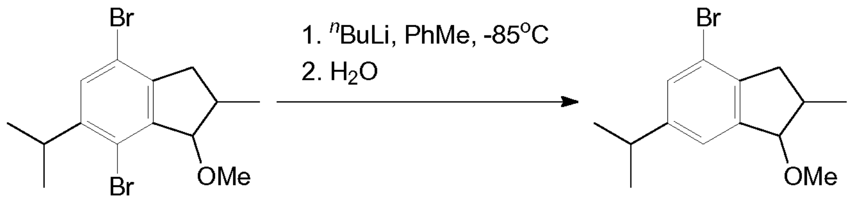


46.0 ml (0.115 mol) of 2.5 M $^n$BuLi in hexanes was added dropwise over 30 min with vigorous stirring to a solution of 41.4 g (0.114 mol) of 4,7-dibromo-6-isopropyl-1-methoxy-2-methylindane in 250 ml of toluene at -85 °C. The resulting mixture was allowed to warm to
-30 °C/-35 °C and stirred for 30 min at this temperature. The reaction was quenched by addition of 150 ml of water. The organic layer was separated, and the aqueous layer was extracted with 100 ml of dichloromethane. The combined organic extract was dried over $K_2CO_3$ and then passed through a short pad of silica gel 60 (40-63 μm). The silica gel layer was additionally washed with 50 ml of dichloromethane. The combined organic eluate was evaporated to dryness to give 34.6 g (~100% yield, the only impurity is toluene) of 4-bromo-6-isopropyl-1-methoxy-2-methylindane as a slightly yellowish liquid. The $^1H$ and $^{13}C$ NMR spectra were identical to those reported above.
Anal. calc. for $C_{14}H_{19}BrO$: C, 59.37; H, 6.76. Found: C, 59.13; H, 6.88.
*Syn*-isomer: $^1H$ NMR ($CDCl_3$): $\delta$ 7.26 (s, 1H, 5-H), 7.15 (s, 1H, 7-H), 4.56 (d, *J*= 5.5 Hz, 1H, 1-H), 3.41 (s, 3H, OMe), 2.81-2.97 (m, 2H, 3-C*H*H' and C*H*Me$_2$), 2.57-2.70 (m, 2H, 3-CH*H'* and 2-H), 1.23 (d, *J* = 6.8 Hz, 6H, CH*Me*$_2$), 1.07 (d, *J* = 6.6 Hz, 3H, 2-Me). $^{13}C\{^1H\}$ NMR ($CDCl_3$): $\delta$ 149.4, 144.5, 140.8, 129.3, 122.0, 120.2, 86.8, 57.0, 39.0, 37.9, 33.9, 24.1, 24.0, 13.5.
Anti-isomer: $^1H$ NMR ($CDCl_3$): $\delta$ 7.28 (s, 1H, 5-H), 7.16 (s, 1H, 7-H), 4.42 (d, *J* = 4.3 Hz, 1H, 1-H), 3.46 (s, 3H, OMe), 3.18 (dd, *J* = 16.2 Hz, *J* = 7.8 Hz, 1H, 3-C*H*H'), 2.88 (sep, *J* = 6.9 Hz, 1H, C*H*Me$_2$), 2.44-2.57 (m, 1H, 2-H), 2.40 (dd, *J* = 16.2 Hz, *J* = 5.5 Hz, 1H, 3-CH*H'*), 1.23 (d, *J* = 6.9 Hz, 6H, CH*Me*$_2$), 1.18 (d, *J* = 6.8 Hz, 3H, 2-Me). $^{13}C\{^1H\}$ NMR ($CDCl_3$): $\delta$ 149.7, 144.1, 140.6, 129.5, 122.2, 120.1, 92.1, 56.6, 39.33, 39.25, 33.9, 24.1, 23.9, 19.4.

**1-methoxy-2-methyl-4-(3,5-Di-*tert*-butylphenyl)-6-isopropylindane**

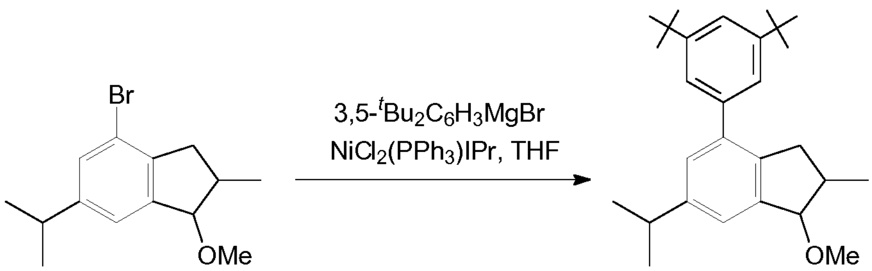


400 ml (200 mmol) of 0.5 M 3,5-di-*tert*-butylphenylmagnesium bromide in THF was added to a mixture of 1.0 g (1.28 mmol) of $NiCl_2(PPh_3)IPr$ and 37.6 g (133 mmol) of 4-bromo-6-isopropyl-1-methoxy-2-methylindane at such a rate as to maintain a gentle reflux. The resulting solution was refluxed for 1 h, then cooled to room temperature, and 150 ml of water was added. The major part of THF was distilled off on rotary evaporator, then 500 ml of dichloromethane and 1000 ml of 1 M HCl were added to the residue. The organic layer was separated, and the aqueous layer was extracted with 150 ml of dichloromethane. The combined organic extract was evaporated to dryness to give a slightly greenish oil. The product was isolated by flash chromatography on silica gel 60 (40-63 μm; eluent: hexanes-dichloromethane = 2:1 vol., then 1:1 vol.). This procedure gave 4-(3,5-di-*tert*-butylphenyl)-6-isopropyl-1-methoxy-2-methylindane as colorless thick oil which slowly crystallized at room temperature. The product is a mixture of two stereoisomers.

Anal. calc. for $C_{28}H_{40}O$: C, 85.66; H, 10.27. Found: C, 85.86; H, 10.43.

*Syn*-isomer: $^1$H NMR ($CDCl_3$): $\delta$ 7.39 (t, *J* = 1.8 Hz, 1H, 4-H in 3,5-$^tBu_2C_6H_3$), 7.29 (d, *J* = 1.8 Hz, 2H, 2,6-H in 3,5-$^tBu_2C_6H_3$), 7.23 (m, 1H, 5-H in indane), 7.20 (m, 1H, 7-H in indane), 4.53 (d, *J* = 5.5 Hz, 1H, 1-H), 3.48 (s, 3H, OMe), 2.98 (sep, *J* = 6.9 Hz, 1H, C*H*Me$_2$), 2.92 (dd, *J* = 15.5 Hz, *J* = 7.1 Hz, 1H, 3-C*H*H'), 2.79 (dd, *J* = 15.5 Hz, *J* = 6.7 Hz, 1H, 3-CH*H'*), 2.49-2.65 (m, 1H, 2-H), 1.37 (s, 18H, 3,5-$^t$*Bu*$_2C_6H_3$), 1.30 (d, *J* = 6.9 Hz, 6H, CH*Me*$_2$), 1.09 (d, *J* = 6.8 Hz, 3H, 2-Me in indane). $^{13}$C{$^1$H} NMR ($CDCl_3$): $\delta$ 150.4, 147.4, 143.5, 140.2, 139.5, 138.7, 126.9, 123.0, 121.8, 120.7, 86.4, 56.9, 39.1, 38.0, 34.9, 34.1, 31.5, 24.3, 24.2, 13.6.

Anti-isomer: $^1$H NMR ($CDCl_3$): $\delta$ 7.39 (t, *J* = 1.8 Hz, 1H, 4-H in 3,5-$^tBu_2C_6H_3$), 7.28 (d, *J* = 1.8 Hz, 2H, 2,6-H in 3,5-$^tBu_2C_6H_3$), 7.24 (m, 1H, 5-H in indane), 7.20 (m, 1H, 7-H in indane), 4.53 (d, *J* = 3.9 Hz, 1H, 1-H), 3.52 (s, 3H, OMe), 3.29 (dd, *J* = 15.3 Hz, *J* = 7.1 Hz, 1H, 3-C*H*H'), 2.98 (sep, *J* = 6.9 Hz, 1H, C*H*Me$_2$), 2.40-2.56 (m, 2H, 3-CH*H'* and 2-H), 1.37 (s, 18H, 3,5-$^t$*Bu*$_2C_6H_3$), 1.30 (d, *J* = 6.9 Hz, 6H, CH*Me*$_2$), 1.13 (d, *J* = 6.8 Hz, 3H, 2-Me in indane). $^{13}$C{$^1$H} NMR ($CDCl_3$): $\delta$ 150.4, 147.7, 142.9, 140.2, 139.6, 138.2, 127.2, 122.9, 121.8, 120.7, 91.6, 56.6, 40.0, 38.1, 34.9, 34.1, 31.5, 24.4, 24.1, 19.2.

**2-methyl-5-isopropyl-7-(3,5-Di-*tert*-butylphenyl)-1*H*-indene**

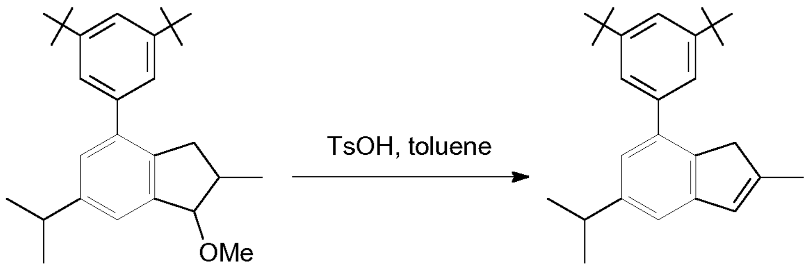


1.0 g of TsOH was added to a solution of 4-(3,5-di-*tert*-butylphenyl)-6-isopropyl-1-methoxy-2-methylindane (as prepared above) in 450 ml of toluene, and the resulting solution was refluxed using Dean-Stark head for 15 min. After that it was cooled to room temperature and washed by 200 ml of 10% aqueous $NaHCO_3$. The organic layer was separated, and the aqueous layer was extracted with 200 ml of dichloromethane. The combined organic extract was evaporated to dryness, and the product was isolated by flash chromatography on silica gel 60 (40-63 $\mu$m; eluent: hexanes-dichloromethane = 10:1, vol.). This procedure gave 46.7 g (98%) of 7-(3,5-di-*tert*-butylphenyl)-5-isopropyl-2-methyl-1*H*-indene as a yellowish glass which slowly crystallized at room temperature.

Anal. calc. for $C_{27}H_{36}$: C, 89.94; H, 10.06. Found: C, 90.24; H, 10.31.

$^1$H NMR ($CDCl_3$): $\delta$ 7.41 (t, *J* = 1.4 Hz, 1H, 4-H in 3,5-$^tBu_2C_6H_3$), 7.38 (d, *J* = 1.4 Hz, 2H, 2,6-H in 3,5-$^tBu_2C_6H_3$), 7.14 (s, 1H, 6-H in indene), 7.02 (s, 1H, 4-H in indene), 6.51 (d, *J* = 1.5 Hz, 1H, 3-H in indene), 3.34 (s, 2H, $CH_2$ in indene), 2.99 (sep, *J* = 6.9 Hz, 1H, C*H*Me$_2$), 2.12 (s, 3H, 2-Me in indene), 1.38 (s, 18H, 3,5-$^t$*Bu*$_2C_6H_3$), 1.31 (d, 6H, *J* = 6.9 Hz, CH*Me*$_2$). $^{13}$C{$^1$H} NMR ($CDCl_3$): $\delta$ 150.5 (two resonances), 147.9, 146.7, 146.5, 140.8, 138.5, 138.3, 127.3, 122.8, 120.8, 116.7, 42.5, 35.0, 34.3, 31.6, 24.4, 16.8.

**Bis[2-methyl-4-(3,5-di-*tert*-butylphenyl)-6-isopropyl-1*H*-inden-1-yl](dimethyl)silane**

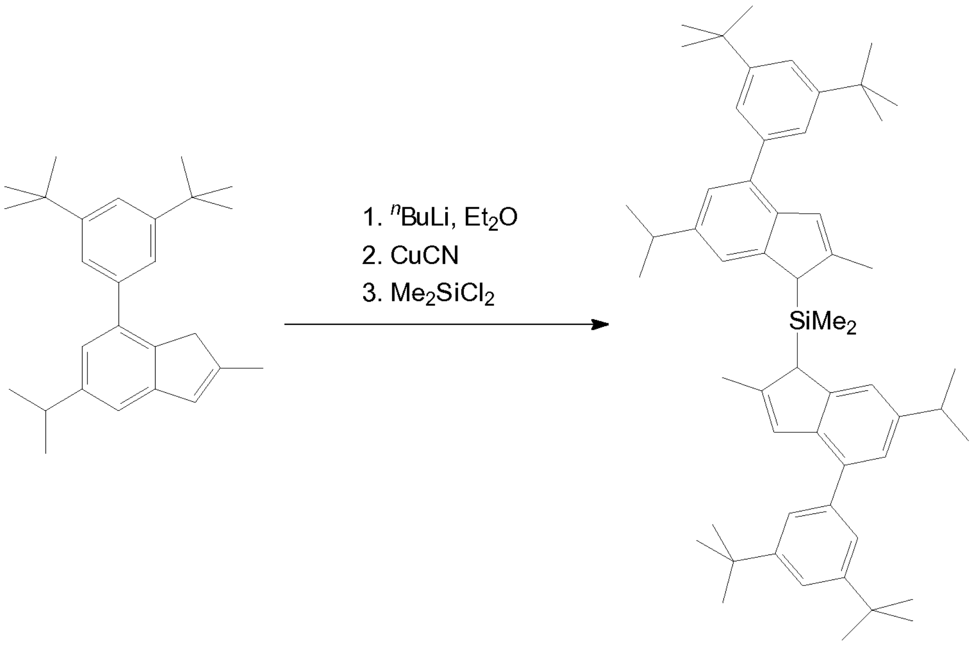


20.0 ml (50.0 mmol) of 2.5 M $^n$BuLi in hexanes was added in one portion to a solution of 18.0 g (50.0 mmol) of 7-(3,5-di-*tert*-butylphenyl)-5-isopropyl-2-methyl-1*H*-indene in 250 ml of ether at -50 °C. This mixture was stirred overnight at room temperature, then the resulting yellow-orange solution was cooled to -40 °C, and 200 mg of CuCN was added. The obtained mixture was stirred for 30 min at -25 °C, and 3.23 g (25.0 mmol) of dichlorodimethylsilane was added in one portion. Further on, this mixture was stirred overnight at ambient temperature, then filtered through a pad of silica gel 60 (40-63 μm) which was additionally washed by 2 x 50 ml of dichloromethane. The combined filtrate was evaporated under vacuum, and the residue was dried in vacuo at elevated temperature. This procedure gave 19.5 g (~100%) of the title product of >90% purity. On the evidence of NMR spectroscopy it was a ca. 3:2 mixture of the stereoisomers. This yellowish glassy product was used without further purification.
$^1$H NMR ($CDCl_3$): $\delta$ 7.48-7.56 (m), 7.38 (m), 7.25-7.30 (m), 6.90 (m), 3.90 (s), 3.88 (s), 3.11 (sept, *J* = 6.9 Hz), 2.36 (s), 1.52 (s), 1.38-1.49 (m), -0.02 (s), -0.08 (s), - 0.09 (s).

***Rac*-dimethylsilanediylbis[2-methyl-4-(3,5-di-*tert*-butylphenyl)-6-isopropyl-inden-1-yl]zirconium dichloride (I-mc3)**

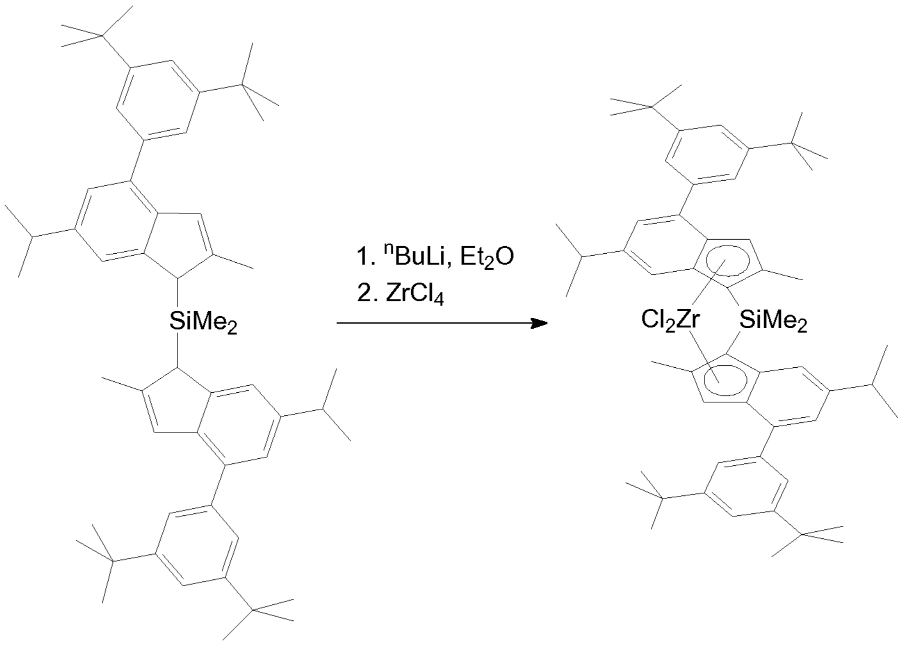


20.0 ml (50.0 mmol) of 2.5 M $^n$BuLi in hexanes was added in one portion to a solution of 19.5 g (25.0 mmol) of bis[2-methyl-4-(3,5-di-*tert*-butylphenyl)-6-isopropyl-1*H*-inden-1-yl](dimethyl)silane (as prepared above) in 250 ml of ether cooled to -30 °C. This mixture was stirred overnight at room temperature. The resulting light-orange solution was cooled to -50 °C and 5.83 g (25.0 mmol) of $ZrCl_4$ was added. The reaction mixture was stirred for 24 h giving orange suspension which then was evaporated to dryness. The residue was poured into 200 ml of hot toluene. This hot mixture was filtered through glass frit (G4). On the evidence of NMR spectroscopy the obtained filtrate included a ca. 1 to 1 mixture of *rac-* and *meso*-zirconocenes. This filtrate was evaporated to ca. 60 ml and then heated to dissolve the formed precipitate. Orange crystals precipitated from this solution overnight at room temperature were collected and dried in vacuum. This procedure gave 7.07 g of pure *meso*-zirconocene. The mother liquor was evaporated to ca. 40 ml. Yellow crystals precipitated from this solution for 4 h at room temperature were collected and dried in vacuum. This procedure gave 6.39 g of pure *rac*-zirconocene. The mother liquor was then evaporated to dryness, and 30 ml of n-hexane was added to the residue. Crystals precipitated from this solution overnight at room temperature were collected and dried in vacuum. This procedure gave 3.92 g of a ca. 6 to 4 mixture *of rac-* and *meso*-zirconocenes. Thus, the total yield of *rac*- and *meso*-zirconocenes isolated in this synthesis was 17.4 g (74%).

*Rac*-zirconocene (I-mc3).

Anal. calc. for $C_{56}H_{74}Cl_2SiZr$: C, 71.75; H, 7.96. Found: C, 71.88; H, 8.10. $^1H$ NMR ($CDCl_3$): $\delta$ 7.52 (d, *J* = 1.8 Hz, 4H), 7.45 (m, 2H), 7.39 (t, *J* = 1.8 Hz, 2H), 7.30 (m, 2H), 6.89 (m, 2H), 2.94 (sept, *J* = 7.0 Hz, 2H), 2.25 (s, 6H), 1.34 (s, 6H), 1.32 (s, 36H), 1.28 (d, *J* = 7.0 Hz, 3H), 1.26 (d, *J* = 7.0 Hz, 3H). $^{13}C\{^1H\}$ NMR ($CDCl_3$): $\delta$ 150.92, 146.39, 139.44, 138.77, 134.74, 131.01, 128.33, 127.30, 123.27, 122.59, 121.45, 119.63, 82.26, 35.05, 34.47, 31.54, 23.59, 23.49, 18.55, 2.87.

*Meso*-zirconocene *(meso-* I-mc3).

Anal. found: C, 72.01; H, 8.15.

$^1H$ NMR ($CDCl_3$): $\delta$ 7.49 (m, 4H), 7.40 (m, 4H), 7.08 (m, 2H), 6.78 (m, 2H), 2.81 (sept, *J* = 6.5 Hz, 2H), 2.43 (s, 6H), 1.48 (s, 3H), 1.34 (s, 36H), 1.25 (d, *J* = 6.5 Hz, 3H), 1.22 (s, 3H), 1.14 (d, *J* = 6.3 Hz, 3H). $^{13}C\{^1H\}$ NMR ($CDCl_3$): $\delta$ 150.77, 145.43, 138.91, 138.63, 135.62, 133.25, 129.23, 125.28, 123.25, 122.17, 121.40, 120.63, 84.23, 35.06, 34.46, 31.57, 23.97, 23.19, 18.60, 3.13, 2.92.

Table 1 summarises the 6-position substituents in the examples:

| **Metallocene** | **$R^6$** |
|---|---|
| C-mc1 | H |
| C-mc2 | H * |
| I-mc3 | *iso*-propyl |
| * with 7-methoxy, | |

***Comparative catalysts***

**CE1 - *Catalyst synthesis with C-mc1***

Inside the glovebox, 80 μL of dry and degassed surfactant solution were mixed with 2 mL of MAO in a septum bottle and left to react overnight. The following day, 64.9 mg of the metallocene C-mc1 was dissolved with 4 mL of the MAO solution in another septum bottle and left to stir inside the glovebox.

After 60 minutes, 1 mL of the surfactant solution and the 4 mL of the MAO-metallocene solution were successively added into a 50mL emulsification glass reactor containing 40mL of PFC at -10 °C and equipped with an overhead stirrer (stirring speed = 600 rpm). Total amount of MAO is 5 mL (300 equivalents). A red-orange emulsion formed immediately and stirred during 15 minutes at 0 °C / 600rpm. Then the emulsion was transferred via a 2/4 teflon tube to 100mL of hot PFC at 90 °C, and stirred at 600rpm until the transfer is completed, then the speed was reduced to 300 rpm. After 15 minutes stirring, the oil bath was removed and the stirrer turned off. The catalyst was left to settle up on top of the PFC and after 45 minutes the solvent was siphoned off. The remaining red catalyst was dried during 2 hours at 50 °C over an argon flow. 0.45 g of a red free flowing powder was obtained.

**CE2 - *Catalyst synthesis with C-mc2***

Inside the glovebox, 80 μL of dry and degassed surfactant solution were mixed with 2 mL of MAO in a septum bottle and left to react overnight. The following day, 69.4 mg of the metallocene *C-mc2* was dissolved with 4 mL of the MAO solution in another septum bottle and left to stir inside the glovebox.

After 60 minutes, 1 mL of the surfactant solution and the 4 mL of the MAO-metallocene solution were successively added into a 50mL emulsification glass reactor containing 40mL of PFC at -10 °C and equipped with an overhead stirrer (stirring speed = 600 rpm). Total amount of MAO is 5 mL (300 equivalents). A red-orange emulsion formed immediately and stirred during 15 minutes at 0 °C / 600rpm. Then the emulsion was transferred via a 2/4 teflon tube to 100mL of hot PFC at 90 °C, and stirred at 600rpm until the transfer is completed, then the speed was reduced to 300 rpm. After 15 minutes stirring, the oil bath was removed and the stirrer turned off. The catalyst was left to settle up on top of the PFC and after 45 minutes the solvent was siphoned off. The remaining red catalyst was dried during 2 hours at 50 °C over an argon flow. 0.74 g of a red free flowing powder was obtained.

**IE3 - *Catalyst synthesis with I-mc3***

Inside the glovebox, 80 μL of dry and degassed surfactant solution were mixed with 2 mL of MAO in a septum bottle and left to react overnight. The following day, 71.2 mg of the metallocene I-mc3 was dissolved with 4 mL of the MAO solution in another septum bottle and left to stir inside the glovebox.

After 60 minutes, 1 mL of the surfactant solution and the 4 mL of the MAO-metallocene solution were successively added into a 50mL emulsification glass reactor containing 40mL of PFC at -10 °C and equipped with an overhead stirrer (stirring speed = 600 rpm). Total amount of MAO is 5 mL (300 equivalents). A red-orange emulsion formed immediately and stirred during 15 minutes at 0 °C / 600rpm. Then the emulsion was transferred via a 2/4 teflon tube to 100mL of hot PFC at 90 °C, and stirred at 600rpm until the transfer is completed, then the speed was reduced to 300 rpm. After 15 minutes stirring, the oil bath was removed and the stirrer turned off. The catalyst was left to settle up on top of the PFC and after 35 minutes the solvent was siphoned off. The remaining red catalyst was dried during 2 hours at 50 °C over an argon flow. 0.60 g of a red free flowing powder was obtained.

**Table 2:** Catalyst composition (ICP)

| Catalyst | Al/Zr (molar) | Zr content (wt%) | MC content (wt%) |
|---|---|---|---|
| CE1 | 280 | 0.36 | 2.71 |
| CE2 | 276 | 0.37 | 2.90 |
| IE3 | 323 | 0.33 | 3.39 |

**Polymerisations**

The polymerisations were performed in a 5 L reactor. 200 µl of triethylaluminum was fed as a scavenger in 5 mL of dry and degassed pentane. The desired amount of hydrogen was then loaded (measured in mmol) and 1100 g of liquid propylene was fed into the reactor.

Procedure A: The temperature was set to 30 °C. The desired amount of catalyst (3 to 30 mg) in 5mL of PFC is flushed into the reactor with a nitrogen overpressure. The temperature is then raised to 70 °C over a period of 15 minutes. The polymerisation is stopped after 30 minutes by venting the reactor and flushing with nitrogen before the polymer is collected. (Polymerisations CP1-CP3)

Procedure B: The temperature was set to 20 °C. The desired amount of catalyst (3 to 30 mg) in 5mL of PFC is flushed into the reactor with a nitrogen overpressure. After 5 minutes of the temperature is raised to 70 °C over a period of 15 minutes. The polymerisation is stopped after 60 minutes by venting the reactor and flushing with nitrogen before the polymer is collected. (Polymerisations CP4-CP6, IP7-IP9)

The catalyst activity was calculated on the basis of the 30 (or 60) minutes period according to the following formula:

**Act Cat:** $$\text{Catalyst Activity (kg/(g(cat)} * \text{h))} = \frac{\text{amount of polymer produced (kg)}}{\text{catalyst loading (g)} \times \text{polymerisation time (h)}}$$

**Act**

**Met:** $$\textbf{Catalyst Metal Activity (kg/(g(cat)} * \textbf{h))} = \frac{\textbf{amount of polymer produced (kg)}}{\textbf{catalyst metal loading (g)} \times \textbf{polymerisation time (h)}}$$

**Table 3:** Polymerization results with CE1, CE2, IE3

| Catalyst | Ex | Cat. (mg) | $H_2$ mmol | Pol. Yield, g | Act cat kg/gcat/h | Act Metal kg/gmetal/h | $MFR_2$ g/10min | $M_w$ kg/mol | $M_w/M_n$ | $T_m$ (°C) | $T_c$ (°C) |
|---|---|---|---|---|---|---|---|---|---|---|---|
| CE2 | CP1 | 18.3 | 1 | 50 | 5.5 | 1480 | 1.9 | 347 | 2.0 | 159.4 | 113.5 |
| | CP2 | 16.1 | 6 | 120 | 15.0 | 4042 | 19.0 | 190 | 2.1 | 156.2 | 113.8 |
| | CP3 | 13.6 | 15 | 114 | 16.8 | 4531 | 120 | 106 | 2.2 | 156.6 | 116.5 |
| CE1 | CP4 | 11.1 | 1 | 99 | 9.0 | 2487 | 9.4** | 574.0 | 2.5 | 156.5 | 111.9 |
| | CP5 | 5.5 | 6 | 100 | 18.2 | 5051 | 1.9 | 302.0 | 2.4 | 157.5 | 112.9 |
| | CP6 | 10.7 | 15 | 218 | 20.4 | 5659 | 21.0 | 179.0 | 2.4 | 156.6 | 112.7 |
| IE3 | IP7 | 10.8 | 1 | 142 | 13.2 | 3990 | 20.1** | 640.0 | 2.6 | 155.6 | 109.2 |
| | IP8 | 12.6 | 6 | 333 | 26.4 | 7997 | 2.0 | 324.0 | 2.5 | 156.3 | 110.5 |
| | IP9 | 9.2 | 15 | 311 | 33.8 | 10231 | 24.1 | 189.0 | 2.6 | 155.0 | 111.5 |

* Procedure A for (Polymerisations CP1-CP3) and Procedure B for (Polymerisations CP4-CP6, IP7-IP9)

** $MFR_{21}$ (g/10min)

It can be seen from table 3 above that using catalysts of the invention the activity is clearly higher using the same amount of $H_2$ in the polymerisation when inventive catalysts are compared to the results obtained using comparative catalysts. The polymers still have higher Mw (lower MFR) and possess high melting points.

Table 4 summarises the important results herein succinctly.

Table 4

| **MC/ catalyst** | Polymerisation example | **Activity** (kg-PP/g-cat/h) | **Activity** (kg-PP/g-Zr/h) | **Mw** (kg/mol) | $M_w/M_n$ | **Tm** (°C) |
|---|---|---|---|---|---|---|
| C-mc1/ CE1 | CP5 | 18.2 | 5051 | 302 | 2.4 | 157.5 |
| C-mc2 CE2 | CP1 | 5.5 | 1480 | 347 | 2.0 | 159.4 |
| I-mc3/ IE3 | IP8 | 26.4 | 7997 | 324 | 2.5 | 156.3 |
| * with 7-methoxy | | | | | | |

each X is a sigma ligand;

L is a divalent bridge selected from $-R'_2C-$, $-R'_2C-CR'_2-$, $-R'_2Si-$, $-R'_2Si-SiR'_2-$, $-R'_2Ge-$, wherein each R' is independently a hydrogen atom, $C_{1-20}$-alkyl, tri($C_{1-20}$-alkyl)silyl, $C_{6-20}$-aryl, $C_{7-20}$-arylalkyl or $C_{7-20}$-alkylaryl;

$R_2$ is a $C_{1-20}$-hydrocarbyl radical;

$R_6$ is a linear or branched aliphatic $C_{1-20}$-hydrocarbyl group, $SR_9$ or $OR_9$; with the proviso that $R_6$ does not represent a group having a quaternary carbon atom directly attached to the indenyl ring;

$R_9$ is a $C_{1-20}$-hydrocarbyl group';

n is independently 1, 2 or 3; and

each $R_8$ is a $C_{1-20}$-hydrocarbyl group.

D. A catalyst of any preceding embodiment comprising a symmetrical complex of formula (III)

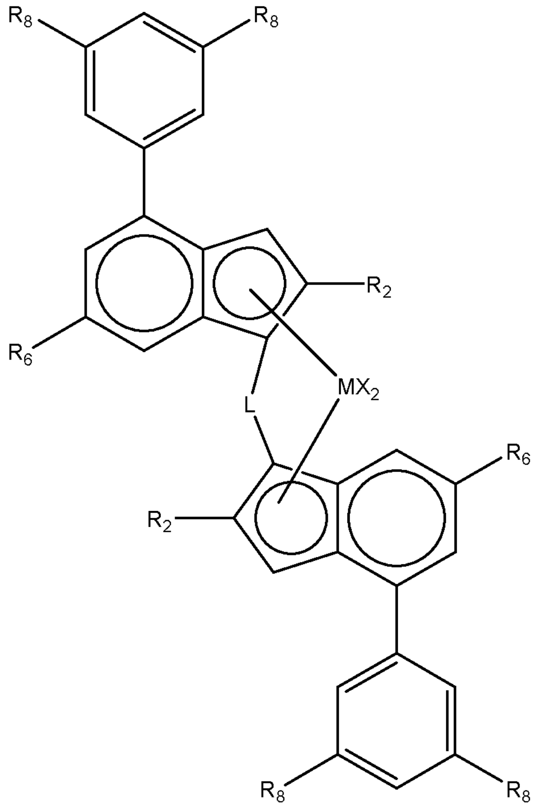

(III)

wherein

M is zirconium or hafnium;

each X is a sigma ligand, preferably each X is independently a hydrogen atom, a halogen atom, $C_{1-6}$-alkoxy group, $C_{1-6}$-alkyl, phenyl or benzyl group;

L is a divalent bridge selected from -R'$_2$C-, -R'$_2$C-CR'$_2$-, -R'$_2$Si-, -R'$_2$Si-SiR'$_2$-, -R'$_2$Ge-, wherein each R' is independently a hydrogen atom, $C_{1-20}$-alkyl, tri($C_{1-20}$-alkyl)silyl, $C_{6-20}$-aryl, $C_{7-20}$-arylalkyl or $C_{7-20}$-alkylaryl; preferably dimethylsilyl, methylene or ethylene;

$R_2$ is a $C_{1-10}$-alkyl group;

$R_6$ is a $C_{1-10}$-alkyl group with the proviso that $R_6$ does not represent a group having a quaternary carbon atom directly attached the indenyl ring;

and each $R^8$ is a $C_{1-20}$-hydrocarbyl group.

E. A catalyst of any preceding embodiment comprising a symmetrical complex of formula (IV):

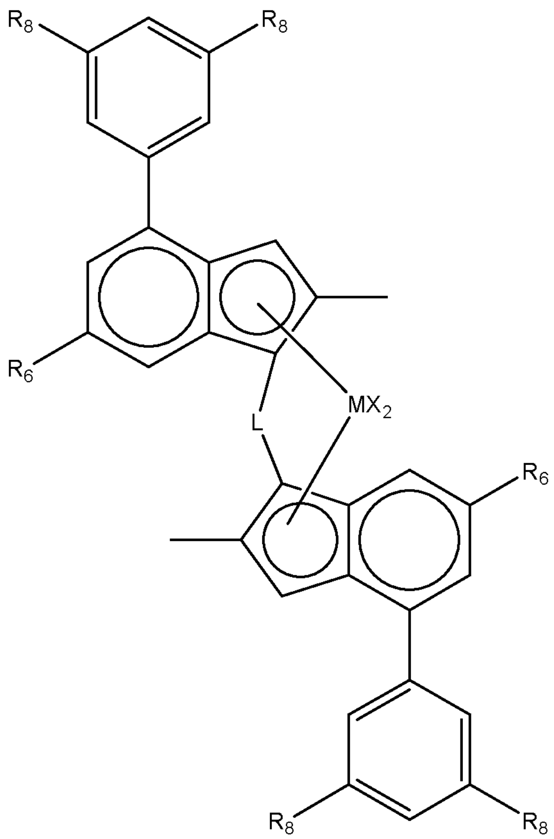

(IV)

wherein

M is zirconium or hafnium;
each X is a sigma ligand, preferably each X is independently a hydrogen atom, a halogen atom, $C_{1-6}$-alkoxy group, $C_{1-6}$-alkyl, phenyl or benzyl group;
L is a divalent bridge selected from -R'$_2$C-, -R'$_2$C-CR'$_2$-, -R'$_2$Si-, -R'$_2$Si-SiR'$_2$-, -R'$_2$Ge-, wherein each R' is independently a hydrogen atom, $C_{1-20}$-hydrocarbyl, tri($C_{1-20}$-alkyl)silyl, $C_{6-20}$-aryl, $C_{7-20}$-arylalkyl or $C_{7-20}$-alkylaryl; preferably dimethylsilyl;
$R_6$ is a linear $C_{1-10}$-alkyl group, -CH($C_{1-4}$-alkyl)$_2$ group or $CH_2$($C_{1-9}$-alkyl) group, where said $C_{1-4}$-alkyl and $C_{1-9}$-alkyl groups can be linear or branched; and
each $R_8$ is a $C_{1-10}$-alkyl group.

F. A catalyst of any preceding embodiment comprising a symmetrical complex of formula (V)

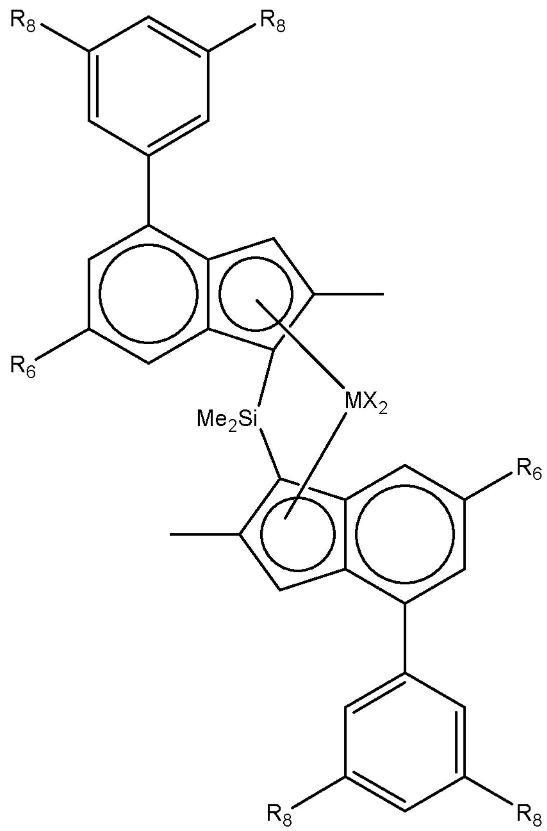

(V)

wherein M is Zr/Hf;
X is a hydrogen atom, a halogen atom, $C_{1-6}$-alkoxy group, $C_{1-6}$-alkyl, phenyl or benzyl group;
$R_6$ is linear $C_{1-6}$-alkyl, -CH($C_{1-4}$-alkyl)$_2$ or -$CH_2$($C_{1-6}$-alkyl), where said $C_{1-4}$-alkyl and $C_{1-6}$-alkyl groups can be linear or branched; and
$R_8$ is $C_{3-8}$-alkyl, such as branched $C_{3-8}$-alkyl.

G. A catalyst of any preceding embodiment comprising a symmetrical complex of formula (VI):

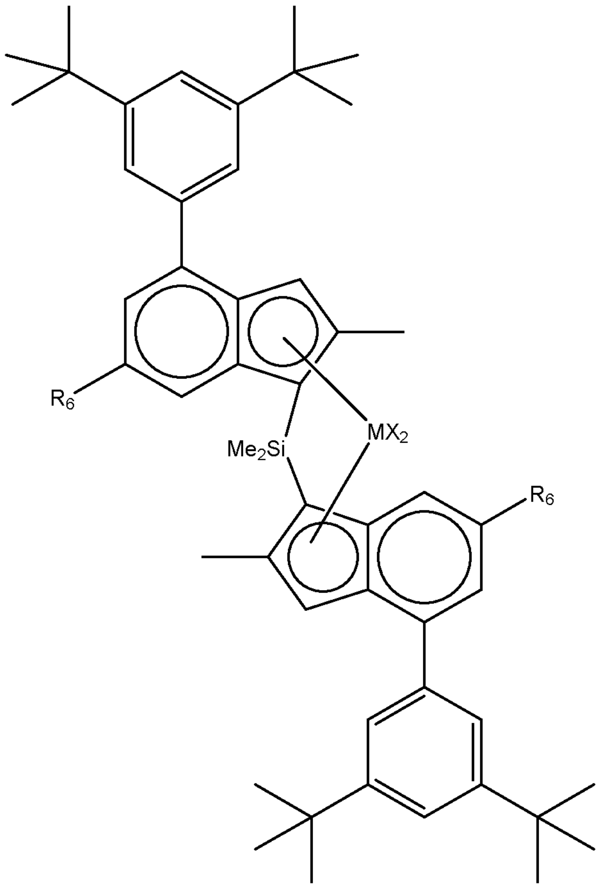

(VI)

wherein M is Zr/Hf
X is a hydrogen atom, a halogen atom, $C_{1-6}$-alkoxy group, $C_{1-6}$-alkyl, phenyl or benzyl group;
$R_6$ is linear $C_{1-6}$-alkyl, -$CH(C_{1-4}$-alkyl$)_2$ or -$CH_2(C_{1-6}$-alkyl) where said $C_{1-4}$-alkyl and $C_{1-6}$-alkyl groups can be linear or branched.

H. A catalyst of any preceding embodiment comprising the complex *Rac*-dimethylsilanediylbis[2,6-dimethyl-4-(3,5-di-*tert*-butylphenyl)-inden-1-yl]zirconium dichloride *Rac*-dimethylsilanediylbis[2-methyl-4-(3,5-di-*tert*-butylphenyl)-6-ethyl-inden-1-yl]zirconium dichloride *Rac*-dimethylsilanediylbis[2-methyl-4-(3,5-di-*tert*-butylphenyl)-6-isopropyl-inden-1-yl]zirconium dichloride *Rac*-dimethylsilanediylbis[2-methyl-4-(3,5-di-*tert*-butylphenyl)-6-neopentyl-inden-1-yl]zirconium dichloride or their Hf analogues.

I. A catalyst of any preceding embodiment wherein said cocatalyst is MAO.

J. A catalyst of any preceding embodiment wherein the two ligands forming the complex are identical.

K. A complex of any of embodiments A to J.

L. A process for the manufacture of a catalyst as defined in any of embodiments A to J comprising obtaining a (i) complex of formula (I) and (ii) a cocatalyst comprising a compound of a group 13 metal, e.g. Al or boron; forming a liquid/liquid emulsion system, which comprises a solution of catalyst components (i) and (ii) dispersed in a solvent, and solidifying said dispersed droplets to form solid particles.

M. A process for the polymerisation of propylene comprising polymerising propylene with a catalyst as defined in any of embodiments A to J.

N. A process as defined in embodiment M wherein said process forms an isotactic polypropylene.

O. A process as defined in embodiment N wherein the catalyst activity in said process is at least 10.0 kg/g(cat)/h.

The invention will now be illustrated by reference to the following nonlimiting Examples.

Chemicals

All the chemicals and chemical reactions were handled under an inert gas atmosphere using Schlenk and glovebox techniques, with oven-dried glassware, syringes, needles or cannulas.

MAO was purchased from Albermarle and used as a 30 wt-% solution in toluene.

The mixture of perfluoroalkylethyl acrylate esters (CAS 65605-70-1) used as the surfactant was purchased from the Cytonix corporation, dried over activated molecular sieves (2 times) and degassed by argon bubbling prior to use.

Perfluoro-1,3-dimethylcyclohexane (PFC, CAS 335-27-3) was dried over activated molecular sieves (2 times) and degassed by argon bubbling prior to use.

Triethylaluminum was purchased from Crompton and used in pure form. Hydrogen is provided by AGA and purified before use.

Propylene is provided by Borealis and adequately purified before use.

2 M HCl, 12 M HCl (Reachim, Russia), silica gel 60 (40-63 um, Merck), $K_2CO_3$ (Merck), $ZrCl_4(THF)_2$ magnesium turnings (Acros), TsOH (Aldrich), nBuLi (Chemetall), n-hexane (Merck), methyl iodide (Acros), 2-Bromo-4-chloro-toluene (Aldrich), 1-bromo-3,5-di-*tert*-butylbenzene (Aldrich), (3,5-di-tert-butylphenyl)boronic acid (Aldrich), Pd(OAc)2 (Strem), PPh3 (Acros), and DME (Merck) were used as received. Toluene (Merck), THF (Merck), dichloromethane (Merck), were kept and distilled over Na/K alloy. Dichlorodimethylsilane (Merck) was distilled before use. $CDCl_3$, DMSO-$d_6$ and $CD_2Cl_2$ (Deutero GmbH) for NMR experiments were dried and kept over $CaH_2$.

Bis(2,6-diisopropylphenyl)imidazolium chloride, i.e. IPr(HCl), and (IPr)$NiCl_2(PPh_3)$ were synthesized as described in [Hintermann, L. Beilstein J. Org. Chem. 2007, 3, 1] and [Matsubara, K.; Ueno, K.; Shibata, Y. Organometallics 2006, 25, 3422], respectively.

4-Bromo-1-methoxy-2-methylindane was obtained as described in [Izmer, V.V.; Lebedev, A.Y.; Nikulin, M.V.; Ryabov, A.N.; Asachenko, A.F.; Lygin, A.V.; Sorokin, D.A.; Voskoboynikov, A.Z. Organometallics 2006, 25, 1217].

Test methods

**ICP analysis**

The elemental analysis of a catalyst was performed by taking a solid sample of mass, M, cooling over dry ice. Samples were diluted up to a known volume, V, by dissolving in nitric acid (HNO3, 65 %, 5 % of V) and freshly deionised (DI) water (5 % of V). The solution was then added to hydrofluoric acid (HF, 40 %, 3 % of V), diluted with DI water up to the final volume, V, and left to stabilise for two hours.

The analysis was run at room temperature using a Thermo Elemental iCAP 6300 Inductively Coupled Plasma - Optical Emmision Spectrometer (ICP-OES) which was calibrated using a blank (a solution of 5 % HNO3, 3 % HF in DI water), and 6 standards of 0.5 ppm, 1 ppm, 10 ppm, 50 ppm, 100 ppm and 300 ppm of Al, with 0.5 ppm, 1 ppm, 5 ppm, 20 ppm, 50 ppm and 100 ppm of Hf and Zr in solutions of 5 % HNO3, 3 % HF in DI water.

Immediately before analysis the calibration is 'resloped' using the blank and 100 ppm Al, 50 ppm Hf, Zr standard, a quality control sample (20 ppm Al, 5 ppm Hf, Zr in a solution of 5 % HNO3, 3 % HF in DI water) is run to confirm the reslope. The QC sample is also run after every 5th sample and at the end of a scheduled analysis set.

The content of hafnium was monitored using the 282.022 nm and 339.980 nm lines and the content for zirconium using 339.198 nm line. The content of aluminium was monitored via the 167.079 nm line, when Al concentration in ICP sample was between 0-10 ppm (calibrated only to 100 ppm) and via the 396.152 nm line for Al concentrations above 10 ppm.

The reported values are an average of three successive aliquots taken from the same sample and are related back to the original catalyst by inputting the original mass of sample and the dilution volume into the software.

**DSC analysis**

Melting temperature $T_m$ and crystallization temperature $T_c$ were measured on approx. 5 mg samples with a Mettler-Toledo 822e differential scanning calorimeter (DSC), according to ISO 11357-3 in a heat/cool/heat cycle with a scan rate of 10 °C/min in the temperature range of +23 to +225 °C under a nitrogen flow rate of 50 ml $min^{-1}$. Melting and crystallization temperatures were taken as the endotherm and exotherm peaks, respectively in the second heating and in the cooling step. Calibration of the instrument was performed with $H_20$, Lead, Tin, Indium, according to ISO 11357-1.

**Melt Flow Rate**

The melt flow rate (MFR) is determined according to ISO 1133 and is indicated in g/10 min. The MFR is an indication of the flowability, and hence the processability, of the molten polymer. The higher the melt flow rate, the lower the viscosity of the polymer. The MFR is determined at 230 °C and may be determined at different loadings such as 2.16 kg ($MFR_2$) or 21.6 kg ($MFR_{21}$).

**Molecular weight averages, molecular weight distribution (Mn, Mw, Mz, MWD)**

Molecular weight averages (Mz, Mw and Mn), Molecular weight distribution (MWD) and its broadness, described by polydispersity index, PDI= Mw/Mn (wherein Mn is the number average molecular weight and Mw is the weight average molecular weight) were determined by Gel Permeation Chromatography (GPC) according to ISO 16014-1:2003, ISO 16014-2:2003, ISO 16014-4:2003 and ASTM D 6474-12 using the following formulas:

$$M_n = \frac{\sum_{i=1}^{N} A_i}{\sum_{i=1}^{N}(A_i/M_i)} \quad (1)$$

$$M_w = \frac{\sum_{i=1}^{N}(A_i x\, M_i)}{\sum_{i=1}^{N} A_i} \quad (2)$$

$$M_z = \frac{\sum_{i=1}^{N}(A_i\, x\, M_i^2)}{\sum_{i=1}^{N}(A_i/M_i)} \quad (3)$$

For a constant elution volume interval $\Delta V_i$, where $A_i$, and $M_i$ are the chromatographic peak slice area and polyolefin molecular weight (MW), respectively associated with the elution volume, $V_i$, where N is equal to the number of data points obtained from the chromatogram between the integration limits.

A high temperature GPC instrument, equipped with either infrared (IR) detector (IR4 or IR5 from PolymerChar (Valencia, Spain) or differential refractometer (RI) from Agilent Technologies, equipped with 3 x Agilent-PLgel Olexis and 1x Agilent-PLgel Olexis Guard columns was used. As the solvent and mobile phase 1,2,4-trichlorobenzene (TCB) stabilized with 250 mg/L 2,6-Di tert butyl-4-methyl-phenol) was used. The chromatographic system was operated at 160 °C and at a constant flow rate of 1 mL/min. 200 μL of sample solution was injected per analysis. Data collection was performed using either Agilent Cirrus software version 3.3 or PolymerChar GPC-IR control software.

The column set was calibrated using universal calibration (according to ISO 16014-2:2003) with 19 narrow MWD polystyrene (PS) standards in the range of 0,5 kg/mol to 11 500 kg/mol. The PS standards were dissolved at room temperature over several hours. The conversion of the polystyrene peak molecular weight to polyolefin molecular weights is accomplished by using the Mark Houwink equation and the following Mark Houwink constants:

$$K_{PS} = 19 \text{ x } 10^{-3} \text{ mL/g}, \alpha_{PS} = 0.655$$

$$K_{PE} = 39 \text{ x } 10^{-3} \text{ mL/g}, \qquad \alpha_{PE} = 0.725$$

$$K_{PP} = 19 \text{ x } 10^{-3} \text{ mL/g}, \alpha_{PP} = 0.725$$

A third order polynomial fit was used to fit the calibration data.

All samples were prepared in the concentration range of 0,5 -1 mg/ml and dissolved at 160 °C for 2.5 hours for PP or 3 hours for PE under continuous gentle shaking.

**Quantification of polypropylene homopolymer microstructure by NMR spectroscopy**

Quantitative nuclear-magnetic resonance (NMR) spectroscopy was used to quantify the isotacticity and content of regio-defects of the polypropylene homopolymers. Quantitative $^{13}C\{^1H\}$ NMR spectra recorded in the solution-state using a Bruker Advance III 400 NMR spectrometer operating at 400.15 and 100.62 MHz for $^1H$ and $^{13}C$ respectively.

All spectra were recorded using a $^{13}C$ optimised 10 mm selective excitation probehead at 125 °C using nitrogen gas for all pneumatics. Approximately 200 mg of material was dissolved in 1,2-tetrachloroethane-$d_2$ (TCE-$d_2$). This setup was chosen primarily for the high resolution needed for tacticity distribution quantification (Busico, V., Cipullo, R., Prog. Polym. Sci. 26 (2001) 443; Busico, V.; Cipullo, R., Monaco, G., Vacatello, M., Segre, A.L., Macromolecules 30 (1997) 6251). Standard single-pulse excitation was employed utilising the NOE and bi-level WALTZ16 decoupling scheme (Zhou, Z., Kuemmerle, R., Qiu, X., Redwine, D., Cong, R., Taha, A., Baugh, D. Winniford, B., J. Mag. Reson. 187 (2007) 225; Busico, V., Carbonniere, P., Cipullo, R., Pellecchia, R., Severn, J., Talarico, G., Macromol. Rapid Commun. 2007, 28, 11289). A total of 8192 (8k) transients were acquired per spectra. Quantitative $^{13}C\{^1H\}$ NMR spectra were processed, integrated and relevant quantitative properties determined from the integrals using proprietary computer programs. All chemical shifts are internally referenced to the methyl signal of the isotactic pentad mmmm at 21.85 ppm.

The tacticity distribution was quantified through integration of the methyl region between 23.6 and 19.7 ppm correcting for any sites not related to the stereo sequences of interest (Busico, V., Cipullo, R., Prog. Polym. Sci. 26 (2001) 443; Busico, V., Cipullo, R., Monaco, G., Vacatello, M., Segre, A.L., Macromolecules 30 (1997) 6251). The pentad isotacticity was determined through direct integration of the methyl region and reported as either the mole fraction or percentage of isotactic pentad mmmm with respect to all steric pentads i.e. [mmmm] = mmmm / sum of all steric pentads. When appropriate integrals were corrected for the presence of sites not directly associated with steric pentads.

Characteristic signals corresponding to regio irregular propene insertion were observed (Resconi, L., Cavallo, L., Fait, A., Piemontesi, F., Chem. Rev. 2000, 100, 1253). The presence of secondary inserted propene in the form of 2,1 erythro regio defects was indicated by the presence of the two methyl signals at 17.7 and 17.2 ppm and confirmed by the presence of other characteristic signals. The amount of 2,1 erythro regio defects was quantified using the average integral (e) of the e6 and e8 sites observed at 17.7 and 17.2 ppm respectively, i.e. e = 0.5 * (e6 + e8). Characteristic signals corresponding to other types of regio irregularity were not observed (Resconi, L., Cavallo, L., Fait, A., Piemontesi, F., Chem. Rev. 2000, 100, 1253). The amount of primary inserted propene (p) was quantified based on the integral of all signals in the methyl region (CH3) from 23.6 to 19.7 ppm paying attention to correct for other species included in the integral not related to primary insertion and for primary insertion signals excluded from this region such that p = CH3 + 2*e. The relative content of a specific type of regio defect was reported as the mole fraction or percentage of said regio defect with respect all observed forms of propene insertion i.e. sum of all primary (1,2), secondary (2,1) and tertiary (3,1) inserted propene units, e.g. [21e] = e/ (p + e + t + i). The total amount of secondary inserted propene in the form of 2,1-erythro or 2,1-threo regio defects was quantified as sum of all said regio irregular units, i.e. [21] = [21e] + [21t].

*Synthesis of the complexes*

**Comparative complexes:**

**C-mcl:**

The metallocene rac-dimethylsilylenebis(2-methyl-4-(3,5 -di-tert-butylphenyl)-inden-1-yl)zirconium dichloride has been synthesized as described in European Patent Application No. 12199251.5.

**C-mc2:**

The metallocene rac-dimethylsilylenebis(2-methyl-4-(3,5-di-tert-butylphenyl)-7-methoxy-inden-1-yl)zirconium dichloride has been synthesized as described in EP-A-2532687.

**Inventive complexes:**

**I-mc1:**

**2-Chloro-4-methylbenzaldehyde**

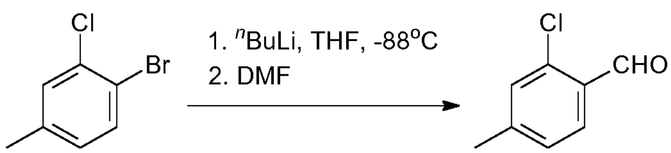

165 ml (413 mmol) of 2.5 M nBuLi in hexanes was added dropwise over 1 h to a solution of 82.2 g (400 mmol) of 4-bromo-3-chlorotoluene in 400 ml of THF cooled to -88 °C. The resulting mixture was stirred for 30 min at this temperature, and then 44.0 g (602 mmol) of DMF was added dropwise by vigorous stirring for 10 min. The reaction mixture was stirred overnight at room temperature, and then 100 ml of water and 400 ml of 3 N HCl were added at 0 °C. The organic layer was separated, the aqueous layer was extracted with 2 x 125 ml of dichloromethane. The combined organic extract was dried over K2CO3 and then passed through a short layer of silica gel 60 (40-63 μm). The silica gel layer was additionally washed by 50 ml of dichloromethane. The combined organic eluate was evaporated to dryness to give a slightly orange liquid which was then distilled under vacuum to give 58.0 g (94%) of the title product (b.p. 99-102 °C/11 mm Hg,) as a colorless liquid crystallized overnight at room temperature.
Anal. calc. for C8H7ClO: C, 62.15; H, 4.56. Found: C, 62.24; H, 4.45.
1H NMR (CDCl3): δ 10.4 (s, 1H, CHO), 7.80 (d, J = 7.8 Hz, 1H, 6-H), 7.25 (s, 1H, 3-H), 7.17 (d, J = 7.8 Hz, 1H, 5-H), 2.40 (s, 3H, 4-Me).

**(2-Chloro-4-methylphenyl)methanol**

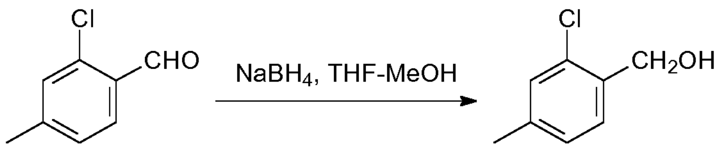


375 ml of methanol was added dropwise with vigorous stirring over 5 h to a mixture of 116 g (0.75 mol) of 2-chloro-4-methylbenzaldehyde and 43.0 g (1.14 mol) of NaBH4 in 750 ml of THF at 0-5 °C. This mixture was stirred overnight at room temperature, and then evaporated to dryness. The resulting oily mass was acidified with 1200 ml of 2 M HCl to pH~1, and the formed product was extracted consequently with 3 x 400 ml of dichloromethane. The combined organic extract was dried over Na2SO4 and evaporated to dryness. This product was used without further purification.
1H NMR (CDCl3): δ 7.29 (d, J = 7.8 Hz, 1H, 5-H), 7.15 (s, 1H, 3-H), 7.04 (d, J = 7.8 Hz, 1H, 6-H), 4.67 (s, 2H, CH2OH), 2.59 (br.s, 1H, CH2OH), 2.30 (s, 3H, 4-Me). 13C{1H} NMR (CDCl3): δ 138.9, 135.0, 132.4, 129.7, 128.6, 127.6, 62.5, 20.7.

**2-Chloro-1-(chloromethyl)-4-methylbenzene**

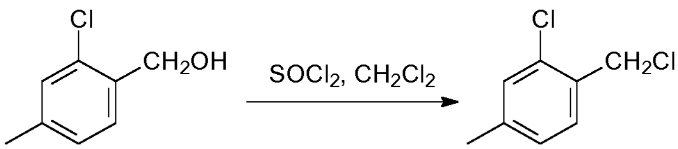


The above-obtained 2-chloro-4-methylbenzyl alcohol dissolved in 750 ml of dichloromethane was added dropwise to 55 ml (754 mmol) of thionyl chloride at +5°C. The resulting solution was stirred overnight at room temperature and then evaporated to dryness. The residue was dissolved in 500 ml dichloromethane, and the formed solution was washed with 250 ml of water. The organic layer was separated and the aqueous layer was extracted with 2 x 150 ml of dichloromethane. The combined organic extract was dried over Na2SO4, passed through a short pad of silica gel 60 (40-63 μm), and then evaporated to dryness. Crude product was distilled under vacuum to give 114 g (87%) of the title product as a colorless liquid, b.p. 92-95 °C/5 mm Hg.
Anal. calc. for C8H8Cl2: C, 54.89; H, 4.61. Found: C, 54.80; H, 4.65.
1H NMR (CDCl3): δ 7.30 (d, J = 7.8 Hz, 1H, 5-H), 7.19 (s, 1H, 3-H), 7.04 (d, J = 7.8 Hz, 1H, 6-H), 4.64 (s, 2H, CH2Cl), 2.30 (s, 3H, Me). 13C{1H} NMR (CDCl3): δ 140.3, 133.7, 131.9, 130.6, 130.2, 127.9, 43.5, 20.8.

**3-(2-Chloro-4-methylphenyl)-2-methylpropanoyl chloride**

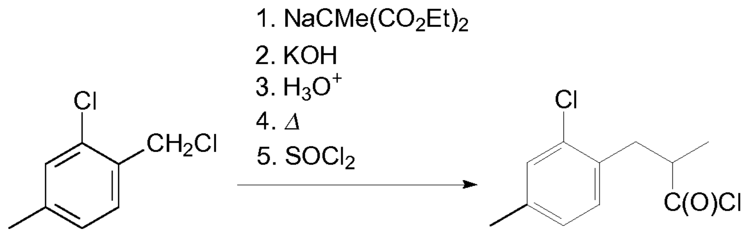


119 g (0.68 mol) of diethyl methylmalonate was added to a solution of sodium ethoxide obtained from 17.0 g (0.74 mol) of sodium metal and 600 ml of dry ethanol. The formed mixture was stirred for 15 min, and then 114 g (0.651 mol) of 2-chloro-1-(chloromethyl)-4-methylbenzene was added by vigorous stirring at such a rate to maintain a gentle reflux. The resulting mixture was refluxed for 2 h and then cooled to room temperature. A solution of 135 g of KOH in 550 ml of water was added. This mixture was refluxed for 4 h to saponificate the ester formed. Ethanol and water were distilled off until vapor temperature reached 95oC, and 3000 ml of water and then 12 M HCl (to pH~1) were added to the residue. The precipitated substituted methylmalonic acid was filtered off and washed with water. This diacid was dearboxylated at 160-180 °C to form a slightly orange oil crystallized at room temperature. A mixture of the formed acid and 166 ml of thionyl chloride was stirred for 24 h at room temperature. After evaporation of an excess of thionyl chloride, the residue was distilled under vacuum to give 123 g (82%) of the title product, b.p. 105-117oC/5 mm Hg.
Anal. calc. for C11H12Cl2O: C, 57.16; H, 5.23. Found: C, 57.36; H, 5.38.
1H NMR (CDCl3): δ 7.19 (s, 1H, 3-H), 7.10 (d, J = 7.7 Hz, 1H, 5-H), 7.00 (d, J = 7.7 Hz, 1H, 6-H), 3.20-3.32 (m, 2H, CHH' and CHMe), 2.82 (dd, J = 12.8 Hz, J = 6.4 Hz, 1H, CHH'), 2.30 (s, 3H, 4-Me), 1.30 (d, J = 6.8 Hz, 3H, CHMe). 13C{1H} NMR (CDCl3): δ 177.1, 138.6, 133.8, 132.1, 131.2, 130.2, 127.7, 51.4, 36.5, 20.7, 16.7.

**4-Chloro-2,6-dimethylindan-1-one**

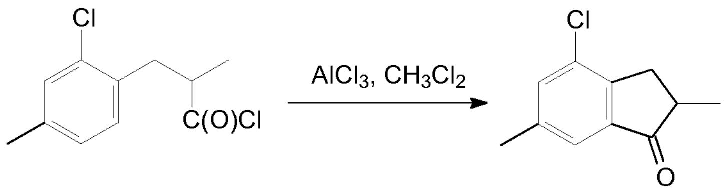


A solution of 123 g (531 mmol) of 3-(2-chloro-4-methylphenyl)-2-methylpropanoyl chloride in 100 ml of dichloromethane was added dropwise at 5°C to a stirred suspension of 85.0 g (638 mmol) of AlCl3 in 500 ml of dichloromethane This mixture was stirred overnight at room temperature and then poured on 500 g of crushed ice. The organic layer was separated, and the aqueous layer was extracted with 3 x 100 ml of dichloromethane. The combined organic extract was washed with aqueous K2CO3, dried over K2CO3, passed through a short pad of silica gel 60 (40-63 μm), and then evaporated to dryness. Crude product was distilled under vacuum to give 98.4 g (95%) of a colorless liquid, b.p. 131-132 °C/8 mm Hg.
Anal. calc. for C11H11ClO: C, 67.87; H, 5.70. Found: C, 68.01; H, 5.69.
1H NMR (CDCl3): δ 7.42 (s, 1H, 7-H), 7.38 (s, 1H, 5-H), 3.32 (dd, J = 17.3 Hz, J = 7.8 Hz, 1H, 3-CHH'), 2.68-2.76 (m, 1H, 2-H), 2.62 (dd, 1H, J = 17.3 Hz, J = 3.6 Hz, 3-CHH'), 2.38 (s, 3H, 6-Me), 1.31 (d, J = 7.5 Hz, 3H, 2-Me). 13C{1H} NMR (CDCl3): δ 208.2, 148.0, 139.3, 138.1, 135.0, 132.1, 122.2, 42.0, 33.3, 20.7, 16.1.

**4-Chloro-1-methoxy-2,6-dimethylindane**

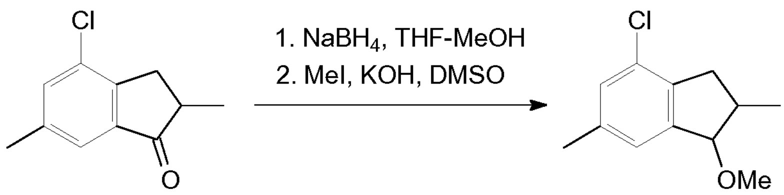


205 ml of methanol was added dropwise by vigorous stirring over 5 h at 0-5 °C to a mixture of 98.4 g (0.505 mol) of 4-chloro-2,6-dimethylindan-1-one and 29.0 g (0.767 mol) of NaBH4 in 510 ml of THF. This mixture was stirred overnight at room temperature and then evaporated to dryness. The residue was acidified by 2 M HCl to pH 5-6, and the formed 4-chloro-2,6-dimethylindan-1-ol was extracted with 3 x 300 ml of dichloromethane. The combined organic extract was dried over Na2SO4 and evaporated to dryness. The obtained white solid was dissolved in 800 ml of DMSO and 132 g (2.35 mol) of KOH and 163 g (1.15 mol) of MeI were added to it. This mixture was stirred for 5 h at ambient temperature. The solution was decanted from an excess of KOH, the latter was additionally washed with 3 x 350 ml of dichloromethane. The combined organic extract was washed with 3000 ml of water. The organic layer was separated, and the aqueous layer was extracted with 3 x 300 ml of dichloromethane. The combined organic extract was washed with 7 x 1500 ml of water, dried over Na2SO4, and then evaporated to dryness. The residue was distilled under vacuum to give 99.9 g (94%) of the title product consisting of two diastereomers, b.p. 104-105oC/8 mm Hg.
Anal. calc. for C12H15ClO: C, 68.40; H, 7.18. Found: C, 68.58; H, 7.25.
Syn-isomer. 1H NMR (CDCl3): δ 7.05 (s, 2H, 5-H and 7-H), 4.51 (d, J = 5.7 Hz, 1H, 1-H), 3.41 (s, 3H, OMe), 2.92 (dd, J = 15.3 Hz, J = 6.4 Hz, 1H, 3-CHH'), 2.68-2.59 (m, 2H, 3-CHH' and 2-H), 2.32 (s, 3H, 6-Me), 1.07 (d, J = 6.8 Hz, 3H, 2-Me). 13C{1H} NMR (CDCl3): δ 144.6, 138.3, 137.8, 130.7, 128.7, 124.1, 86.4, 57.0, 38.2, 36.9, 21.0, 13.5.
Anti-isomer. 1H NMR (CDCl3): δ 7.05 (s, 1H, 7-H), 7.07 (s, 1H, 5-H), 4.37 (d, J = 3.9 Hz, 1H, 1-H), 3.45 (s, 3H, OMe), 3.19 (dd, J = 16.2 Hz, J = 7.6 Hz, 1H, 3-CHH'), 2.50 (m, 1H, 2-H), 2.42 (dd, J = 16.2 Hz, J = 5.0 Hz, 1H, 3-CHH'), 2.32 (s, 3H, 6-Me), 1.16 (d, J = 6.8 Hz, 3H, 2-Me). 13C{1H} NMR (CDCl3): δ 144.2, 138.1 (two resonances), 130.7, 128.9, 124.2, 91.8, 56.6, 39.4, 37.2, 21.0, 19.3.

**4-(3,5-Di-tert-butylphenyl)-1-methoxy-2,6-dimethylindane**

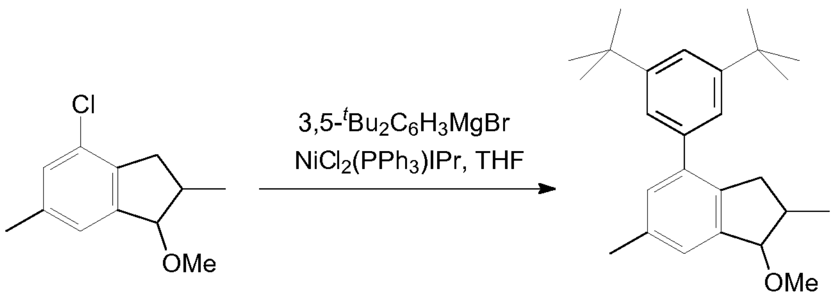


400 ml (200 mmol) of 0.5 M 3,5-di-tert-butylphenylmagnesium bromide in THF was added at room temperature to a mixture of 2.00 g (2.56 mmol, 1.74 mol.%) of NiCl2(PPh3)IPr and 31.1 g (148 mmol) of 4-chloro-1-methoxy-2,6-dimethylindane. The resulting mixture was refluxed for 3.5 h, then cooled to room temperature, followed by the addition of 100 ml of water. The major part of THF was distilled off on rotary evaporator. To the residue 400 ml of dichloromethane and 1000 ml of 1 M HCl were added. The organic layer was separated, the aqueous layer was extracted with 2 x 100 ml of dichloromethane. The combined organic extract was evaporated to dryness to give a slightly yellowish oil. The product was isolated by flash-chromatography on silica gel 60 (40-63 μm; eluent: hexanes-dichloromethane = 2:1, vol., then 1:1, vol.). This procedure gave a colorless thick oil containing two stereoisomers.
Anal. calc. for C26H36O: C, 85.66; H, 9.95. Found: C, 85.89; H, 10.13.
Syn-isomer. 1H NMR (CDCl3): δ 7.39 (t, J = 1.4 Hz, 1H, 4-H in 3,5-tBu2C6H3), 7.28 (d, J = 1.4 Hz, 2H, 2,6-H in 3,5-tBu2C6H3), 7.18 (s, 1H, 5-H in indane), 7.16 (s, 1H, 7-H in indane), 4.51 (d, J = 5.5 Hz, 1H, 1-H in indane), 3.47 (s, 3H, OMe), 2.91 (dd, J = 15.5 Hz, J = 7.1 Hz, 1H, 3-CHH' in indane), 2.79 (dd, J = 15.5 Hz, J = 6.8 Hz, 1H, 3-CHH' in indane), 2.45 (m, 1H, 2-H in indane), 2.41 (s, 3H, 6-Me in indane), 1.36 (s, 18H, 3,5-tBu2C6H3), 1.08 (d, J = 6.8 Hz, 3H, 2-Me in indane). 13C{1H} NMR (CDCl3): δ 150.4, 143.6, 140.0, 139.5, 138.3, 136.1, 129.2, 124.6, 123.0, 120.7, 86.3, 56.9,

39.2, 38.0, 34.9, 31.5, 21.3, 13.6.
Anti-isomer. 1H NMR (CDCl3): δ 7.39 (t, J = 1.6 Hz, 1H, 4-H in 3,5-tBu2C6H3), 7.28 (d, 2H, J = 1.6 Hz, 2,6-H in 3,5-tBu2C6H3), 7.19 (s, 1H, 5-H in indane), 7.17 (s, 1H, 7-H in indane), 4.39 (d, J = 3.6 Hz, 1H, 1-H in indane), 3.50 (s, 3H, OMe), 3.31 (dd, J = 15.3 Hz, J = 6.8 Hz, 1H, 3-CHH' in indane), 2.50 (m, 1H, 2-H in indane), 2.45 (dd, J = 15.3 Hz, J = 5.0 Hz, 1H, 3-CHH' in indane), 2.41 (s, 3H, 6-Me in indane), 1.36 (s, 18H, 3,5-tBu2C6H3), 1.10 (d, J = 6.8 Hz, 3H, 2-Me in indane). 13C{1H} NMR (CDCl3): δ 150.4, 142.9, 140.0, 139.6, 137.8, 136.4, 129.6, 124.7, 122.9, 120.7, 91.5, 56.6, 40.0, 38.1, 34.9, 31.5, 21.3, 19.1.

**7-(3,5-Di-tert-butylphenyl)-2,5-dimethyl-1H-indene**

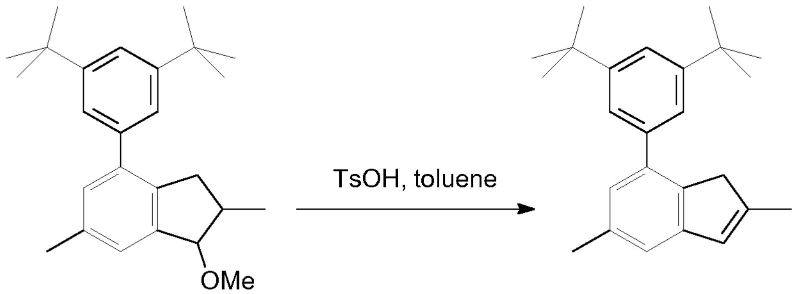


1.8 g of TsOH was added to a solution of 4-(3,5-di-tert-butylphenyl)-1-methoxy-2,6-dimethylindane (prepared above) in 500 ml of toluene, and the resulting solution was refluxed using Dean-Stark head for 15 min. After cooling to room temperature the reaction mixture was washed by 200 ml of 10% aqueous NaHCO3. The organic layer was separated, and the aqueous layer was additionally extracted with 2 x 100 ml of dichloromethane. The combined organic extract was evaporated to dryness, and the product was isolated by flash-chromatography on silica gel 60 (40-63 μm; eluent: hexanes-dichloromethane = 10:1, vol.). This procedure gave 47.4 g (97%) of white crystalline material.
Anal. calc. for C25H32: C, 90.30; H, 9.70. Found: C, 90.44; H, 9.51.
1H NMR (CDCl3): δ 7.40 (t, J = 1.8 Hz, 1H, 4-H in 3,5-tBu2C6H3), 7.36 (d, J = 1.8 Hz, 2H, 2,6-H in 3,5-tBu2C6H3), 7.07 (s, 1H, 6-H in indene), 6.97 (s, 1H, 4-H in indene), 6.48 (m, 1H, 3-H in indene), 3.34 (s, 2H, CH2 in indene), 2.42 (s, 3H, 5-Me in indene), 2.12 (s, 3H, 2-Me in indene), 1.37 (s, 18H, 3,5-tBu2C6H3). 13C{1H} NMR (CDCl3): δ 150.5, 146.7, 146.5, 140.6, 138.2, 138.0, 136.5, 127.1, 125.1, 122.8, 120.8, 119.5, 42.5, 34.9, 31.6, 21.5, 16.8.

**Bis[2,6-dimethyl-4-(3,5-di-tert-butylphenyl)-1H-inden-1-yl](dimethyl)silane**

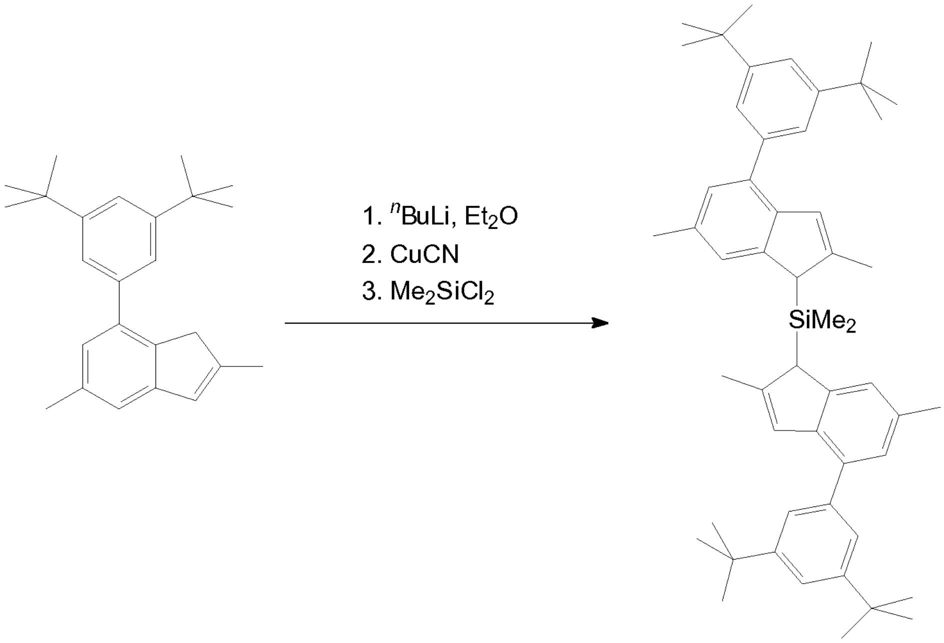


20.0 ml (50.0 mmol) of 2.5 M $^n$BuLi in hexanes was added in one portion to a solution of 16.6 g (50.0 mmol) of 7-(3,5-di-*tert*-butylphenyl)-2,5-dimethyl-1*H*-indene in 250 ml of ether at -50 °C. This mixture was stirred overnight at room temperature, then the resulting yellow suspension was cooled to -40 °C, and 200 mg of CuCN was added. The resulting mixture was stirred for 30 min at -25 °C, and 3.23 g (25.03 mmol) of dichlorodimethylsilane was added in one portion. Then this mixture was stirred overnight at ambient temperature. This solution was filtered through a pad of silica gel 60 (40-63 μm) which was additionally washed by 2 x 50 ml of dichloromethane. The combined filtrate was evaporated under vacuum, and the residue was dried in vacuo at elevated temperature. This procedure gave 18.4 g (~100%) of the title product of >90% purity (ca. 1:1 mixture of stereoisomers based on NMR analysis) as a yellowish glass which was used without further purification.

$^1$H NMR ($CDCl_3$): $\delta$ 7.53 (m), 7.48-7.52 (m), 7.37 (m), 7.31 (m), 7.25 (m), 7.23 (m), 6.91 (m), 6.89 (m), 3.90 (s), 2.543 (s), 2.538 (s), 2.37 (s), 2.34 (s), 1.51 (s), - 0.05 (s), -0.09 (2s).

**_Rac_-dimethylsilanediylbis[2,6-dimethyl-4-(3,5-di-*tert*-butylphenyl)-inden-1-yl]zirconium dichloride (I-mc1)**

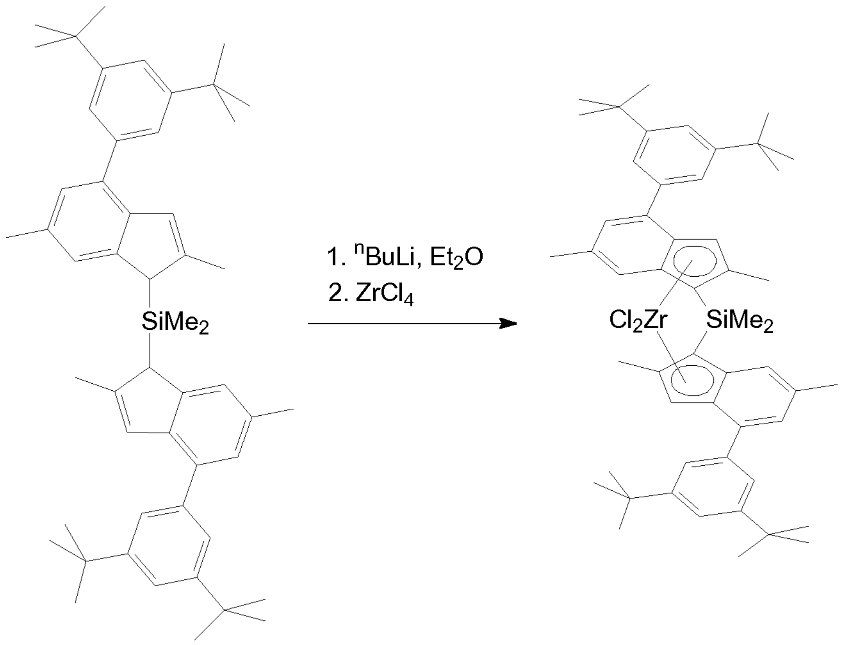


20.0 ml (50.0 mmol) of 2.5 M $^n$BuLi in hexanes was added in one portion to a solution of 18.4 g (25.0 mmol) of bis[4-(3,5-di-*tert*-butylphenyl)-2,6-dimethyl-1*H*-inden-1-yl](dimethyl)silane (as prepared above) in 250 ml of ether cooled to -30°°C. This mixture was stirred overnight at room temperature. The resulting light-orange solution was cooled to -50 °C, and 5.83 g (25.0 mmol) of $ZrCl_4$ $ZrCl_4$ was added. The reaction mixture was stirred for 24 h and then evaporated to dryness. The residue was poured into 200 ml of hot toluene. The obtained hot suspension was filtered through glass frit (G4). On the evidence of NMR spectroscopy, the filtrate included a ca. 1 to 1 mixture of *rac-* and *meso*-zirconocenes. This filtrate was evaporated to 110 ml and heated to dissolve a precipitate. Orange crystals precipitated from this solution overnight at room temperature were collected and dried in vacuum. This procedure gave a ca. 2 to 3 mixture of *rac-* and *meso*-zirconocenes. The mother liquor was evaporated to ca. 50 ml. Again, crystals precipitated from this solution at room temperature were collected and dried in vacuum. This procedure gave 1.72 g of pure *rac*-zirconocene. The mother liquor was evaporated to ca. 25 ml, heated almost to the boiling point, and 25 ml of n-hexane was added. Crystals precipitated from this solution at room temperature were collected and dried in vacuum. This procedure gave 1.68 g of a ca. 3 to 2 mixture *of rac-* and *meso*-zirconocenes. The above-described ca. 2 to 3 mixture of *rac-* and *meso*-complexes was re-crystallized from 80 ml of toluene. Orange crystals precipitated from this solution overnight at room temperature were collected and dried in vacuum. This procedure gave 0.98 g of pure *meso*-zirconocene. The mother liquor was evaporated to ca. 60 ml. Crystals precipitated from this solution overnight at room temperature were collected and dried in vacuo. This procedure gave 7.10 g of a ca. 7 to 3 mixture of *meso-* and *rac*-zirconocenes. Again, the mother liquor was evaporated to ca. 40 ml. Crystals precipitated from this solution overnight at room temperature were collected and dried in vacuum. This procedure gave 2.10 g of pure *rac*-zirconocene. Thus, the total yield of *rac-* and *meso*-zirconocenes isolated in this reaction was 13.6 g (62%).
*Rac*-complex (I-mc1).
Anal. calc. for $C_{52}H_{66}Cl_2SiZr$: C, 70.87; H, 7.55. Found: C, 71.05; H, 7.69.
$^1$H NMR ($CDCl_3$): $\delta$ 7.52 (m, 4H), 7.39 (m, 4H), 7.24 (m, 2H), 6.91 (m, 2H), 2.38 (s, 6H), 2.25 (s, 6H), 1.33 (s, 6H), 1.32 (s, 36H). $^{13}C\{^1H\}$ NMR ($CDCl_3$): $\delta$ 151.0, 139.4, 138.6, 135.7, 134.8, 130.6, 128.9, 128.4, 123.3, 122.8, 122.5, 121.4, 82.2, 35.1, 31.5, 22.2, 18.4, 2.8.
*Meso*-complex (meso- I-mc1).
Anal. found: C, 71.16; H, 7.72.
$^1$H NMR ($CDCl_3$): $\delta$ 7.46 (m, 4H), 7.39 (m, 2H), 7.37 (m, 2H), 6.96 (m, 2H), 6.73 (m, 2H), 2.42 (s, 6H), 2.26 (s, 6H), 1.48 (s, 3H), 1.33 (s, 36H), 1.22 (s, 3H). $^{13}C\{^1H\}$ NMR ($CDCl_3$): $\delta$ 150.6, 138.7, 138.2, 136.1, 134.2, 133.1, 129.2, 128.1, 123.8, 123.2, 121.3, 120.6, 83.6, 35.0, 31.5, 22.0, 18.8, 3.0.

**Synthesis of I-mc2** : **3-Bromo-4-methylacetophenone**

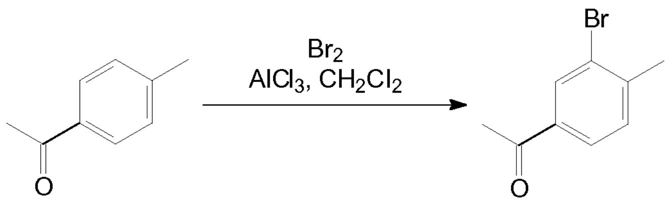


A solution of 97 g (723 mol) of 4-methylacetophenone in 100 ml of dichloromethane was added by vigorous stirring over 30 min to a suspension of 237 g (1.78 mol) of $AlCl_3$ in 700 ml of dichloromethane at 0 °C. The reaction mixture was then stirred for 10 min at this temperature, then 40.0 ml (125 g, 0.781 mol, 8% excess) of bromine was added dropwise over 1 h. The resulting mixture was stirred overnight at room temperature and then poured onto 2000 $cm^3$ of ice. The organic layer was separated, and the aqueous layer was extracted with 3 x 150 ml of dichloromethane. The combined organic extract was washed with aqueous $K_2CO_3$, dried over $K_2CO_3$, passed through a short pad of silica gel 60 (40-63 μm), and then evaporated to dryness. Crude product was distilled under vacuum to give 143 g (93%) of a colorless liquid (b.p. 125-132 °C/8 mm Hg) which completely solidified on standing at room temperature.
Anal. calc. for $C_9H_9BrO$: C, 50.73; H, 4.26. Found: C, 50.89; H, 4.12.
$^1H$ NMR ($CDCl_3$): $\delta$ 8.09 (s, 1H, 2-H), 7.77 (d, $J$ = 7.8 Hz, 1H, 6-H), 7.31 (d, $J$ = 7.8 Hz, 1H, 5-H), 2.56 (s, 3H, COMe), 2.44 (s, 3H, 4-Me).

**1-[3-Bromo-4-(bromomethyl)phenyl] ethanone**

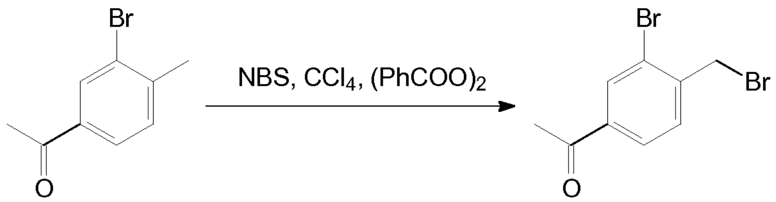


222 g (1.25 mol) of N-bromosuccinimide and 2 g of benzoyl peroxide were added to a solution of 265 g (1.24 mol) of 3-bromo-4-methylacetophenone in 2000 ml of tetrachloromethane. The formed mixture was refluxed for 2 h, then filtered through glass frit (G3), and the filtrate was evaporated to dryness. The residue was dried in vacuo to yield crude 4-acetyl-2-bromobenzyl bromide as red oil (purity ~70%) which was used without further purification.
$^1H$ NMR ($CDCl_3$): $\delta$ 8.12 (s, 1H, 2-H), 7.85 (d, $J$ = 7.8 Hz, 1H, 6-H), 7.54 (d, $J$ = 7.8 Hz, 1H, 5-H), 4.59 (s, 2H, $CH_2Br$), 2.58 (s, 3H, COMe).

**Diethyl (4-acetyl-2-bromobenzyl)(methyl)malonate**

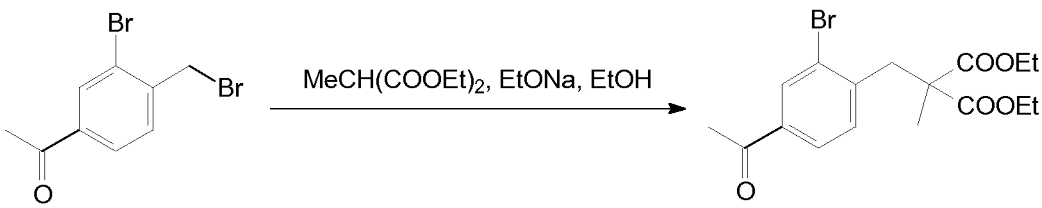


225 g (1.29 mol) of diethyl methylmalonate was added to a solution of sodium ethoxide prepared from 27.4 g (1.19 mol) of sodium metal and 1000 ml of dry ethanol. This mixture was stirred for 15 min; then, 4-acetyl-2-bromobenzyl bromide (prepared above) was added at such a rate as to maintain a gentle reflux. The obtained mixture was refluxed for 3 h, and then ethanol was distilled off at atmospheric pressure. The reaction mixture was cooled to room temperature, 2500 ml of water was added, and crude product was extracted with 3 x 200 ml of ether. The combined organic extract was dried over $Na_2SO_4$, passed through a short layer of silica gel 60 (40-63 μm), and then evaporated to dryness. The residue was distilled under vacuum to give 217 g (45%) of diethyl (4-acetyl-2-bromobenzyl)(methyl)malonate (b.p.

180-200 °C/5 mm Hg) as a slightly yellowish oil which completely solidified on standing at room temperature.
Anal. calc. for $C_{17}H_{21}BrO_5$: C, 53.00; H, 5.49. Found: C, 53.22; H, 5.25.
$^1H$ NMR ($CDCl_3$): $\delta$ 8.13 (s, 1H, 3-H), 7.78 (d, *J* = 7.9 Hz, 1H, 5-H), 7.30 (d, *J* = 7.9 Hz, 1H, 6-H), 4.23 (m, 4H, OC*H*$_2$Me), 3.54 (s, 2H, ArC*H*$_2$), 2.57 (s, 3H, C(O)Me), 1.39 (s, 3H, *Me*C(COOEt)$_2$), 1.26 (t, *J* = 7.1 Hz, 6H, OCH$_2$*Me*). $^{13}C\{^1H\}$ NMR ($CDCl_3$): $\delta$ 195.9, 171.2, 141.6, 136.9, 132.6, 131.3, 126.7, 126.4, 61.4, 54.6, 39.2, 26.3, 19.3, 13.7.

**3-(2-Bromo-4-ethylphenyl)-2-methylpropanoic acid**

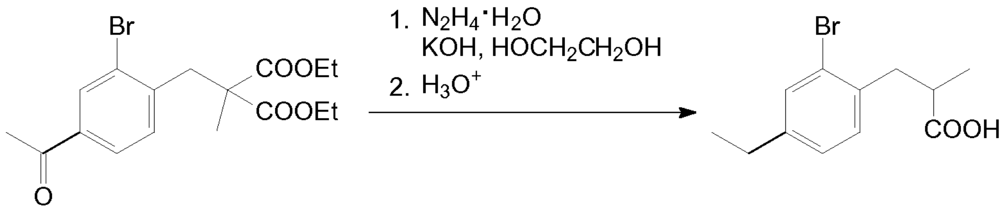


125 g of KOH was added in small portions by vigorous stirring over 30 min to a warm turbid mixture of 141 g (366 mmol) of diethyl (4-acetyl-2-bromobenzyl)(methyl)malonate, 63 ml of hydrazine hydrate and 1000 ml of ethylene glycol. The resulting slightly yellowish solution was refluxed for 5 h. Further on, the reflux condenser was replaced by a Claisen distillation head with condenser, and a mixture of water, hydrazine, and ethylene glycol was distilled off until the distillation temperature reached 195°C (eventually this reaction mixture was thus evaporated to about half its initial volume in 1 h). The residue was cooled to ca. 100 °C and then poured into 2500 ml of cold water. This mixture was acidified by 12 M HCl to pH~1, and crude product was extracted with 500 ml of dichloromethane. The aqueous layer was additionally extracted with 2 x 250 ml of dichloromethane. The combined organic extract was washed with 1000 ml of water and then evaporated to dryness. The residue was distilled under reduced pressure to give 60.2 g (61%) of 3-(2-bromo-4-ethylphenyl)-2-methylpropanoic acid (b.p. 144-172 °C/5 mm Hg) as a colorless oily liquid.
Anal. calc. for $C_{12}H_{15}BrO_2$: C, 53.15; H, 5.58. Found: C, 53.28; H, 5.74.
$^1H$ NMR ($CDCl_3$): $\delta$ 11.99 (s, 1H, $CO_2H$), 7.38 (s, 1H, 3-H), 7.12 (d, *J* = 7.8 Hz, 1H, 5-H), 7.04 (d, *J* = 7.8 Hz, 1H, 6-H), 3.15 (dd, *J* = 13.5 Hz, *J* = 6.8 Hz, 1H, ArC*H*H'), 2.85-2.94 (m, 1H, C*H*(Me)$CO_2H$), 2.76 (dd, *J* = 13.5 Hz, *J* = 7.7 Hz, 1H, ArC*H*H'), 2.58 (q, *J* = 7.5 Hz, 2H, C*H*$_2$Me), 1.19-1.22 (m, 6H, CH*Me* and CH$_2$*Me*). $^{13}C\{^1H\}$ NMR ($CDCl_3$): $\delta$ 182.6, 144.5, 135.4, 132.2, 131.1, 126.9, 124.5, 39.5, 38.8, 28.0, 16.6, 15.2.

**3-(2-Bromo-4-ethylphenyl)-2-methylpropanoyl chloride**

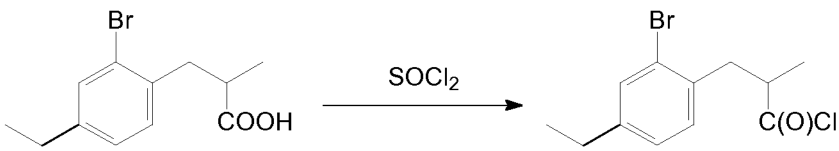


A mixture of 60.2 g (0.222 mol) of 3-(2-bromo-4-ethylphenyl)-2-methylpropanoic acid and 57 ml (0,781 mol) of thionyl chloride was stirred at room temperature for 24 h. Then the excess of thionyl chloride was distilled off, and the residue was distilled under vacuum to give 53.1 g (83%) of the title product, b.p. 132-133°C/5 mm Hg.
Anal. calc. for $C_{12}H_{14}BrClO$: C, 49.77; H, 4.87. Found: C, 49.85; H, 5.00.
$^1H$ NMR ($CDCl_3$): $\delta$ 7.39 (s, 1H, 3-H), 7.13 (d, *J* = 7.8 Hz, 1H, 5-H), 7.07 (d, *J* = 7.8 Hz, 1H, 6-H), 3.19-3.36 (m, 2H, ArC*H*H' and C*H*(COCl)), 2.81 (dd, *J* = 13.2 Hz, *J* = 6.8 Hz, 1H, ArC*H*H'), 2.60 (q, *J* = 7.6 Hz, 2H, C*H*$_2$Me), 1.31 (d, *J* = 6.8 Hz, 3H, CH*Me*), 1.21 (t, *J* = 7.6 Hz, 3H, CH$_2$*Me*). $^{13}C\{^1H\}$ NMR ($CDCl_3$): $\delta$ 177.0, 145.1, 134.0, 132.4, 131.3, 127.1, 124.4, 51.4, 38.9, 28.0, 16.7, 15.2.

**4-Bromo-6-ethyl-2-methylindan-1-one**

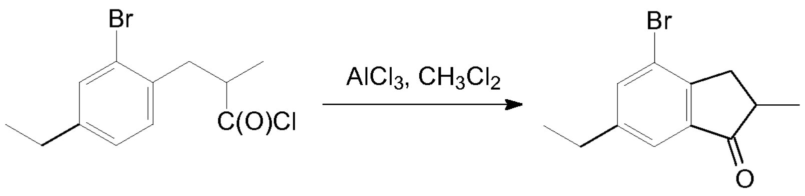


*A solution of 53.1 g (183 mmol*) of 3-(2-bromo-4-ethylphenyl)-2-methylpropanoyl chloride in 50 ml of dichloromethane was added dropwise to a stirred suspension of 30.0 g (225 mmol, 1.2 eq.) of $AlCl_3$ in 200 ml of dichloromethane at 5°C. This mixture was stirred overnight at room temperature and then poured on 500 g of crushed ice. The organic layer was separated, and the aqueous layer was extracted with 2 x 100 ml of dichloromethane. The combined organic extract was washed by aqueous $K_2CO_3$, dried over $K_2CO_3$, and then evaporated to dryness. The product was isolated by flash-chromatography on silica gel 60 (40-63 μm; eluent: hexanes-dichloromethane = 1:1, vol.). This procedure gave 43.8 g (95%) of a yellowish solid.
Anal. calc. for $C_{12}H_{13}BrO$: C, 56.94; H, 5.18. Found: C, 57.19; H, 5.36.
$^1H$ NMR ($CDCl_3$): $\delta$ 7.61 (s, 1H, 7-H), 7.53 (s, 1H, 5-H), 3.30 (dd, *J* = 17.4 Hz, *J* = 7.8 Hz, 1H, 3-C*H*H'), 2.65-2.80 (m, 3H, 2-H and C*H*$_2$Me), 2.61 (dd, *J* = 17.4 Hz, *J* = 3.6 Hz, 1H, 3-C*H*H'), 1.32 (d, *J* = 7.5 Hz, 3H, 2-Me), 1.25 (t, *J* = 7.5 Hz, 3H, $CH_2$*Me*). $^{13}C\{^1H\}$ NMR ($CDCl_3$): $\delta$ 208.6, 150.5, 146.0, 138.3, 137.3, 121.7, 121.7, 42.3, 35.5, 28.2, 16.2, 15.3.

**4-Bromo-6-ethyl-1-methoxy-2-methylindane**

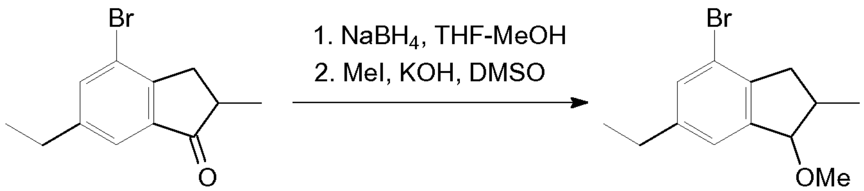


150 ml of methanol was added dropwise by vigorous stirring over 5 h to a mixture of 43.8 g (173 mmol) of 4-bromo-6-ethyl-2-methylindan-1-one and 9.8 g (259 mmol) of $NaBH_4$ in 300 ml of THF at 0-5 °C. This mixture was stirred overnight at room temperature and then evaporated under vacuum. 500 ml of dichloromethane and 500 ml of water were added to the obtained white mass, and the resulting mixture was acidified by 2 M HCl to pH~4. The organic layer was separated, and the aqueous layer was extracted with 2 x 200 ml of dichloromethane. The combined organic extract was dried over $Na_2SO_4$ and then evaporated to dryness. The resulting white solid was immediately dissolved in 370 ml of DMSO, and 45 g (802 mmol, 4.64 eq.) of KOH and 55.5 g (391 mmol, 2.26 eq.) of MeI were added. The formed mixture was stirred overnight at room temperature, then the solution was decanted from an excess of KOH, the latter was washed with 3 x 150 ml of dichloromethane, and 1500 ml of water was added to the combined extract. The organic layer was separated, and the aqueous layer was extracted with 2 x 150 ml of dichloromethane. The combined organic extract was washed with 3 x 1000 ml of water, dried over $K_2CO_3$, and evaporated to dryness. The product was isolated by flash-chromatography on silica gel 60 (40-63 μm; eluent: hexanes-dichloromethane = 2:1, vol., then 1:1, vol.). This procedure gave 40.5 g (87%) of a colorless thick oil composed of a mixture of two diastereoisomers.
Anal. calc. for $C_{13}H_{17}BrO$: C, 58.01; H, 6.37. Found: C, 58.22; H, 6.49.
*Syn*-isomer: $^1H$ NMR ($CDCl_3$): $\delta$ 7.24 (s, 1H, 5-H), 7.12 (s, 1H, 7-H), 4.55 (d, *J* = 5.7 Hz, 1H, 1-H), 3.41 (s, 3H, OMe), 2.90 (dd, *J* = 17.6 Hz, *J* = 8.9 Hz, 1H, 3-C*H*H'), 2.56-2.70 (m, 4H, 3-CH*H*', 2-H and C*H*$_2$Me), 1.22 (t, *J* = 7.5 Hz, 3H, $CH_2$*Me*), 1.07 (d, *J*= 6.6 Hz, 3H, 2-Me). $^{13}C\{^1H\}$ NMR ($CDCl_3$): $\delta$ 144.6, 144.5, 140.7, 130.6, 123.5, 120.1, 86.7, 57.0, 39.0, 37.9, 28.4, 15.7, 13.5.
Anti-isomer: $^1H$ NMR ($CDCl_3$): $\delta$ 7.26 (s, 1H, 5-H), 7.13 (s, 1H, 7-H), 4.42 (d, *J*= 4.3 Hz, 1H, 1-H), 3.45 (s, 3H, OMe), 3.18 (dd, *J* = 16.2 Hz, *J* = 7.5 Hz, 1H, 3-C*H*H'), 2.62 (q, *J* = 7.5 Hz, 2H, C*H*$_2$Me), 2.44-2.56 (m, 1H, 2-H), 2.40 (dd, *J* = 16.2 Hz, *J* = 5.2 Hz, 1H, 3-CH*H*'), 1.22 (t, *J* = 7.5 Hz, 3H, $CH_2$*Me*), 1.17 (d, *J* = 7.1 Hz, 3H, 2-Me). $^{13}C\{^1H\}$ NMR ($CDCl_3$): $\delta$ 144.9, 144.1, 140.5, 130.9, 123.6, 120.1, 92.0, 56.6, 39.3, 39.2, 28.4, 19.4, 15.6.

**4-(3,5-Di-*tert*-butylphenyl)-6-ethyl-1-methoxy-2-methylindane**

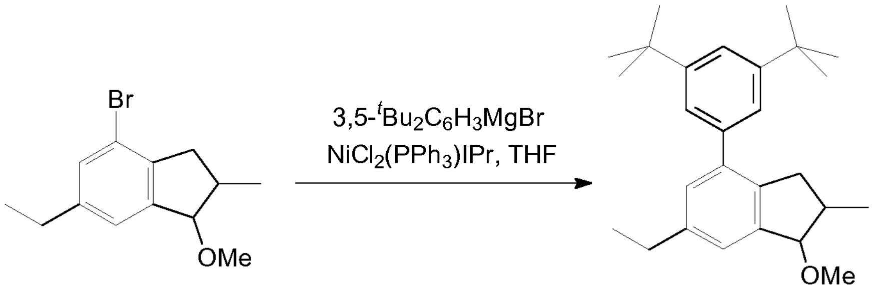


400 ml (200 mmol) of 0.5 M 3,5-di-*tert*-butylphenylmagnesium bromide in THF was added to a mixture of 1.0 g (1.28 mmol, 0.85 mol.%) of $NiCl_2(PPh_3)IPr$ and 40.5 g (151 mmol) of 4-bromo-6-ethyl-1-methoxy-2-methylindane, at such a rate as to maintain a gentle reflux. The resulting solution was refluxed for 1 h, then cooled to room temperature, and 150 ml of water was added. The major part of THF was distilled off using rotary evaporator, then 500 ml of dichloromethane and 1000 ml of 1 M HCl were added to the residue. The organic layer was separated, and the aqueous layer was extracted with 150 ml of dichloromethane. The combined organic extract was evaporated to dryness to give ca. 68 g of a slightly greenish oil. The product was isolated by flash-chromatography on silica gel 60 (40-63 μm; eluent: hexanes-dichloromethane = 2:1 vol., then 1:1 vol.). This procedure gave a colourless thick oil composed of a mixture of two diastereoisomers.

Anal. calc. for $C_{27}H_{38}O$: C, 85.66; H, 10.12. Found: C, 85.92; H, 10.37.

*Syn*-isomer: $^{1}H$ NMR ($CDCl_3$): $\delta$ 7.39 (t, *J* = 1.6 Hz, 1H, 4-H in 3,5-$^{t}Bu_2C_6H_3$), 7.28 (d, *J*= 1.6 Hz, 2H, 2,6-H in 3,5-$^{t}Bu_2C_6H_3$), 7.20 (s, 1H, 5-H in indane), 7.18 (s, 1H, 7-H in indane), 4.53 (d, *J* = 5.5 Hz, 1H, 1-H in indane), 3.48 (s, 3H, OMe), 2.92 (dd, *J* = 15.5 Hz, *J* = 7.1 Hz, 1H, 3-C*H*H' in indane), 2.79 (dd, *J* = 15.5 Hz, *J* = 6.6 Hz, 1H, 3-CH*H'* in indane), 2.71 (q, *J* = 7.5 Hz, 2H, C$H_2$Me), 2.50-2.63 (m, 1H, 2-H in indane), 1.36 (s, 18H, 3,5-$^{t}Bu_2C_6H_3$), 1.29 (t, *J* = 7.5 Hz, 3H, $CH_2$*Me*), 1.08 (d, *J* = 6.8 Hz, 3H, 2-Me in indane). $^{13}C\{^{1}H\}$ NMR ($CDCl_3$): $\delta$ 150.4, 143.57, 142.7, 140.1, 139.6, 138.6, 128.2, 123.3, 123.0, 120.7, 86.4, 56.9, 39.2, 38.0, 34.9, 31.5, 28.8, 15.9, 13.6.

Anti-isomer: $^{1}H$ NMR ($CDCl_3$): $\delta$ 7.39 (t, *J*= 1.8 Hz, 1H, 4-H in 3,5-$^{t}Bu_2C_6H_3$), 7.28 d, *J* = 1.8 Hz, 2H, 2,6-H in 3,5-$^{t}Bu_2C_6H_3$), 7.21 (s, 1H, 5-H in indane), 7.18 (s, 1H, 7-H in indane), 4.41 (d, *J* = 3.9 Hz, 1H, 1-H in indane), 3.51 (s, 3H, OMe), 3.30 (dd, *J* = 15.3 Hz, *J* = 6.8 Hz, 1H, 3-C*H*H' in indane), 2.71 (q, *J* = 7.5 Hz, 2H, C$H_2$Me), 2.40-2.55 (m, 2H, 2-Me and 3-CH*H'* in indane), 1.36 (s, 18H, 3,5-$^{t}Bu_2C_6H_3$), 1.28 (t, *J* = 7.5 Hz, 3H, $CH_2$*Me*), 1.12 (d, *J* = 6.8 Hz, 3H, 2-Me in indane). $^{13}C\{^{1}H\}$ NMR ($CDCl_3$): $\delta$ 150.4, 143.0 (two resonances), 140.1, 139.6, 138.1, 128.5, 123.4, 122.9, 120.7, 91.5, 56.7, 40.0, 38.1, 34.9, 31.5, 28.8, 19.2, 15.9.

**2-methylMethyl-5-ethyl-7-(3,5-Di-*tert*-butylphenyl)-1*H*-indene**

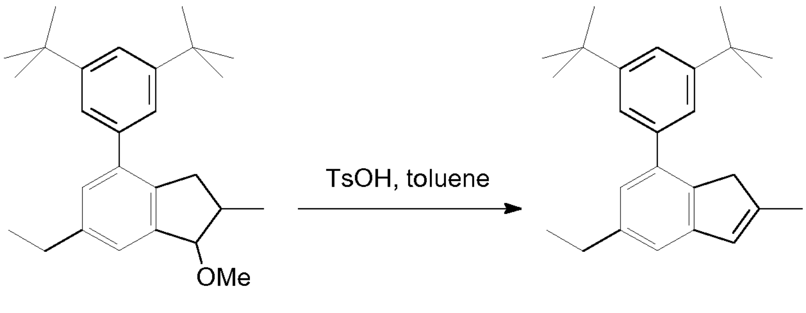


0.75 g of TsOH was added to a solution of 4-(3,5-di-*tert*-butylphenyl)-6-ethyl-1-methoxy-2-methylindane (as prepared above) in 450 ml of toluene. The resulting solution was refluxed using Dean-Stark head for 15 min, then cooled to room temperature, and finally washed with 200 ml of 10% aqueous $NaHCO_3$. The organic layer was separated, and the aqueous layer was extracted with 150 ml of dichloromethane. The combined organic extract was evaporated to dryness, and the product was isolated by flash-chromatography on silica gel 60 (40-63 μm; eluent: hexanes-dichloromethane = 10:1, vol.). This procedure gave 52.0 g (99%) of crude 7-(3,5-di-*tert*-butylphenyl)-5-ethyl-2-methyl-1*H*-indene as a white crystalline solid. This material was re-crystallized from 100 ml of n-hexane to give 43.8 g of a white crystalline product. The following crystallization of the evaporated mother liquor from 10 ml of n-hexane gave the second crop (6.30 g) of the analytically pure product.

Anal. calc. for $C_{26}H_{34}$: C, 90.11; H, 9.89. Found: C, 90.35; H, 10.05.
$^1$H NMR ($CDCl_3$): $\delta$ 7.41 (t, *J* = 1.8 Hz, 1H, 4-H in 3,5-$^tBu_2C_6H_3$), 7.37 (d, *J* = 1.8 Hz, 2H, 2,6-H in 3,5-$^tBu_2C_6H_3$), 7.10 (s, 1H, 6-H in indene), 7.00 (s, 1H, 4-H in indene), 6.50 (q, *J* = 1.3 Hz, 1H, 3-H in indene), 3.34 (s, 2H, $CH_2$ in indene), 2.72 (q, *J* = 7.6 Hz, 2H, C*$H_2$*Me), 2.13 (s, 3H, 2-Me in indene), 1.38 (s, 18H, 3,5-*$^tBu_2$*$C_6H_3$), 1.29 (t, *J* = 7.6 Hz, 3H, $CH_2$*Me*). $^{13}C\{^1H\}$ NMR ($CDCl_3$): $\delta$ 150.5, 146.7, 146.5, 143.2, 140.7, 138.3, 138.3, 127.2, 124.1, 122.8, 120.8, 118.3, 42.5, 35.0, 31.6, 29.0, 16.8, 16.1.

**Bis[2-methyl-4-(3,5-di-tert-butylphenyl)-6-ethyl-1H-inden-1-yl](dimethyl)silane**

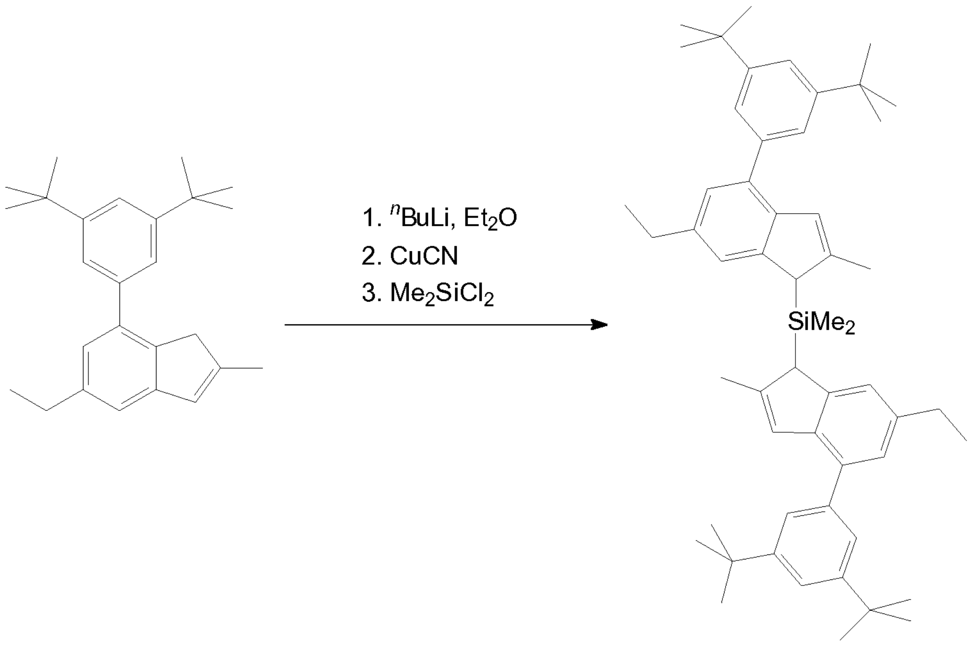


20.0 ml (50.0 mmol) of 2.5 M $^n$BuLi in hexanes was added in one portion to a solution of 17.3 g (50.0 mmol) of 2-methyl-7-(3,5-di-*tert*-butylphenyl)-5-ethyl-1*H*-indene in 250 ml of ether at -50 °C. This mixture was stirred overnight at room temperature, then the resulting yellow solution was cooled to -40 °C, and 200 mg of CuCN was added. The obtained mixture was stirred for 30 min at -25 °C, and then 3.23 g (25.0 mmol) of dichlorodimethylsilane was added in one portion. Then this mixture was stirred overnight at ambient temperature, then filtered through a pad of silica gel 60 (40-63 μm) which was additionally washed by 2 x 50 ml of dichloromethane. The combined filtrate was evaporated under vacuum, and the residue was dried in vacuo at elevated temperature. This procedure gave 18.9 g (~100%) of the title compound of >90% purity. On the evidence of NMR spectroscopy this product is a ca. 1:1 mixture of the stereoisomers. The obtained yellowish glassy product was used without further purification.
$^1$H NMR ($CDCl_3$): $\delta$ 7.44-7.53 (m), 7.39 (m), 7.31 (m), 7.23 (m), 7.22 (m), 6.88 (m), 6.87 (m), 3.87 (s), 2.81 (q, *J* = 7.4 Hz), 2.33 (s), 2.32 (s), 1.48 (s), 1.36 (t, *J* = 7.4 Hz), -0.07 (s), -0.12 (s).

***Rac*-dimethylsilanediylbis[2-methyl-4-(3,5-di-*tert*-butylphenyl)-6-ethyl-inden-1-yl]zirconium dichloride (I-mc2)**

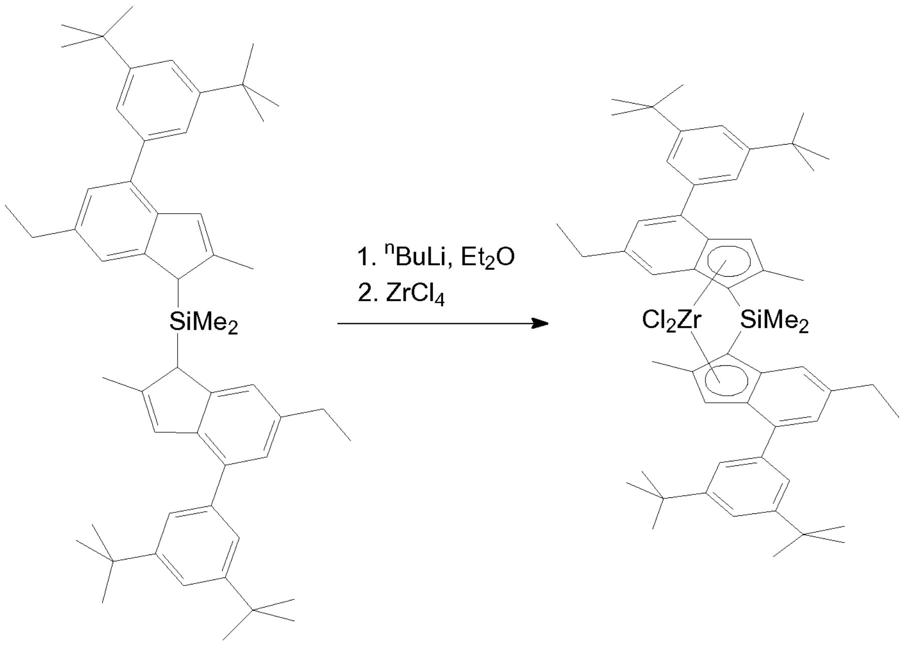


20.0 ml (50.0 mmol) of 2.5 M $^n$BuLi in hexanes was added in one portion to a solution of 18.9 g (25.0 mmol) of bis[2-methyl-4-(3,5-di-*tert*-butylphenyl)-6-ethyl-1*H*-inden-1-yl](dimethyl)silane (as prepared above) in 250 ml of ether cooled to -30 °C. This mixture was stirred overnight at room temperature. The resulting light-orange solution was cooled to -50 °C, and 5.83 g (25.0 mmol) of $ZrCl_4$ was added. The reaction mixture was stirred for 24 h giving yellow-orange suspension which was then evaporated to dryness. The residue was poured into 200 ml of hot toluene. The obtained hot mixture was filtered through glass frit (G4). On the evidence of NMR spectroscopy the obtained filtrate included a ca. 1 to 1 mixture of *rac*- and *meso*-zirconocenes. Yellow crystals precipitated from this filtrate overnight at room temperature were collected and dried in vacuum. This procedure gave 2.80 g of pure *rac*-zirconocene. The mother liquor was evaporated to ca. 75 ml. Crystals precipitated from this filtrate overnight at room temperature were collected and dried in vacuo. This procedure gave 9.07 g of a ca. 55 to 45 mixture *of rac*- and *meso*-zirconocenes. The mother liquor was evaporated to ca. 15 ml. Crystals precipitated from this filtrate overnight at room temperature were collected and dried in vacuo. This procedure gave 1.73 g of *meso*-zirconocene contaminated with ca. 1% of *rac*-isomer. The mother liquor was evaporated to dryness. To the residue ca. 25 ml of n-hexane was added. Crystals precipitated from this solution for 2 h at room temperature were collected and dried in vacuo. This procedure gave 3.36 g of a ca. 3 to 7 mixture *of rac*- and *meso*-zirconocenes. Thus, the overall yield of *rac*- and *meso*-zirconocenes isolated in this synthesis was 17.0 g (75%).
*Rac*-zirconocene (I-mc2).
Anal. calc. for $C_{54}H_{70}Cl_2SiZr$: C, 71.32; H, 7.76. Found: C, 71.58; H, 7.90.
$^1H$ NMR ($CDCl_3$): $\delta$ 7.52 (d, *J* = 1.8 Hz, 4H), 7.42 (m, 2H), 7.39 (t, *J* = 1.8 Hz, 2H), 7.27 (m, 2H), 6.91 (m, 2H), 2.69 (q, *J* = 7.4 Hz, 4H), 2.26 (s, 6H), 1.34 (s, 6H), 1.32 (s, 36H), 1.24 (t, *J* = 7.4 Hz, 6H). $^{13}C\{^1H\}$ NMR ($CDCl_3$): $\delta$ 150.94, 141.94, 139.46, 138.66, 134.74, 130.86, 128.45, 128.21, 123.29, 122.71, 121.44, 121.23, 82.24, 35.05, 31.55, 29.43, 18.50, 15.07, 2.88.
*Meso*-zirconocene (meso I-mc2).
Anal. found: C, 71.43; H, 7.89.
$^1H$ NMR ($CDCl_3$): $\delta$ 7.48 (m, 4H), 7.39 (m, 4H), 7.00 (m, 2H), 6.75 (m, 2H), 2.55 (m, 4H), 2.43 (s, 6H), 1.48 (s, 3H), 1.33 (s, 36H), 1.22 (s, 3H), 1.17 (t, *J* = 7.4 Hz, 6H). $^{13}C\{^1H\}$ NMR ($CDCl_3$): $\delta$ 150.68, 140.68, 138.74, 138.43, 135.81, 133.20, 129.15, 127.06, 123.22, 122.08, 121.38, 121.30, 83.95, 35.01, 31.52, 29.40, 18.67, 14.99, 3.04, 2.94.

**I-mc3:**

**1-(Chloromethyl)-4-isopropylbenzene**

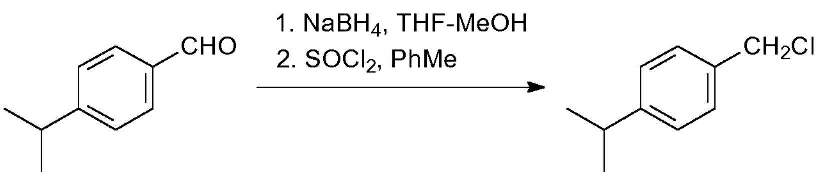


500 ml of methanol was added dropwise by vigorous stirring over 5 h to a mixture of 148 g (1.0 mol) 4-isopropylbenzaldehyde and 37.8 g (1.0 mol) of $NaBH_4$ in 1000 ml of THF at 0-5 °C. This mixture was stirred overnight at room temperature and then evaporated under vacuum. The residue was acidified with 1200 ml of 2 M HCl to pH~1, and the formed (4-isopropylphenyl)methanol was extracted with 3 x 400 ml of dichloromethane. The combined organic extract was dried over $Na_2SO_4$ and evaporated to dryness. To the residue dissolved in 1000 ml of dichloromethane 73 ml (1.0 mol) of thionyl chloride was added dropwise at +5°C. The resulting solution was stirred at room temperature overnight, evaporated to dryness, and then the residue was dissolved in 750 ml dichloromethane. The formed solution was washed by 250 ml of water. The organic layer was separated, the aqueous layer was extracted with 2 x 150 ml of dichloromethane. The combined organic extract was dried over $Na_2SO_4$, passed through a short pad of silica gel 60 (40-63 μm), and evaporated to dryness. Crude product was distilled under vacuum to give 142 g (84%) of a colorless liquid, b.p. 107-112 °C/15 mm Hg.
Anal. calc. for $C_{10}H_{13}Cl$: C, 71.21; H, 7.77. Found: C, 71.25; H, 7.63.
$^1H$ NMR ($CDCl_3$): $\delta$7.31 (d, $J$ = 8.1 Hz, 2H), 7.21 (d, $J$ = 8.1 Hz, 2H), 4.56 (s, 2H), 2.96-2.85 (m, 1H), 1.24 (d, $J$ = 6.8 Hz, 6H).

**3-(4-Isopropylphenyl)-2-methylpropanoyl chloride**

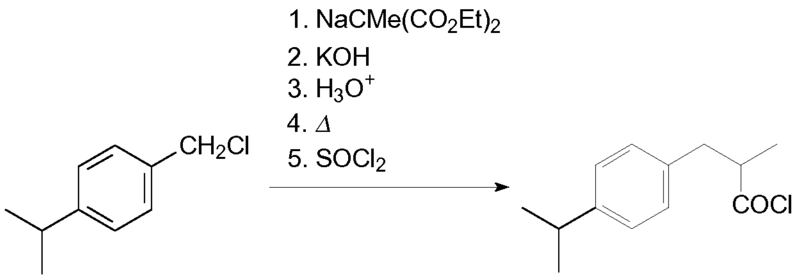


120 g (0.691 mol) of diethyl methylmalonate was added to a solution of sodium ethoxide prepared from 19.0 g (0.827 mol) of sodium metal and 500 ml of anhydrous ethanol. This mixture was stirred for 15 min, then 117 g (0.691 mmol) of 1-(chloromethyl)-4-isopropylbenzene was added at such a rate as to maintain a gentle reflux. This mixture was refluxed for 3 h and then cooled to room temperature. A solution of 138 g of KOH in 370 ml of water was added. The obtained mixture was refluxed for 4 h to saponificate the ester formed. Ethanol and water were distilled off until the temperature reached 95 °C, and 2000 ml of water and then 12 M HCl (to pH~1) were added to the residue. The precipitated substituted methylmalonic acid was filtered off, washed with water, and partially dried on the filter. Crude 3-(4-isopropylphenyl)-2-methylpropanoic acid was obtained after decarboxylation of this substituted methylmalonic acid at 180°C. A mixture of this acid and 175 ml of thionyl chloride was stirred at room temperature for 24 h, and then an excess of thionyl chloride was distilled off. The residue was distilled under vacuum to give *135 g* (87%) of the title compound, b.p. 114-119°C/5 mm Hg.
Anal. calc. for $C_{13}H_{17}ClO$: C, 69.48; H, 7.62. Found: C, 69.63; H, 7.80.
$^1H$ NMR ($CDCl_3$): $\delta$7.14-7.17 (m, 2H, 2,6-H), 7.08-7.11 (m, 2H, 3,5-H), 3.09-3.17 (m, 2H, ArC*H*H' and C*H*C(O)Cl), 2.87 (sep, $J$ = 7.0 Hz, 1H, C*H*Me$_2$), 2.68-2.74 (m, 1H, ArCH*H'*), 1.26 (d, $J$ = 7.0 Hz, 3H, CH*Me*), 1.23 (d, $J$ = 7.0 Hz, 6H, CH*Me$_2$*). $^{13}C\{^1H\}$ NMR ($CDCl_3$): $\delta$ 177.1, 147.4, 134.8, 128.9, 126.6, 53.3, 38.8, 33.7, 23.9, 16.6.

**6-Isopropyl-2-methylindan-1-one**

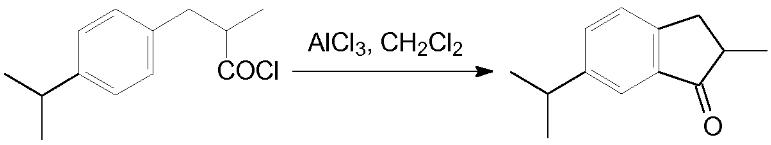


To a suspension of 192 g (1.44 mol, 1.2 eq.) of $AlCl_3$ in 1000 ml of dichloromethane a solution of *270 g (1.2 mol)* of 3-(4-isopropylphenyl)-2-methylpropanoyl chloride in 200 ml of dichloromethane was added dropwise by vigorous stirring at 5°C. The obtained mixture was stirred overnight at room temperature and then poured on 1500 g of the crushed ice. The organic layer was separated, and the aqueous layer was extracted with 3 x 100 ml of dichloromethane. The combined organic extract was washed by aqueous $K_2CO_3$, dried over $K_2CO_3$, passed through a short pad of silica gel 60 (40-63 μm), and then evaporated to dryness. Crude product was distilled under vacuum to give 200 g (89%) of as a colorless liquid, b.p. 125-131 °C/5 mm Hg.
Anal. calc. for $C_{13}H_{16}O$: C, 82.94; H, 8.57. Found: C, 82.92; H, 8.66.
$^1$H NMR ($CDCl_3$): $\delta$ 7.63 (d, *J* = 1.1 Hz, 1H, 7-H), 7.46 (dd, *J* = 7.9 Hz, *J* = 1.8 Hz, 1H, 5-H), 7.36 (d, *J* = 7.9 Hz, 1H, 5-H), 3.35 (dd, *J* = 17.0 Hz, *J* = 7.9 Hz, 1H, 3-C*H*H'), 2.97 (sep, *J* = 7.0 Hz, 1H, C*H*Me$_2$), 2.65-2.74 (m, 2H, 3-CH*H'* and 2-H), 1.30 (d, *J* = 7.3 Hz, 3H, CH*Me*), 1.26 (d, *J* = 7.0 Hz, 6H, CH*Me$_2$*). $^{13}$C{$^1$H} NMR ($CDCl_3$): $\delta$ 209.5, 151.2, 148.4, 136.4, 133.7, 126.2, 121.0, 42.2, 34.5, 33.8, 23.9, 16.2.

**4,7-Dibromo-6-isopropyl-2-methylindan-1-one**

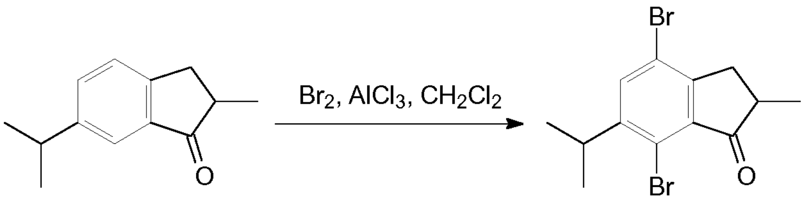


a A solution of 230 g (1.22 mol) of 6-isopropyl-2-methylindan-1-one in 700 ml of dichloromethane was added dropwise with vigorous stirring over 1 h to a suspension of 416 g (3.12 mol, 2.55 eq.) of $AlCl_3$ in 1100 ml of dichloromethane at 0 °C. The reaction mixture was stirred for 10 min at this temperature, then 2 g of iron powder was added. Further on, 402 g (2.52 mol, 2.07 eq.) of bromine was added dropwise for 1 h. The resulting mixture was stirred overnight at room temperature and then poured onto 4000 cm$^3$ of the crushed ice. The organic layer was separated, and the aqueous layer was extracted with 3 x 400 ml of dichloromethane. The combined organic extract was washed with aqueous $K_2CO_3$, dried over $K_2CO_3$, passed through a short pad of silica gel 60 (40-63 μm), and then evaporated to dryness. The resultant dark-red oily liquid was distilled under vacuum: a broad-boiling fraction with b.p. 140-200 °C/6 mm Hg was collected to give 357 g of crude product. This crude product was dissolved in 1200 ml of hot hexane, and the formed solution was kept in a -30 °C freezer over 2 days. A yellowish solid precipitate formed, which was collected and dried in vacuo. This procedure gave 275 g (65%) of 4,7-dibromo-6-isopropyl-2-methylindan-1-one. The mother liquor was evaporated to dryness to give 97.9 g of a mixture (~80% purity) of 4-bromo-6-isopropyl-2-methylindan-1-one and 4,7-dibromo-6-isopropyl-2-methylindan-1-one in ratio equal to ca. 1 to 0.43, i.e. it contains ca. 0.235 mol of 4-bromo-6-isopropyl-2-methylindan-1-one and 0.101 mol of 4,7-dibromo-6-isopropyl-2-methylindan-1-one. This mixture was dissolved in 130 ml of dichloromethane and then brominated via the above-described protocol using 78.5 g of $AlCl_3$ in 200 ml of dichloromethane, 1 g of iron powder and 40.0 g of bromine. This procedure gave 59.3 g of 4,7-dibromo-6-isopropyl-2-methylindan-1-one. Thus, 4,7-dibromo-6-isopropyl-2-methylindan-1-one was obtained in 79% total yield.
Anal. calc. for $C_{13}H_{14}Br_2O$: C, 45.12; H, 4.08. Found: C, 45.42; H, 4.31.
$^1$H NMR ($CDCl_3$): $\delta$ 7.64 (s, 1H, 5-H), 3.59 (sep, *J* = 6.9 Hz, 1H, C*H*Me$_2$), 3.25 (dd, *J* = 17.4 Hz, *J* = 8.0 Hz, 1H, 3-C*H*H'), 2.69-2.82 (m, 1H, 2-H), 2.57 (dd, *J* = 17.4 Hz, *J*= 4.1 Hz, 1H, 3-CH*H'*), 1.33 (d, *J* = 7.2 Hz, 3H, 2-Me), 1.25 (d, *J* = 6.9 Hz, 6H, CH*Me$_2$*). $^{13}$C{$^1$H} NMR ($CDCl_3$): $\delta$ 205.7, 153.0, 149.9, 135.6, 135.1, 121.2, 119.9, 42.8, 34.6, 31.4, 22.7, 16.2.

**4,7-Dibromo-6-isopropyl-1-methoxy-2-methylindane**

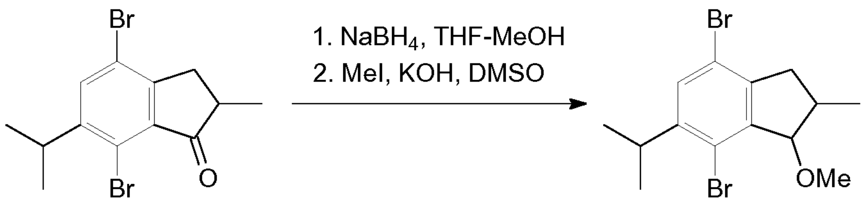


300 ml of methanol was added dropwise over 5 h with vigorous stirring to a mixture of 167 g (0.483 mol) of 4,7-dibromo-6-isopropyl-2-methylindan-1-one and 27.7 g (0.732 mol) of $NaBH_4$ in 600 ml of THF at 0-5 °C. This mixture was stirred overnight at room temperature and then evaporated under vacuum. The residue was acidified by 2 M HCl to pH~5, and the formed 4-bromo-6-isopropyl-2-methylindan-1-ol was extracted with 3 x 300 ml of dichloromethane. The combined organic extract was dried over $Na_2SO_4$ and evaporated to dryness. The residue was immediately dissolved in 1050 ml of DMSO, then 127 g (2.26 mol, 4.7 eq.) of KOH and 157 g (1.1 mol, 2.28 eq.) of iodomethane were added. This mixture was stirred for 5 h at ambient temperature and then poured into 3000 ml of water. Crude product was extracted with 3 x 500 ml of dichloromethane. The combined organic extract was washed with 7 x 1000 ml of water, dried over $Na_2SO_4$, and then evaporated to dryness. Crude product was distilled under vacuum to give 174 g (99%) of a colorless liquid (b.p. 148-162 °C/5 mm Hg) containing a mixture of two stereoisomers.
Anal. calc. for $C_{14}H_{18}Br_2O$: C, 46.44; H, 5.01. Found: C, 46.63; H, 5.18.
*Syn*-isomer: $^1$H NMR ($CDCl_3$): $\delta$ 7.29 (s, 1H, 5-H), 4.66 (d, *J* = 5.5 Hz, 1H, 1-H), 3.55 (s, 3H, OMe), 3.36 (sep, *J* = 6.9 Hz, 1H, C*H*Me$_2$), 2.95 (dd, J=16.0 Hz, *J* = 7.8 Hz, 1H, 3-C*H*H'), 2.75 (dd, *J* = 16.0 Hz, *J* = 9.6 Hz, 1H, 3-CH*H'*), 2.38-2.53 (m, 1H, 2-H), 1.25 (d, *J* = 7.1 Hz, 3H, 2-Me), 1.22 (d, *J* = 6.9 Hz, 3H, CHMeMe'), 1.20 (d, *J*= 6.9 Hz, 3H, CHMe*Me*'). $^{13}$C{$^1$H} NMR ($CDCl_3$): $\delta$ 147.7, 145.6, 143.8, 129.7, 121.4, 119.1, 88.0, 59.5, 40.7, 38.7, 32.5, 22.9, 22.8, 13.5.
Anti-isomer: $^1$H NMR ($CDCl_3$): $\delta$ 7.32 (s, 1H, 5-H), 4.48 (s, 1H, 1-H), 3.46 (s, 3H, OMe), 3.25-3.43 (m, 2H, C*H*Me$_2$ and 3-C*H*H'), 2.52-2.63 (m, 1H, 2-H), 2.49 (dd, *J* = 16.7 Hz, *J* = 1.6 Hz, 1H, 3-CH*H'*), 1.22 (d, 6H, *J* = 6.9 Hz, CH*Me$_2$*), 1.05 (d, *J* = 7.3 Hz, 3H, 2-Me). $^{13}$C{$^1$H} NMR ($CDCl_3$): $\delta$ 148.0, 143.2, 143.1, 130.1, 122.0, 119.7, 93.4, 57.1, 40.7, 36.1, 32.4, 22.9, 22.8, 19.9.

**1-methoxy-2-methyl-4-bromo-6-isopropylindane**

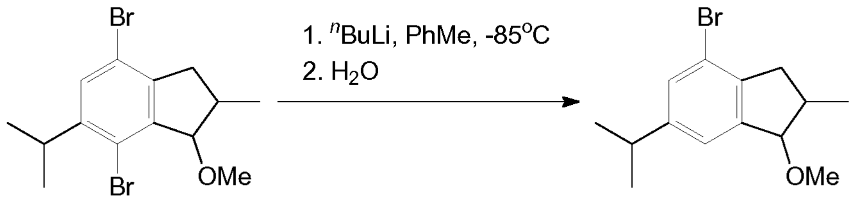


46.0 ml (0.115 mol) of 2.5 M $^n$BuLi in hexanes was added dropwise over 30 min with vigorous stirring to a solution of 41.4 g (0.114 mol) of 4,7-dibromo-6-isopropyl-1-methoxy-2-methylindane in 250 ml of toluene at -85 °C. The resulting mixture was allowed to warm to
-30 °C/-35 °C and stirred for 30 min at this temperature. The reaction was quenched by addition of 150 ml of water. The organic layer was separated, and the aqueous layer was extracted with 100 ml of dichloromethane. The combined organic extract was dried over $K_2CO_3$ and then passed through a short pad of silica gel 60 (40-63 µm). The silica gel layer was additionally washed with 50 ml of dichloromethane. The combined organic eluate was evaporated to dryness to give 34.6 g (~100% yield, the only impurity is toluene) of 4-bromo-6-isopropyl-1-methoxy-2-methylindane as a slightly yellowish liquid. The $^1$H and $^{13}$C NMR spectra were identical to those reported above.
Anal. calc. for $C_{14}H_{19}BrO$: C, 59.37; H, 6.76. Found: C, 59.13; H, 6.88.
*Syn*-isomer: $^1$H NMR ($CDCl_3$): $\delta$ 7.26 (s, 1H, 5-H), 7.15 (s, 1H, 7-H), 4.56 (d, *J* = 5.5 Hz, 1H, 1-H), 3.41 (s, 3H, OMe), 2.81-2.97 (m, 2H, 3-C*H*H' and C*H*Me$_2$), 2.57-2.70 (m, 2H, 3-CH*H'* and 2-H), 1.23 (d, *J* = 6.8 Hz, 6H, CH*Me$_2$*), 1.07 (d, *J* = 6.6 Hz, 3H, 2-Me). $^{13}$C{$^1$H} NMR ($CDCl_3$): $\delta$ 149.4, 144.5, 140.8, 129.3, 122.0, 120.2, 86.8, 57.0, 39.0, 37.9, 33.9, 24.1, 24.0, 13.5.
Anti-isomer: $^1$H NMR ($CDCl_3$): $\delta$ 7.28 (s, 1H, 5-H), 7.16 (s, 1H, 7-H), 4.42 (d, *J*= 4.3 Hz, 1H, 1-H), 3.46 (s, 3H, OMe), 3.18 (dd, *J* = 16.2 Hz, *J* = 7.8 Hz, 1H, 3-C*H*H'), 2.88 (sep, *J* = 6.9 Hz, 1H, C*H*Me$_2$), 2.44-2.57 (m, 1H, 2-H), 2.40 (dd, *J* = 16.2 Hz, *J*= 5.5 Hz, 1H, 3-CH*H'*), 1.23 (d, *J* = 6.9 Hz, 6H, CH*Me$_2$*), 1.18 (d, *J* = 6.8 Hz, 3H, 2-Me). $^{13}$C{$^1$H} NMR ($CDCl_3$): $\delta$ 149.7, 144.1, 140.6, 129.5, 122.2, 120.1, 92.1, 56.6, 39.33, 39.25, 33.9, 24.1, 23.9, 19.4.

**1-methoxy-2-methyl-4-(3,5-Di-*tert*-butylphenyl)-6-isopropylindane**

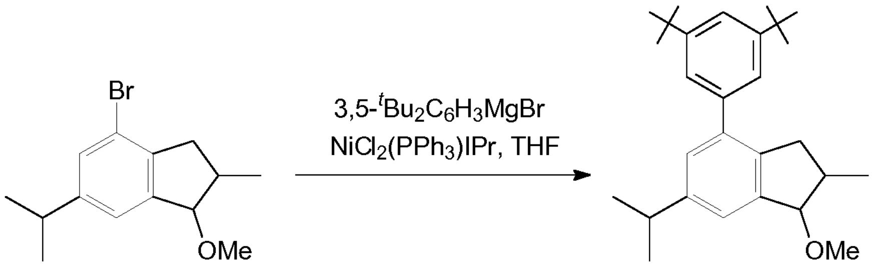


400 ml (200 mmol) of 0.5 M 3,5-di-*tert*-butylphenylmagnesium bromide in THF was added to a mixture of 1.0 g (1.28 mmol) of $NiCl_2(PPh_3)IPr$ and 37.6 g (133 mmol) of 4-bromo-6-isopropyl-1-methoxy-2-methylindane at such a rate as to maintain a gentle reflux. The resulting solution was refluxed for 1 h, then cooled to room temperature, and 150 ml of water was added. The major part of THF was distilled off on rotary evaporator, then 500 ml of dichloromethane and 1000 ml of 1 M HCl were added to the residue. The organic layer was separated, and the aqueous layer was extracted with 150 ml of dichloromethane. The combined organic extract was evaporated to dryness to give a slightly greenish oil. The product was isolated by flash chromatography on silica gel 60 (40-63 μm; eluent: hexanes-dichloromethane = 2:1 vol., then 1:1 vol.). This procedure gave 4-(3,5-di-*tert*-butylphenyl)-6-isopropyl-1-methoxy-2-methylindane as colorless thick oil which slowly crystallized at room temperature. The product is a mixture of two stereoisomers. Anal. calc. for $C_{28}H_{40}O$: C, 85.66; H, 10.27. Found: C, 85.86; H, 10.43.
*Syn*-isomer: $^1H$ NMR ($CDCl_3$): $\delta$ 7.39 (t, *J* = 1.8 Hz, 1H, 4-H in 3,5-$^tBu_2C_6H_3$), 7.29 (d, *J* = 1.8 Hz, 2H, 2,6-H in 3,5-$^tBu_2C_6H_3$), 7.23 (m, 1H, 5-H in indane), 7.20 (m, 1H, 7-H in indane), 4.53 (d, *J* = 5.5 Hz, 1H, 1-H), 3.48 (s, 3H, OMe), 2.98 (sep, *J* = 6.9 Hz, 1H, C*H*Me$_2$), 2.92 (dd, *J* = 15.5 Hz, *J* = 7.1 Hz, 1H, 3-C*H*H'), 2.79 (dd, *J* = 15.5 Hz, *J* = 6.7 Hz, 1H, 3-CH*H'*), 2.49-2.65 (m, 1H, 2-H), 1.37 (s, 18H, 3,5-$^t$*Bu*$_2C_6H_3$), 1.30 (d, *J* = 6.9 Hz, 6H, CH*Me*$_2$), 1.09 (d, *J* = 6.8 Hz, 3H, 2-Me in indane). $^{13}C\{^1H\}$ NMR ($CDCl_3$): $\delta$ 150.4, 147.4, 143.5, 140.2, 139.5, 138.7, 126.9, 123.0, 121.8, 120.7, 86.4, 56.9, 39.1, 38.0, 34.9, 34.1, 31.5, 24.3, 24.2, 13.6.
Anti-isomer: $^1H$ NMR ($CDCl_3$): $\delta$ 7.39 (t, *J* = 1.8 Hz, 1H, 4-H in 3,5-$^tBu_2C_6H_3$), 7.28 (d, *J* = 1.8 Hz, 2H, 2,6-H in 3,5-$^tBu_2C_6H_3$), 7.24 (m, 1H, 5-H in indane), 7.20 (m, 1H, 7-H in indane), 4.53 (d, *J* = 3.9 Hz, 1H, 1-H), 3.52 (s, 3H, OMe), 3.29 (dd, *J* = 15.3 Hz, *J* = 7.1 Hz, 1H, 3-C*H*H'), 2.98 (sep, *J* = 6.9 Hz, 1H, C*H*Me$_2$), 2.40-2.56 (m, 2H, 3-C*H*H' and 2-H), 1.37 (s, 18H, 3,5-$^t$*Bu*$_2C_6H_3$), 1.30 (d, *J* = 6.9 Hz, 6H, CH*Me*$_2$), 1.13 (d, *J* = 6.8 Hz, 3H, 2-Me in indane). $^{13}C\{^1H\}$ NMR ($CDCl_3$): $\delta$ 150.4, 147.7, 142.9, 140.2, 139.6, 138.2, 127.2, 122.9, 121.8, 120.7, 91.6, 56.6, 40.0, 38.1, 34.9, 34.1, 31.5, 24.4, 24.1, 19.2.

**2-methyl-5-isopropyl-7-(3,5-Di-*tert*-butylphenyl)-1*H*-indene**

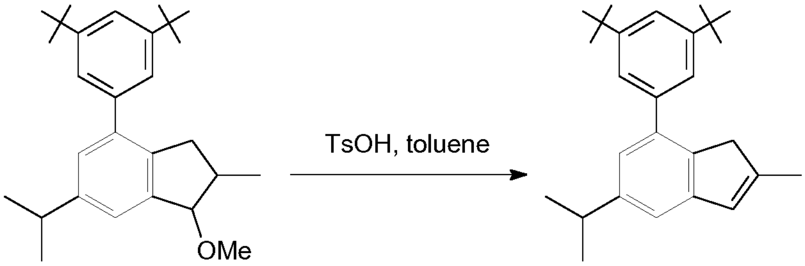


1.0 g of TsOH was added to a solution of 4-(3,5-di-*tert*-butylphenyl)-6-isopropyl-1-methoxy-2-methylindane (as prepared above) in 450 ml of toluene, and the resulting solution was refluxed using Dean-Stark head for 15 min. After that it was cooled to room temperature and washed by 200 ml of 10% aqueous $NaHCO_3$. The organic layer was separated, and the aqueous layer was extracted with 200 ml of dichloromethane. The combined organic extract was evaporated to dryness, and the product was isolated by flash chromatography on silica gel 60 (40-63 μm; eluent: hexanes-dichloromethane = 10:1, vol.). This procedure gave 46.7 g (98%) of 7-(3,5-di-*tert*-butylphenyl)-5-isopropyl-2-methyl-1*H*-indene as a yellowish glass which slowly crystallized at room temperature.

Anal. calc. for $C_{27}H_{36}$: C, 89.94; H, 10.06. Found: C, 90.24; H, 10.31.
$^{1}H$ NMR ($CDCl_3$): $\delta$ 7.41 (t, *J* = 1.4 Hz, 1H, 4-H in 3,5-$^{t}Bu_2C_6H_3$), 7.38 (d, *J* = 1.4 Hz, 2H, 2,6-H in 3,5-$^{t}Bu_2C_6H_3$), 7.14 (s, 1H, 6-H in indene), 7.02 (s, 1H, 4-H in indene), 6.51 (d, *J* = 1.5 Hz, 1H, 3-H in indene), 3.34 (s, 2H, $CH_2$ in indene), 2.99 (sep, *J* = 6.9 Hz, 1H, CH*Me*$_2$), 2.12 (s, 3H, 2-Me in indene), 1.38 (s, 18H, 3,5-$^{t}Bu_2C_6H_3$), 1.31 (d, 6H, *J* = 6.9 Hz, CH*Me*$_2$). $^{13}C\{^{1}H\}$ NMR ($CDCl_3$): $\delta$ 150.5 (two resonances), 147.9, 146.7, 146.5, 140.8, 138.5, 138.3, 127.3, 122.8, 120.8, 116.7, 42.5, 35.0, 34.3, 31.6, 24.4, 16.8.

**Bis[2-methyl-4-(3,5-di-*tert*-butylphenyl)-6-isopropyl-1*H*-inden-1-yl](dimethyl)silane**

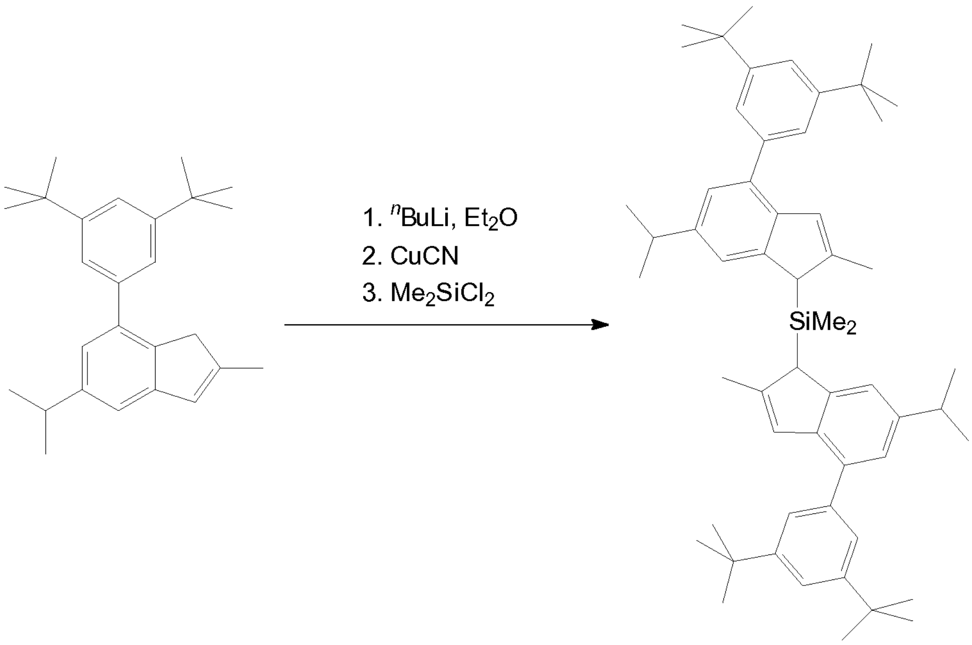


20.0 ml (50.0 mmol) of 2.5 M $^{n}$BuLi in hexanes was added in one portion to a solution of 18.0 g (50.0 mmol) of 7-(3,5-di-*tert*-butylphenyl)-5-isopropyl-2-methyl-1*H*-indene in 250 ml of ether at -50 °C. This mixture was stirred overnight at room temperature, then the resulting yellow-orange solution was cooled to -40 °C, and 200 mg of CuCN was added. The obtained mixture was stirred for 30 min at -25 °C, and 3.23 g (25.0 mmol) of dichlorodimethylsilane was added in one portion. Further on, this mixture was stirred overnight at ambient temperature, then filtered through a pad of silica gel 60 (40-63 μm) which was additionally washed by 2 x 50 ml of dichloromethane. The combined filtrate was evaporated under vacuum, and the residue was dried in vacuo at elevated temperature. This procedure gave 19.5 g (~100%) of the title product of >90% purity. On the evidence of NMR spectroscopy it was a ca. 3:2 mixture of the stereoisomers. This yellowish glassy product was used without further purification.
$^{1}H$ NMR ($CDCl_3$): $\delta$ 7.48-7.56 (m), 7.38 (m), 7.25-7.30 (m), 6.90 (m), 3.90 (s), 3.88 (s), 3.11 (sept, *J* = 6.9 Hz), 2.36 (s), 1.52 (s), 1.38-1.49 (m), -0.02 (s), -0.08 (s),-0.09 (s).

***Rac*-dimethylsilanediylbis[2-methyl-4-(3,5-di-*tert*-butylphenyl)-6-isopropyl-inden-1-yl]zirconium dichloride (I-mc3)**

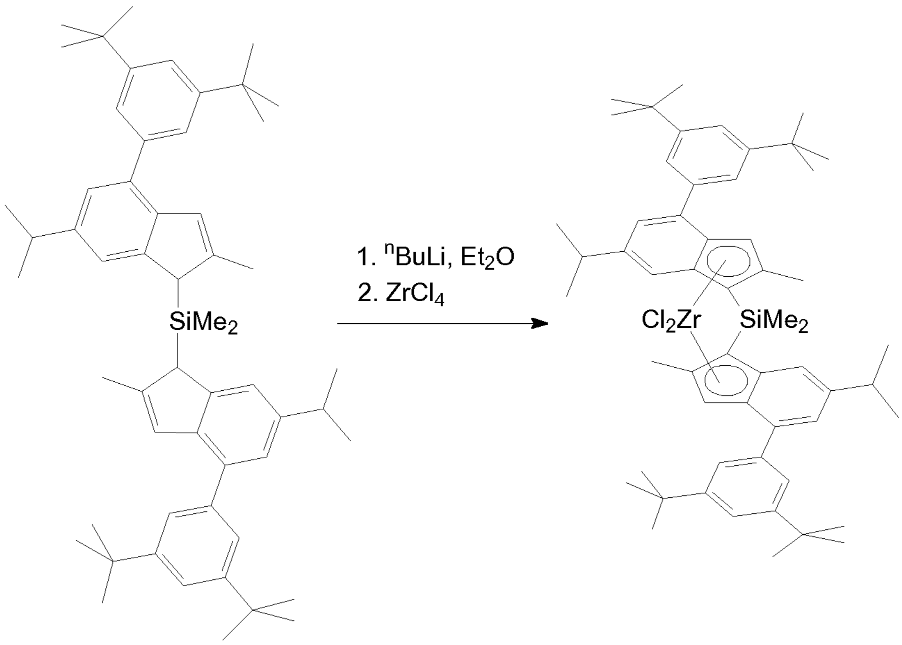


20.0 ml (50.0 mmol) of 2.5 M $^n$BuLi in hexanes was added in one portion to a solution of 19.5 g (25.0 mmol) of bis[2-methyl-4-(3,5-di-*tert*-butylphenyl)-6-isopropyl-1*H*-inden-1-yl](dimethyl)silane (as prepared above) in 250 ml of ether cooled to -30 °C. This mixture was stirred overnight at room temperature. The resulting light-orange solution was cooled to -50 °C and 5.83 g (25.0 mmol) of $ZrCl_4$ was added. The reaction mixture was stirred for 24 h giving orange suspension which then was evaporated to dryness. The residue was poured into 200 ml of hot toluene. This hot mixture was filtered through glass frit (G4). On the evidence of NMR spectroscopy the obtained filtrate included a ca. 1 to 1 mixture of *rac-* and *meso*-zirconocenes. This filtrate was evaporated to ca. 60 ml and then heated to dissolve the formed precipitate. Orange crystals precipitated from this solution overnight at room temperature were collected and dried in vacuum. This procedure gave 7.07 g of pure *meso*-zirconocene. The mother liquor was evaporated to ca. 40 ml. Yellow crystals precipitated from this solution for 4 h at room temperature were collected and dried in vacuum. This procedure gave 6.39 g of pure *rac*-zirconocene. The mother liquor was then evaporated to dryness, and 30 ml of n-hexane was added to the residue. Crystals precipitated from this solution overnight at room temperature were collected and dried in vacuum. This procedure gave 3.92 g of a ca. 6 to 4 mixture of *rac-* and *meso*-zirconocenes. Thus, the total yield of *rac-* and *meso*-zirconocenes isolated in this synthesis was 17.4 g (74%).
*Rac*-zirconocene (I-mc3).
Anal. calc. for $C_{56}H_{74}Cl_2SiZr$: C, 71.75; H, 7.96. Found: C, 71.88; H, 8.10.
$^1$H NMR ($CDCl_3$): $\delta$ 7.52 (d, *J* = 1.8 Hz, 4H), 7.45 (m, 2H), 7.39 (t, *J*= 1.8 Hz, 2H), 7.30 (m, 2H), 6.89 (m, 2H), 2.94 (sept, *J* = 7.0 Hz, 2H), 2.25 (s, 6H), 1.34 (s, 6H), 1.32 (s, 36H), 1.28 (d, *J* = 7.0 Hz, 3H), 1.26 (d, *J* = 7.0 Hz, 3H). $^{13}$C{$^1$H} NMR ($CDCl_3$): $\delta$ 150.92, 146.39, 139.44, 138.77, 134.74, 131.01, 128.33, 127.30, 123.27, 122.59, 121.45, 119.63, 82.26, 35.05, 34.47, 31.54, 23.59, 23.49, 18.55, 2.87.
*Meso*-zirconocene (*meso*- I-mc3).
Anal. found: C, 72.01; H, 8.15.
$^1$H NMR ($CDCl_3$): $\delta$ 7.49 (m, 4H), 7.40 (m, 4H), 7.08 (m, 2H), 6.78 (m, 2H), 2.81 (sept, *J* = 6.5 Hz, 2H), 2.43 (s, 6H), 1.48 (s, 3H), 1.34 (s, 36H), 1.25 (d, *J* = 6.5 Hz, 3H), 1.22 (s, 3H), 1.14 (d, *J* = 6.3 Hz, 3H). $^{13}$C{$^1$H} NMR ($CDCl_3$): $\delta$ 150.77, 145.43, 138.91, 138.63, 135.62, 133.25, 129.23, 125.28, 123.25, 122.17, 121.40, 120.63, 84.23, 35.06, 34.46, 31.57, 23.97, 23.19, 18.60, 3.13, 2.92.

**I-mc4: 4-Methylisobutyrophenone**

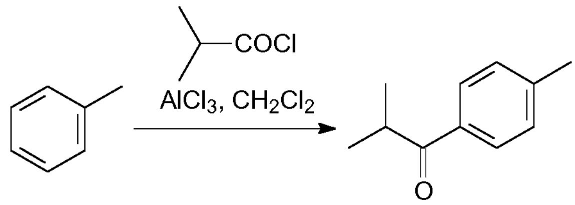


240 g (1.8 mol, 1.2 eq.) of $AlCl_3$ was added in small portions over 2 h to a stirred mixture of 500 ml of toluene and 160 g (1.5 mol) of 2-methylpropanoyl chloride at 0-5 °C. This mixture was stirred overnight at room temperature and then poured into 2000 ml of the crushed ice. The organic layer was separated; the aqueous layer was extracted with 2 x 200 ml of toluene. The combined organic extract was dried over $Na_2SO_4$, passed through a short pad of silica gel 60 (40-63 μm), and then evaporated to dryness. The crude product was distilled under vacuum to give 216 g (89%) of a colorless liquid, b.p. 125-127 °C/20 mm Hg.
Anal. calc. for $C_{11}H_{14}O$: C, 81.44; H, 8.70. Found: C, 81.38; H, 8.79.
$^1H$ NMR ($CDCl_3$): $\delta$ 7.86 (d, *J* = 7.6 Hz, 2H), 7.25 (d, *J* = 7.6 Hz, 2H), 3.53 (sep, *J* = 6.8 Hz, 1H), 2.39 (s, 3H), 1.20 (d, *J* = 6.8 Hz, 6H).

**3-Bromo-4-methylisobutyrophenone**

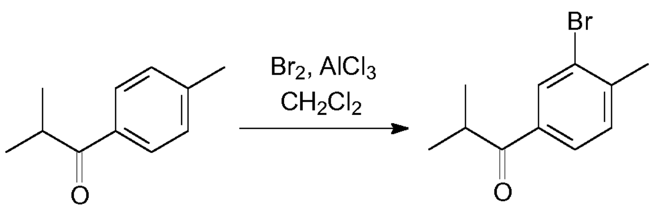


A solution of 216 g (1.33 mol) of 4-methylisobutyrophenone in 700 ml of dichloromethane was added over 30 min to a suspension of 445 g (3.34 mol) of $AlCl_3$ in 1150 ml of dicloromethane at 0 °C. The reaction mixture was stirred for 10 min at this temperature, then 71.5 ml (223 g, 1.4 mol, 5% excess) of bromine was added dropwise over 2 h. The resulting mixture was stirred overnight at room temperature and then poured onto 4000 $cm^3$ of ice. The organic layer was separated, and the aqueous layer was extracted with 3 x 300 ml of dichloromethane. The combined extract was washed with aqueous $K_2CO_3$, dried over $K_2CO_3$, passed through a short pad of silica gel, and, finally, evaporated to dryness. Crude product was distilled under vacuum to give 290 g (90%) of a colorless liquid, b.p. 124-127 °C/6 mm Hg.
Anal. calc. for $C_{11}H_{13}BrO$: C, 54.79; H, 5.43. Found: C, 54.90; H, 5.61.
$^1H$ NMR ($CDCl_3$): $\delta$ 8.11 (s, 1H, 2-H), 7.78 (d, *J* = 7.8 Hz, 1H, 6-H), 7.32 (d, *J* = 7.8 Hz, 1H, 5-H), 3.48 (sep, *J* = 6.8 Hz, 1H, C*H*$Me_2$), 2.45 (s, 3H, 4-Me), 1.20 (d, *J* = 6.8 Hz, 6H, CH*$Me_2$*). $^{13}C\{^1H\}$ NMR ($CDCl_3$): $\delta$ 202.8, 143.1, 135.5, 132.3, 130.9, 127.1, 125.3, 35.3, 23.1, 19.0.

**3-Bromo-4-methylpivalophenone**

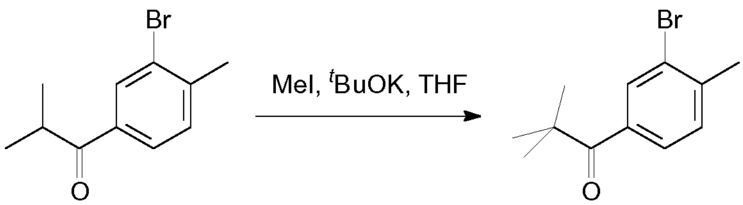


To the mixture of 241 g (1.0 mol) of 3-bromo-4-methylisobutyrophenone and 170 g (1.2 mol) of iodomethane in 200 ml of THF a solution of 157 g (1.4 mol) of potassium *tert*-butoxide in 1000 ml of THF was added dropwise by vigorous stirring for 5 h at 0-5°C. This mixture was stirred overnight at room temperature. Further on, 1000 ml of water and 400 ml of n-hexane were added to the reaction mixture. The organic layer was separated, and the aqueous layer

was extracted with 500 ml of dichloromethane. The combined organic extract was dried over $Na_2SO_4$ and then evaporated to dryness. Crude product was distilled under vacuum to give 248 g (97%) of a colorless liquid, b.p. 159-163 °C/20-21 mm Hg.

Anal. calc. for $C_{12}H_{15}BrO$: C, 56.49; H, 5.93. Found: C, 56.58; H, 6.10.

$^1$H NMR ($CDCl_3$): $\delta$ 7.91 (d, $J$ = 1.5 Hz, 1H, 2-H), 7.59 (dd, $J$ = 8.1 Hz, $J$ = 1.8 Hz, 1H, 6-H), 7.27 (d, $J$ = 8.1 Hz, 1H, 5-H), 2.44 (s, 3H, 4-Me), 1.36 (s, 9H, $^t$Bu). $^{13}C\{^1H\}$ NMR ($CDCl_3$): $\delta$ 206.9, 141.1, 137.4, 132.1, 130.3, 126.9, 124.7, 44.2, 28.0, 22.9.

**2-Bromo-4-pivaloylbenzyl bromide**

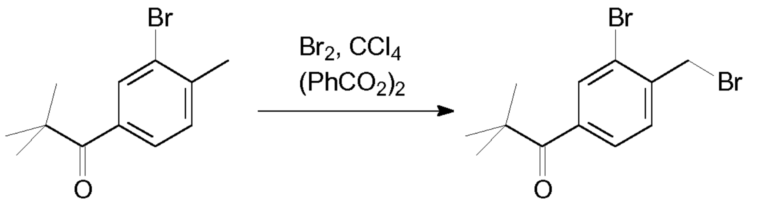


154 g (0.961 mol) of bromine and 1 g of benzoyl peroxide were added to a solution of 245 g (0.961 mol) of 3-bromo-4-methylpivalophenone in 1300 ml of tetrachloromethane. This mixture was refluxed for 2 h (until the disappearance of the red color). The resulting mixture was cooled to room temperature, filtered through glass frit (G3), and the filtrate was evaporated to dryness. Crude product was distilled under vacuum to give 263 g (82%) of a colorless oil, b.p. 135-155 °C/7 mm Hg.

Anal. calc. for $C_{12}H_{14}Br_2O$: C, 43.15; H, 4.22. Found: C, 43.39; H, 4.42.

$^1$H NMR ($CDCl_3$): $\delta$ 7.87 (d, $J$ = 1.8 Hz, 1H, 3-H), 7.61 (dd, $J$ = 8.1 Hz, $J$ = 1.8 Hz, 1H, 5-H), 7.49 (d, $J$ = 8.1 Hz, 1H, 6-H), 4.59 (s, 2H, C*H*$_2$Br), 1.34 (s, 9H, *$^t$Bu*). $^{13}C\{^1H\}$ NMR ($CDCl_3$): $\delta$ 206.9, 140.0, 139.5, 132.7, 130.8, 127.2, 124.2, 44.3, 32.3, 27.8.

**Diethyl (4-pivaloyl-2-bromobenzyl)(methyl)malonate**

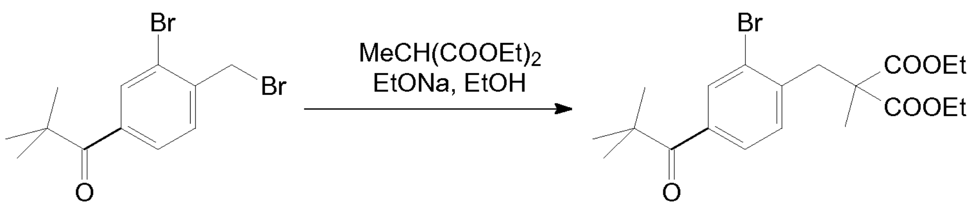


137 g (0.787 mol) of diethyl methylmalonate was added to a solution of sodium ethoxide prepared from 18.1 g (0.787 mol) of sodium metal and 600 ml of anhydrous ethanol. This mixture was stirred for 15 min, then 263 g (0.787 mol) of 2-bromo-4-pivaloylbenzyl bromide was added at such a rate as to maintain a gentle reflux. Additionally, this mixture was refluxed for 3 h, then ethanol was distilled off at atmospheric pressure. The obtained mixture was cooled to room temperature, 1000 ml of water and 500 ml of dichloromethane were added. The organic layer was separated, and the aqueous layer was extracted with 2 x 300 ml of dichloromethane. The combined organic extract was dried over $Na_2SO_4$ and evaporated to dryness. Crude product was distilled under vacuum to give 274 g (81%) of a colorless oil, b.p. 170-176 °C/7 mm Hg.

Anal. calc. for $C_{20}H_{27}BrO_5$: C, 56.21; H, 6.37. Found: C, 56.39; H, 6.41.

$^1$H NMR ($CDCl_3$): $\delta$ 7.89 (d, $J$ = 1.8 Hz, 1H, 3-H), 7.56 (dd, $J$ = 8.1 Hz, $J$ = 1.8 Hz, 1H, 5-H), 7.24 (d, $J$ = 8.1 Hz, 1H, 6-H), 4.17-4.27 (m, 4H, C*H*$_2$Me), 3.53 (s, 2H, ArC*H*$_2$), 1.40 (s, 3H, *Me*C(COOEt)$_2$), 1.33 (s, 9H, $^t$Bu), 1.26 (t, $J$= 7.1 Hz, 6H, CH$_2$*Me*). $^{13}C\{^1H\}$ NMR ($CDCl_3$): $\delta$ 206.8, 171.5, 139.5, 138.2, 132.6, 130.8, 126.6, 126.1, 61.5, 54.8, 44.2, 39.2, 27.9, 19.4, 13.9.

3-(2-Bromo-4-pivaloylphenyl)-2-methylpropanoic acid

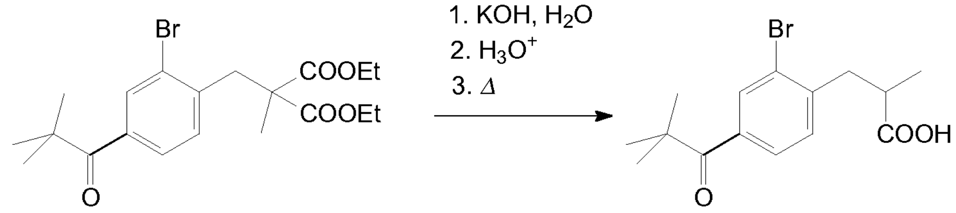


A solution of 160 g of KOH in 420 ml of water was added to a solution of 274 g (0.640 mol) of (4-pivaloyl-2-bromobenzyl)(methyl)malonate in 300 ml of methanol. This mixture was refluxed for 4 h. Methanol and water were distilled off until vapor temperature reached 95 °C, and 3000 ml of water and then 12 M HCl (to pH 1) were added to the residue. The precipitated substituted methylmalonic acid was filtered off, washed with water, and partially dried on the filter. Crude 3-(2-bromo-4-pivaloylphenyl)-2-methylpropanoic acid was obtained as reddish oil after decarboxylation of this substituted methylmalonic acid at 180 °C. Yield 189 g (90%).
Anal. calc. for $C_{15}H_{19}BrO_3$: C, 55.06; H, 5.85. Found: C, 54.83; H, 5.60.
$^1$H NMR ($CDCl_3$): $\delta$ 11.30 (br. s, 1H, COOH), 7.89 (d, $J$ = 1.5 Hz, 1H, 3-H), 7.59 (dd, $J$ = 7.8 Hz, $J$ = 1.8 Hz, 1H, 5-H), 7.27 (d, $J$ = 7.8 Hz, 1H, 6-H), 3.21 (dd, $J$ = 13.4 Hz, $J$ = 7.1 Hz, 1H, ArC*H*H'), 2.88-3.00 (m, 1H, C*H*COOH), 2.84 (dd, $J$ = 13.4 Hz, $J$ = 7.3 Hz, 1H, ArCH*H'*), 1.34 (s, 9H, $^t$Bu), 1.24 (d, $J$ = 7.1 Hz, 3H, Me). $^{13}$C{$^1$H} NMR ($CDCl_3$): $\delta$ 207.1, 181.4, 141.5, 138.3, 132.6, 130.8, 126.9, 124.6, 44.3, 39.1, 39.1,27.9,16.9.

3-(2-Bromo-4-neopentylphenyl)-2-methylpropanoic acid

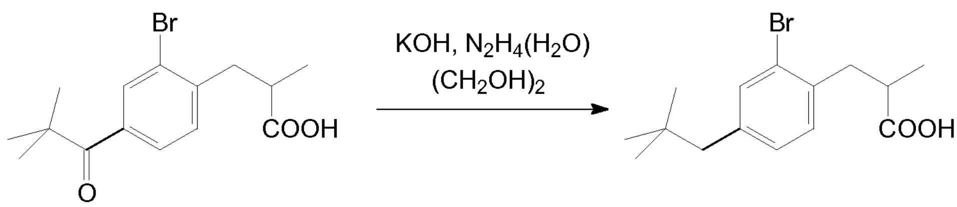


1000 ml of ethylene glycol, and 125 g of KOH were added in one portion to a mixture of 189 g (0.577 mol) of 3-(2-bromo-4-pivaloylphenyl)-2-methylpropanoic acid 100 ml of hydrazine hydrate. The resulting yellowish solution was refluxed for 5 h. Then, a reflux condenser was replaced by a Claisen distillation head with Liebig condenser. A mixture of hydrazine, water and ethylene glycol was slowly distilled off until the distillation temperature reached 198 °C (eventually this reaction mixture was evaporated to about half its initial volume over 5 h). The residue cooled to room temperature was poured into 4 L of water, acidified by 12 N HCl to pH~1, and 700 ml of dichloromethane was added. The organic layer was separated and the aqueous layer was extracted with 2 x 250 ml of dichloromethane. The combined organic extract was washed with 1 L of water and then evaporated to dryness to give 178 g (99%) of a red viscous oil of the title compound which was further used without an additional purification.
$^1$H NMR ($CDCl_3$): $\delta$ 11.6 (br. s, 1H, COOH), 7.31 (d, $J$ = 1.5 Hz, 1H, 3-H), 7.11 (d, $J$ = 7.6 Hz, 1H, 6-H), 6.98 (dd, $J$ = 7.6 Hz, $J$ = 1.5 Hz, 1H, 5-H), 3.16 (dd, $J$ = 13.5 Hz, $J$ = 6.8 Hz, 1H, ArC*H*H'), 2.84-2.98 (m, 1H, C*H*COOH), 2.77 (dd, $J$ = 13.5 Hz, $J$ = 7.8 Hz, 1H, ArCH*H'*), 2.42 (s, 2H, C$H_2$$^t$Bu), 1.21 (d, $J$ = 6.8 Hz, 3H, Me), 0.89 (s, 9H, $^t$Bu). $^{13}$C{$^1$H} NMR ($CDCl_3$): $\delta$ 182.6, 140.2, 135.6, 134.5, 130.4, 129.5, 124.0, 49.3, 39.5, 38.9, 31.7, 29.3, 16.7.

**3-[2-Bromo-4-neopentyl-phenyl]-2-methylpropanoyl chloride**

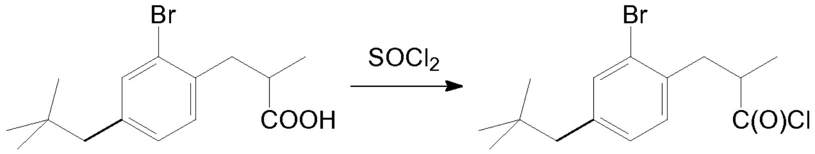


A mixture of 178 g (0.569 mol) of 3-(2-bromo-4-neopentylphenyl)-2-methylpropanoic acid and 145 ml (1.99 mol, 3.5 eq.) of thionyl chloride was stirred for 24 h at room temperature. Excess thionyl chloride was distilled off. The residue was distilled under vacuum to give 166 g (88%) of the title product, b.p. 153-157°C/ 7 mm Hg.
Anal. calc. for $C_{15}H_{20}BrClO$: C, 54.32; H, 6.08. Found: C, 54.12; H, 6.35.
$^1$H NMR ($CDCl_3$): $\delta$ 7.33 (d, *J* = 1.5 Hz, 1H, 3-H), 7.11 (d, *J* = 7.8 Hz, 1H, 6-H), 7.00 (dd, *J* = 7.8 Hz, *J* = 1.5 Hz, 1H, 5-H), 3.19-3.37 (m, 2H, ArC*H*H'and C*H*COCl), 2.82 (dd, *J* = 12.9 Hz, *J* = 6.8 Hz, 1H, ArCH*H*'), 2.43 (s, 2H, C$H_2$$^t$Bu), 1.31 (d, *J* = 6.8 Hz, 3H, Me), 0.90 (s, 9H, $^t$Bu). $^{13}C\{^1H\}$ NMR ($CDCl_3$): $\delta$ 177.1, 140.8, 134.7, 134.2, 130.6, 129.7, 123.8, 51.4, 49.3, 38.9, 31.8, 29.3, 16.8.

**4-Bromo-6-neopentyl-2-methylindan-1-one**

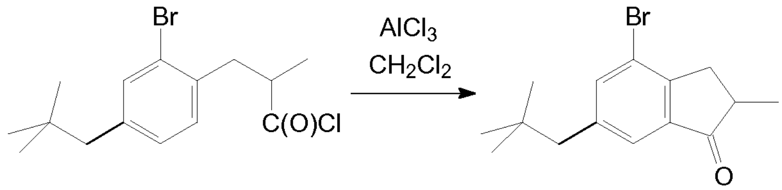


166 g (0.499 mol) of 3-[2-bromo-4-neopentyl-phenyl]-2-methylpropanoyl chloride dissolved in 100 ml of dichloromethane was added dropwise with vigorous stirring to a suspension of 80.0 g (0.600 mol, 1.2 eq.) of $AlCl_3$ in 500 ml of dichloromethane at +5°C. This mixture was stirred overnight at room temperature and then poured on 500 g of crushed ice. The organic layer was separated, and the aqueous layer was extracted with 3 x 100 ml of dichloromethane. The combined organic extract was washed by aqueous $K_2CO_3$, dried over $K_2CO_3$, passed through a short pad of silica gel 60 (40-63 $\mu$m), and then evaporated to dryness. The crude product was distilled under vacuum to give 137 g (93%) of a yellowish oil, b.p. 155-162 °C/7 mm Hg.
Anal. calc. for $C_{15}H_{19}BrO$: C, 61.03; H, 6.49. Found: C, 61.19; H, 6.44.
$^1$H NMR ($CDCl_3$): $\delta$ 7.54 (d, *J* = 1.3 Hz, 1H, 7-H), 7.45 (d, *J* = 1.3 Hz, 1H, 5-H), 3.31 (dd, *J* = 17.4 Hz, *J* = 7.8 Hz, 1H, 3-C*H*H'), 2.67-2.81 (m, 1H, 2-H), 2.62 (dd, *J* = 17.4 Hz, *J* = 4.0 Hz, 1H, 3-CH*H*'), 2.53 (s, 2H, C$H_2$$^t$Bu), 1.33 (d, *J* = 7.6 Hz, 3H, 2-Me), 0.91 (s, 9H, $^t$Bu). $^{13}C\{^1H\}$ NMR ($CDCl_3$): $\delta$ 208.6, 150.6, 141.5, 139.3, 137.8, 124.2, 121.0, 49.3, 42.2, 35.5, 31.7, 29.1, 16.1.

**1-methoxy-2-methyl-4-Bromo-6-neopentyl-indane**

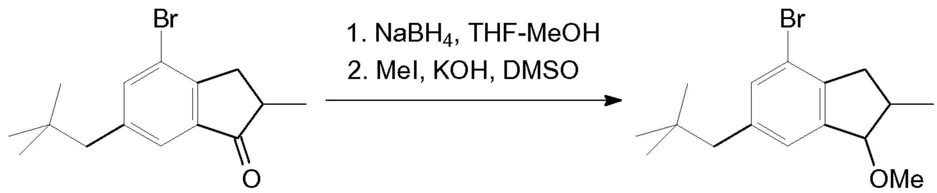


235 ml of methanol was added dropwise with vigorous stirring to a mixture of 137 g (0.464 mol) of 4-bromo-6-neopentyl-2-methylindan-1-one and 26.3 g (0.695 mmol) of $NaBH_4$ in 470 ml of THF for 5 h at 0-5 °C. This mixture was stirred overnight at room temperature and then evaporated to dryness under vacuum. The residue was acidified by 2 M HCl to pH~5, and the crude product was extracted with 3 x 250 ml of dichloromethane. The combined organic extract was dried over $Na_2SO_4$ and evaporated to dryness. The resulting yellowish oil was immediately dissolved in 450 ml of DMSO, then 104 g (1.86 mol, 4 eq.) of KOH and 132 g (0.930 mol, 2 eq.) of iodomethane were added. The obtained

mixture was stirred for 5 h at ambient temperature, and then 2 L of water was added. The organic layer was separated, and the aqueous layer was extracted with 3 x 100 ml of dichloromethane. The combined organic extract was additionally washed with 5 x 1000 ml of water, dried over $Na_2SO_4$, and then evaporated to dryness. Fractional distillation of the residue gave 131 g (91%) of 1-methoxy-2-methyl-4-bromo-6-neopentyl-indane as a mixture of two stereoisomers, b.p. 137-139°C/ 5-6 mm Hg.
Anal. calc. for $C_{16}H_{23}BrO$: C, 61.74; H, 7.45. Found: C, 61.96; H, 7.61.
$^1H$ NMR ($CDCl_3$): $\delta$ 7.19 (s, 1H, 5-H of *anti*-isomer), 7.18 (s, 1H, 5-H of *syn*-isomer), 7.05 (s, 1H, 7-H of *anti*-isomer), 7.05 (s, 1H, 7-H of *syn*-isomer), 4.53 (d, *J* = 5.6 Hz, 1H, 1-H of *syn*-isomer), 4.43 (d, *J* = 4.3 Hz, 1H, 1-H of *anti*-isomer), 3.42 (s, 3H, OMe of *anti*-isomer), 3.38 (s, 3H, OMe of *syn*-isomer), 3.19 (dd, *J* = 16.2 Hz, *J* = 7.6 Hz, 1H, ArC*H*H' of *anti*-isomer), 2.91 (dd, *J* = 15.7 Hz, *J* = 6.8 Hz, 1H, ArC*H*H' of *syn*-isomer), 2.34-2.71 (m, 8H, ArCH*H*', 2-H and C$H_2$$^t$Bu of both isomers), 1.17 (d, *J* = 7.1 Hz, 3H, 2-Me of *anti*-isomer), 1.10 (d, *J* = 6.8 Hz, 3H, 2-Me of *syn*-isomer), 0.90 (s, 18H, $^t$Bu of both isomers). $^{13}C\{^1H\}$ NMR ($CDCl_3$): $\delta$ 143.8, 143.3, 141.2, 140.7, 140.3, 139.9, 133.2, 133.0, 126.3, 126.2, 119.5, 119.5, 91.9, 86.7, 56.7, 56.2, 49.6 (two resonances), 39.4, 39.16, 39.15, 38.2, 31.8, 31.8, 29.3 (two resonances), 19.3, 13.5.

**2-methylMethyl-7-(3,5-Di-*tert*-butylphenyl)-5-neopentyl-1*H*-indene**

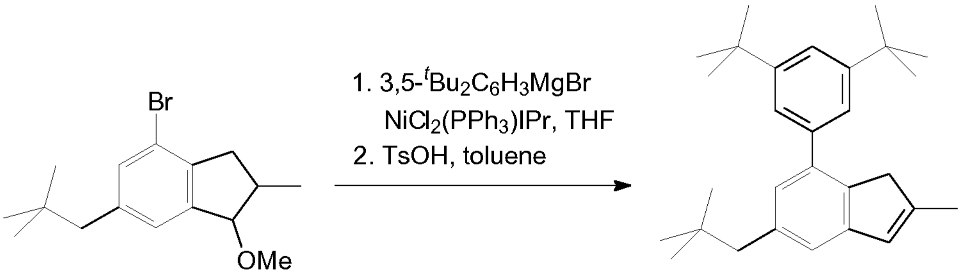


220 ml (110 mmol, 1.25 eq.) of 0.5 M 3,5-di-*tert*-butylphenylmagnesium bromide in THF was added to a mixture of 1.38 g (1.77 mmol, 2 mol. %) of $NiCl_2(PPh_3)$IPr and 27.5 g (88.2 mmol) of 1-methoxy-2-methyl-4-bromo-6-neopentyl-indane, at such a rate as to maintain a gentle reflux. The resulting solution was refluxed for 0.5 h, then cooled to room temperature, and 50 ml of water was added. The major part of THF was distilled off in a rotary evaporator, and then 500 ml of dichloromethane and 500 ml of 1 M HCl were added to the residue. The organic layer was separated, and the aqueous layer was extracted with 150 ml of dichloromethane. The combined organic extract was evaporated to dryness to give a greenish-yellow oil. A mixture of this product, 1.5 g of TsOH, and 600 ml of toluene was refluxed using Dean-Stark head for 15 min. The obtained mixture was washed by 200 ml of 10% aqueous $NaHCO_3$. The organic layer was separated, and the aqueous layer was extracted with 150 ml of dichloromethane. The combined organic extract was evaporated to dryness, and the product was isolated by flash-chromatography on silica gel 60 (40-63 $\mu$m; eluent: hexanes-dichloromethane = 10:1, vol.). This procedure gave 31.7 g (93%) of a colorless oil which solidified on standing at room temperature.
Anal. calc. for $C_{29}H_{40}$: C, 89.63; H, 10.37. Found: C, 89.86; H, 10.59.
$^1H$ NMR ($CDCl_3$): $\delta$ 7.41 (t, *J* = 1.8 Hz, 1H, 4-H in 3,5-$^t$Bu$_2$C$_6$H$_3$), 7.37 (d, *J* = 1.8 Hz, 2H, 2,6-H in 3,5-$^t$Bu$_2$C$_6$H$_3$), 7.02 (s, 1H, 6-H in indenyl), 6.92 (s, 1H, 4-H in indenyl), 6.51 (q, *J* = 1.3 Hz, 1H, 3-H in indenyl), 3.35 (s, 2H, $CH_2$ in indenyl), 2.57 (s, 2H, C$H_2$$^t$Bu), 2.13 (s, 3H, 2-Me in indenyl), 1.38 (s, 18H, 3,5-$^t$*Bu*$_2$C$_6$H$_3$), 0.95 (s, 9H, CH$_2$$^t$*Bu*). $^{13}C\{^1H\}$ NMR ($CDCl_3$): $\delta$ 150.5, 146.2, 146.0, 140.7, 138.5, 138.3, 137.6, 127.2, 126.8, 122.9, 121.0, 120.8, 50.3, 42.6, 34.9, 31.8, 31.6, 29.5, 16.8.

**Bis[2-methyl-4-(3,5-di-*tert*-butylphenyl)-6-neopentyl-1*H*-inden-1-yl](dimethyl)silane**

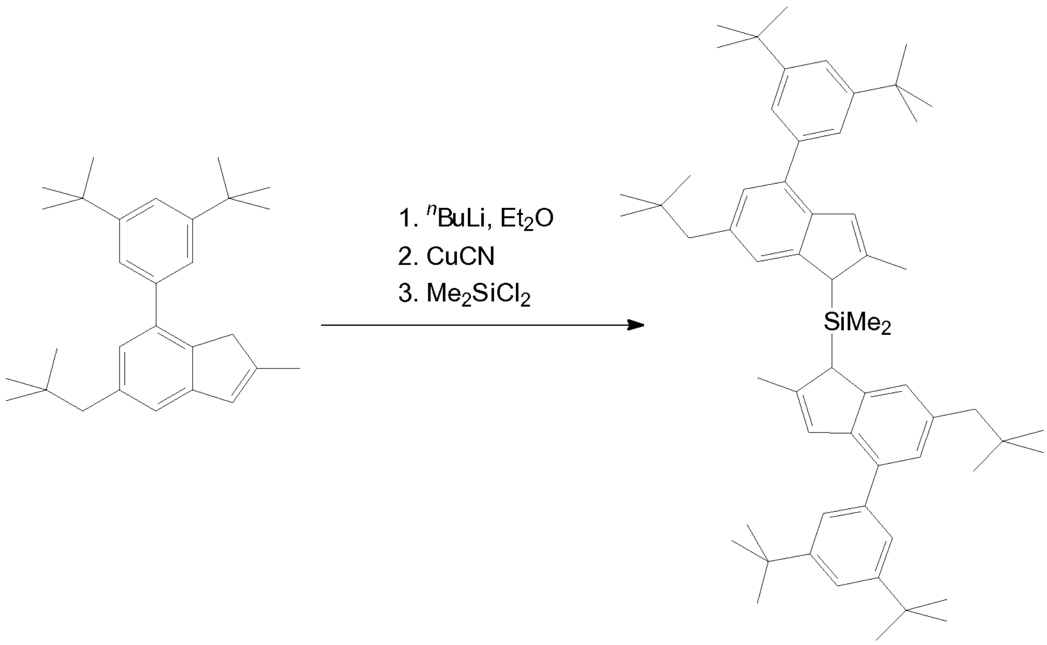


16.0 ml (40.0 mmol) of 2.5 M $^n$BuLi in hexanes was added in one portion to a solution of 15.8 g (40.6 mmol) of 2-methyl-7-(3,5-di-*tert*-butylphenyl)-5-neopentyl-1*H*-indene in 250 ml of ether at -50 °C. This mixture was stirred overnight at room temperature, then the resulting mixture was cooled to -40 °C, and 200 mg of CuCN was added. The obtained mixture was stirred for 30 min at -25 °C, then 2.58 g (20.0 mmol) of dichlorodimethylsilane was added in one portion. Then, this mixture was stirred overnight at ambient temperature, filtered through a pad of silica gel 60 (40-63 μm) which was additionally washed by 2x50 ml of dichloromethane. The combined filtrate was evaporated under vacuum, and the title product was isolated by flash-chromatography on silica gel 60 (40-63 μm; eluent: hexanes-dichloromethane = 10:1, vol.). This procedure gave 13.9 g (82%) of a yellowish glassy solid which on the evidence of NMR spectroscopy has >95% purity in a ca. 3 to 2 mixture of the stereoisomers.
$^1$H NMR ($CDCl_3$): $\delta$ 7.45-7.56 (m), 7.26 (m), 7.20 (m), 7.18 (m), 6.91 (m), 4.02 (s), 3.95 (s), 2.56-2.79 (m), 2.37 (s), 2.28 (s), 1.50 (s), 1.05 (s), 1.04 (s), -0.12 (s), - 0.13 (s), -0.19 (s).

***Rac*-dimethylsilanediylbis[$\eta^5$-4-(3,5-di-*tert*-butylphenyl)-6-neopentyl-2-methyl-1*H*-inden-1-yl]zirconium dichloride (I-mc4)**

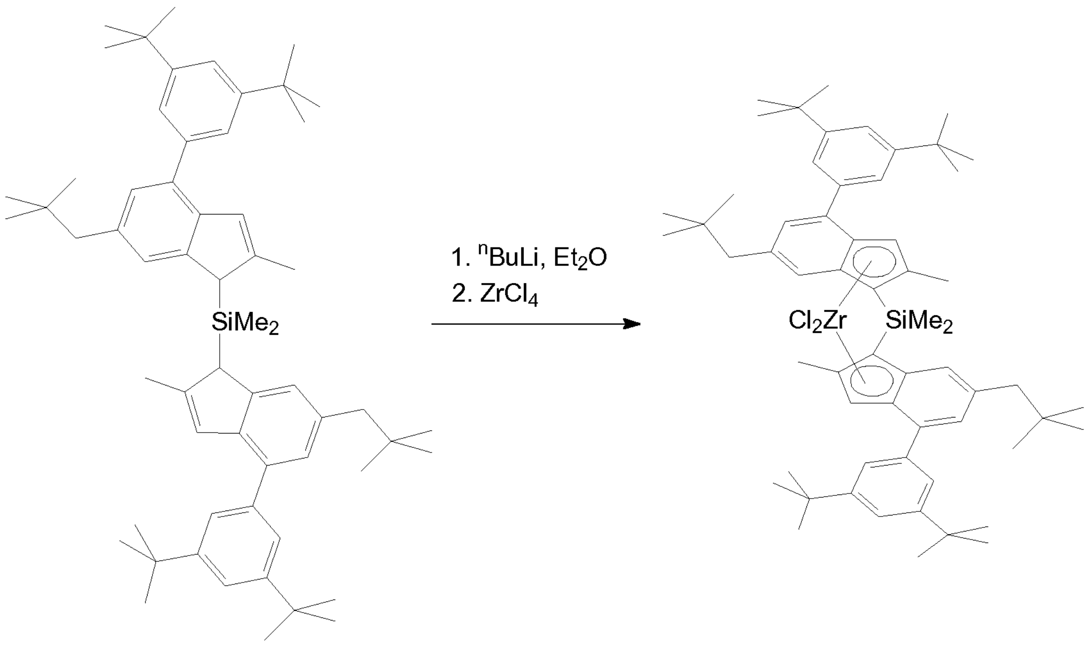


13.4 ml (33.5 mmol) of 2.5 M *n*BuLi in hexanes was added in one portion to a solution of 13.9 g (16.7 mmol) of bis[2-methyl-4-(3,5-di-*tert*-butylphenyl)-6-neopentyl-1*H*-inden-1-yl](dimethyl)silane (prepared above) in 200 ml of ether cooled to -30 °C. This mixture was stirred overnight at room temperature, then cooled to -50 °C, and 3.90 g (16.7 mmol) of $ZrCl_4$ was added. The reaction mixture was stirred for 24 h, and then the formed orange suspension was evaporated to dryness. The residue was poured into 200 ml of hot toluene. The obtained hot mixture was filtered through glass frit (G4). On the evidence of NMR spectroscopy the obtained filtrate included a ca. 1 to 1 mixture of *rac*- and *meso*-zirconocenes. This filtrate was evaporated to ca. 60 ml and then heated to dissolve the precipitate. Crystals precipitated from this solution for 1.5 h at room temperature were collected and dried in vacuum. This procedure gave 4.94 g of pure *meso*-zirconocene. The mother liquor was evaporated to ca. 40 ml. Crystals precipitated from this solution for 1.5 h at room temperature were collected and dried in vacuum. This procedure gave 1.53 g of pure *rac*-zirconocene. The obtained mother liquor was evaporated to dryness, and 35 ml of n-hexane was added. Crystals precipitated from this solution for 1.5 h at room temperature were collected and dried in vacuum. This procedure gave 3.31 g of pure *rac*-zirconocene. Again, the obtained mother liquor was evaporated to dryness, and 25 ml of n-hexane was added. Crystals precipitated from this solution overnight at room temperature were collected and dried in vacuum. This procedure gave 0.64 g of a ca. 6 to 1 mixture of *meso*- and *rac*-zirconocenes. Thus, the overall yield of *rac*- and *meso*-zirconocenes isolated in this synthesis was 10.4 g (63%).
*Rac*-zirconocene (I-mc4).
Anal. calc. for $C_{60}H_{82}Cl_2SiZr$: C, 72.54; H, 8.32. Found: C, 72.56; H, 8.44.
$^1H$ NMR ($CDCl_3$): $\delta$ 7.52 (m, 4H), 7.40 (m, 2H), 7.38 (m, 2H), 7.23 (m, 2H), 6.90 (m, 2H), 2.53 (d, *J* = 18.2 Hz, 2H), 2.50 (d, *J* = 18.2 Hz, 2H), 2.25 (s, 6H), 1.32 (2s, 42H), 1.00 (s, 18H). $^{13}C\{^1H\}$ NMR ($CDCl_3$): $\delta$ 150.87, 138.72, 138.44, 137.90, 134.85, 131.07, 130.37, 128.45, 124.31, 123.31, 122.42, 121.41, 82.48, 50.93, 35.02, 32.43, 31.52, 29.71, 18.86, 2.69.
*Meso*-zirconocene (*meso* I-mc4).
Anal. found: C, 72.67; H, 8.49.
$^1H$ NMR ($CDCl_3$): $\delta$ 7.46 (m, 4H), 7.38 (m, 2H), 7.32 (m, 2H), 6.96 (m, 2H), 6.76 (m, 2H), 2.44 (s, 6H), 2.42 (d, *J* = 13.2 Hz, 2H), 2.32 (d, *J* = 13.2 Hz, 2H), 1.44 (s, 3H), 1.33 (s, 36H), 1.22 (s, 3H), 0.96 (s, 18H). $^{13}C\{^1H\}$ NMR ($CDCl_3$): $\delta$ 150.60, 138.77, 137.39, 136.46, 135.93, 133.10, 129.61, 129.04, 125.02, 123.29, 121.22, 121.16, 83.88, 50.84, 34.97, 32.08, 31.49, 29.60, 18.77, 3.03, 2.50.

Table 1 summarises the 6-position substituents in the examples:

| **Metallocene** | $R^6$ |
|---|---|
| C-mc1 | H |
| C-mc2 | H * |
| I-mc1 | methyl |

(continued)

| Metallocene | $R^6$ |
|---|---|
| I-mc2 | ethyl |
| I-mc3 | *iso*-propyl |
| I-mc4 | *neo*-pentyl |
| * with 7-methoxy, | |

***Comparative catalysts***

**CE1 - *Catalyst synthesis with C-mc1***

Inside the glovebox, 80 µL of dry and degassed surfactant solution were mixed with 2 mL of MAO in a septum bottle and left to react overnight. The following day, 64.9 mg of the metallocene C-mc1 was dissolved with 4 mL of the MAO solution in another septum bottle and left to stir inside the glovebox.

After 60 minutes, 1 mL of the surfactant solution and the 4 mL of the MAO-metallocene solution were successively added into a 50mL emulsification glass reactor containing 40mL of PFC at -10 °C and equipped with an overhead stirrer (stirring speed = 600 rpm). Total amount of MAO is 5 mL (300 equivalents). A red-orange emulsion formed immediately and stirred during 15 minutes at 0 °C / 600rpm. Then the emulsion was transferred via a 2/4 teflon tube to 100mL of hot PFC at 90 °C, and stirred at 600rpm until the transfer is completed, then the speed was reduced to 300 rpm. After 15 minutes stirring, the oil bath was removed and the stirrer turned off. The catalyst was left to settle up on top of the PFC and after 45 minutes the solvent was siphoned off. The remaining red catalyst was dried during 2 hours at 50 °C over an argon flow. 0.45 g of a red free flowing powder was obtained.

**CE2** - ***Catalyst synthesis with C-mc2***

Inside the glovebox, 80 µL of dry and degassed surfactant solution were mixed with 2 mL of MAO in a septum bottle and left to react overnight. The following day, 69.4 mg of the metallocene *C-mc2* was dissolved with 4 mL of the MAO solution in another septum bottle and left to stir inside the glovebox.

After 60 minutes, 1 mL of the surfactant solution and the 4 mL of the MAO-metallocene solution were successively added into a 50mL emulsification glass reactor containing 40mL of PFC at -10 °C and equipped with an overhead stirrer (stirring speed = 600 rpm). Total amount of MAO is 5 mL (300 equivalents). A red-orange emulsion formed immediately and stirred during 15 minutes at 0 °C / 600rpm. Then the emulsion was transferred via a 2/4 teflon tube to 100mL of hot PFC at 90 °C, and stirred at 600rpm until the transfer is completed, then the speed was reduced to 300 rpm. After 15 minutes stirring, the oil bath was removed and the stirrer turned off. The catalyst was left to settle up on top of the PFC and after 45 minutes the solvent was siphoned off. The remaining red catalyst was dried during 2 hours at 50 °C over an argon flow. 0.74 g of a red free flowing powder was obtained.

**IE1 - *Catalyst synthesis with I-mc1***

Inside the glovebox, 80 µL of dry and degassed surfactant solution were mixed with 2 mL of MAO in a septum bottle and left to react overnight. The following day, 67.0 mg of the metallocene *I-mc1* was dissolved with 4 mL of the MAO solution in another septum bottle and left to stir inside the glovebox.

After 60 minutes, 1 mL of the surfactant solution and the 4 mL of the MAO-metallocene solution were successively added into a 50mL emulsification glass reactor containing 40mL of PFC at -10 °C and equipped with an overhead stirrer (stirring speed = 600 rpm). Total amount of MAO is 5 mL (300 equivalents). A red emulsion formed immediately and stirred during 15 minutes at 0 °C / 600rpm. Then the emulsion was transferred via a 2/4 teflon tube to 100mL of hot PFC at 90 °C, and stirred at 600rpm until the transfer is completed, then the speed was reduced to 300 rpm. After 15 minutes stirring, the oil bath was removed and the stirrer turned off. The catalyst was left to settle up on top of the PFC and after 35 minutes the solvent was siphoned off. The remaining red catalyst was dried during 2 hours at 50 °C over an argon flow. 0.50 g of a red free flowing powder was obtained.

**IE2** - ***Catalyst synthesis with I-mc2***

Inside the glovebox, 80 µL of dry and degassed surfactant solution were mixed with 2 mL of MAO in a septum bottle and left to react overnight. The following day, 69.1 mg of the metallocene *I-mc2* was dissolved with 4 mL of the

MAO solution in another septum bottle and left to stir inside the glovebox.

After 60 minutes, 1 mL of the surfactant solution and the 4 mL of the MAO-metallocene solution were successively added into a 50mL emulsification glass reactor containing 40mL of PFC at -10 °C and equipped with an overhead stirrer (stirring speed = 600 rpm). Total amount of MAO is 5 mL (300 equivalents). A red-orange emulsion formed immediately and stirred during 15 minutes at 0 °C / 600rpm. Then the emulsion was transferred via a 2/4 teflon tube to 100mL of hot PFC at 90 °C, and stirred at 600 rpm until the transfer is completed, then the speed was reduced to 300 rpm. After 15 minutes stirring, the oil bath was removed and the stirrer turned off. The catalyst was left to settle up on top of the PFC and after 35 minutes the solvent was siphoned off. The remaining red catalyst was dried during 2 hours at 50 °C over an argon flow. 0.51 g of a red free flowing powder was obtained.

**IE3 - *Catalyst synthesis with I-mc3***

Inside the glovebox, 80 μL of dry and degassed surfactant solution were mixed with 2 mL of MAO in a septum bottle and left to react overnight. The following day, 71.2 mg of the metallocene I-mc3 was dissolved with 4 mL of the MAO solution in another septum bottle and left to stir inside the glovebox.

After 60 minutes, 1 mL of the surfactant solution and the 4 mL of the MAO-metallocene solution were successively added into a 50mL emulsification glass reactor containing 40mL of PFC at -10 °C and equipped with an overhead stirrer (stirring speed = 600 rpm). Total amount of MAO is 5 mL (300 equivalents). A red-orange emulsion formed immediately and stirred during 15 minutes at 0 °C / 600rpm. Then the emulsion was transferred via a 2/4 teflon tube to 100mL of hot PFC at 90 °C, and stirred at 600rpm until the transfer is completed, then the speed was reduced to 300 rpm. After 15 minutes stirring, the oil bath was removed and the stirrer turned off. The catalyst was left to settle up on top of the PFC and after 35 minutes the solvent was siphoned off. The remaining red catalyst was dried during 2 hours at 50 °C over an argon flow. 0.60 g of a red free flowing powder was obtained.

**IE4 - *Catalyst synthesis with I-mc4***

Inside the glovebox, 80 μL of dry and degassed surfactant solution were mixed with 2 mL of MAO in a septum bottle and left to react overnight. The following day, 76.8 mg of the metallocene I-mc4 was dissolved with 4 mL of the MAO solution in another septum bottle and left to stir inside the glovebox.

After 60 minutes, 1 mL of the surfactant solution and the 4 mL of the MAO-metallocene solution were successively added into a 50mL emulsification glass reactor containing 40mL of PFC at -10 °C and equipped with an overhead stirrer (stirring speed = 600 rpm). Total amount of MAO is 5 mL (300 equivalents). A red-orange emulsion formed immediately and stirred during 15 minutes at 0 °C / 600rpm. Then the emulsion was transferred via a 2/4 teflon tube to 100mL of hot PFC at 90 °C, and stirred at 600rpm until the transfer is completed, then the speed was reduced to 300 rpm. After 15 minutes stirring, the oil bath was removed and the stirrer turned off. The catalyst was left to settle up on top of the PFC and after 35 minutes the solvent was siphoned off. The remaining red catalyst was dried during 2 hours at 50 °C over an argon flow. 0.59 g of a red free flowing powder was obtained.

**Table 2:** Catalyst composition (ICP)

| Catalyst | Al/Zr (molar) | Zr content (wt%) | MC content (wt%) |
|---|---|---|---|
| CE1 | 280 | 0.36 | 2.71 |
| CE2 | 276 | 0.37 | 2.90 |
| IE1 | 316 | 0.38 | 3.67 |
| IE2 | 280 | 0.43 | 4.29 |
| IE3 | 323 | 0.33 | 3.39 |
| IE4 | 279 | 0.38 | 4.14 |

**Polymerisations**

The polymerisations were performed in a 5 L reactor. 200 μl of triethylaluminum was fed as a scavenger in 5 mL of dry and degassed pentane. The desired amount of hydrogen was then loaded (measured in mmol) and 1100 g of liquid propylene was fed into the reactor.

Procedure A: The temperature was set to 30 °C. The desired amount of catalyst (3 to 30 mg) in 5mL of PFC is flushed into the reactor with a nitrogen overpressure. The temperature is then raised to 70 °C over a period of 15 minutes. The polymerisation is stopped after 30 minutes by venting the reactor and flushing with nitrogen before the polymer is collected. (Polymerisations CP1-CP3)

Procedure B: The temperature was set to 20 °C. The desired amount of catalyst (3 to 30 mg) in 5mL of PFC is flushed into the reactor with a nitrogen overpressure. After 5 minutes of the temperature is raised to 70 °C over a period of 15 minutes. The polymerisation is stopped after 60 minutes by venting the reactor and flushing with nitrogen before the polymer is collected. (Polymerisations CP4-CP6, IP1-IP12)

The catalyst activity was calculated on the basis of the 30 (or 60) minutes period according to the following formula:

$$\textbf{Act Cat:}\quad \text{Catalyst Activity } (kg/(g(cat) * h)) = \frac{\text{amount of polymer produced (kg)}}{\text{catalyst loading (g)} \times \text{polymerisation time (h)}}$$

Act

$$\textbf{Met:}\quad \textbf{Catalyst Metal Activity } (\mathbf{kg/(g(cat) * h)}) = \frac{\textbf{amount of polymer produced (kg)}}{\textbf{catalyst metal loading (g)} \times \textbf{polymerisation time (h)}}$$

**Table 3:** Polymerization results with CE1, CE2, IE1-IE4

| Catalyst | Ex | Cat. (mg) | $H_2$ mmol | Pol. Yield, g | Act cat kg/gcat/h | Act Metal kg/gmetal/h | $MFR_2$ g/10min | $M_w$ kg/mol | $M_w/M_n$ | $T_m$ (°C) | $T_c$ (°C) |
|---|---|---|---|---|---|---|---|---|---|---|---|
| CE2 | CP1 | 18.3 | 1 | 50 | 5.5 | 1480 | 1.9 | 347 | 2.0 | 159.4 | 113.5 |
| | CP2 | 16.1 | 6 | 120 | 15.0 | 4042 | 19.0 | 190 | 2.1 | 156.2 | 113.8 |
| | CP3 | 13.6 | 15 | 114 | 16.8 | 4531 | 120 | 106 | 2.2 | 156.6 | 116.5 |
| CE1 | CP4 | 11.1 | 1 | 99 | 9.0 | 2487 | 9.4** | 574.0 | 2.5 | 156.5 | 111.9 |
| | CP5 | 5.5 | 6 | 100 | 18.2 | 5051 | 1.9 | 302.0 | 2.4 | 157.5 | 112.9 |
| | CP6 | 10.7 | 15 | 218 | 20.4 | 5659 | 21.0 | 179.0 | 2.4 | 156.6 | 112.7 |
| IE1 | IP1 | 14.9 | 1 | 406 | 27.2 | 7165 | 30.0 ** | 495.0 | 2.6 | 155.6 | 112.7 |
| | IP2 | 12.5 | 6 | 547 | 43.7 | 11509 | 3.2 | 289.0 | 2.5 | 156.7 | 111.4 |
| | IP3 | 8.5 | 15 | 466 | 54.8 | 14421 | 37.3 | 157.0 | 2.5 | 156.9 | 114.5 |
| IE2 | IP4 | 10.0 | 1 | 245 | 24.5 | 5688 | 32.6** | 611.0 | 2.4 | 156.4 | 109.7 |
| | IP5 | 10.0 | 6 | 398 | 39.8 | 9244 | 2.9 | 329.0 | 2.3 | 157.5 | 111.6 |
| | IP6 | 8.2 | 15 | 437 | 53.3 | 12391 | 26.9 | 175.0 | 2.8 | 156.3 | 113.4 |
| IE3 | IP7 | 10.8 | 1 | 142 | 13.2 | 3990 | 20.1** | 640.0 | 2.6 | 155.6 | 109.2 |
| | IP8 | 12.6 | 6 | 333 | 26.4 | 7997 | 2.0 | 324.0 | 2.5 | 156.3 | 110.5 |
| | IP9 | 9.2 | 15 | 311 | 33.8 | 10231 | 24.1 | 189.0 | 2.6 | 155.0 | 111.5 |

| Catalyst | Ex | Cat. (mg) | $H_2$ mmol | Pol. Yield, g | Act cat kg/gcat/h | Act Metal kg/gmetal/h | $MFR_2$ g/10min | $M_w$ kg/mol | $M_w/M_n$ | $T_m$ (°C) | $T_c$ (°C) |
|---|---|---|---|---|---|---|---|---|---|---|---|
| IE4 | IP10 | 14.5 | 1 | 265 | 18.3 | 4809 | 25.0** | 560.0 | 2.4 | 155.3 | 111.6 |
| | IP11 | 12.7 | 6 | 423 | 33.3 | 8771 | 120.0** | 344.0 | 2.4 | 156.0 | 111.0 |
| | IP12 | 10.8 | 15 | 468 | 43.4 | 11408 | 16.0 | 186.0 | 2.6 | 155.6 | 112.7 |

* Procedure A for (Polymerisations CP1-CP3) and Procedure B for (Polymerisations CP4-CP6, IP1-IP12)

** $MFR_{21}$ (g/10min)

It can be seen from table 3 above that using catalysts of the invention the activity is clearly higher using the same amount of $H_2$ in the polymerisation when inventive catalysts are compared to the results obtained using comparative catalysts. The polymers still have higher Mw (lower MFR) and possess high melting points.

Table 4 summarises the important results herein succinctly.

Table 4

| **MC/ catalyst** | $R^6$ | Polymerisation example | **Activity** (kg-PP/g-cat/h) | **Activity** (kg-PP/g-Zr/h) | **Mw** (kg/mol) | $M_w/M_n$ | **Tm** (°C) |
|---|---|---|---|---|---|---|---|
| C-mc1/ CE1 | - | CP5 | 18.2 | 5051 | 302 | 2.4 | 157.5 |
| C-mc2 CE2 | - * | CP1 | 5.5 | 1480 | 347 | 2.0 | 159.4 |
| I-mc1/ IE1 | methyl | IP2 | 43.7 | 11509 | 289 | 2.5 | 156.7 |
| I-mc2/ IE2 | ethyl | IP5 | 39.8 | 9244 | 329 | 2.3 | 157.5 |
| I-mc3/ IE3 | *iso*-propyl | IP8 | 26.4 | 7997 | 324 | 2.5 | 156.3 |
| I-mc4/ IE4 | *neo*-pentyl | IP11 | 33.3 | 8771 | 344 | 2.4 | 156.0 |
| * with 7-methoxy | | | | | | | |

## Claims

1. A process for the preparation of a compound of formula (A'):

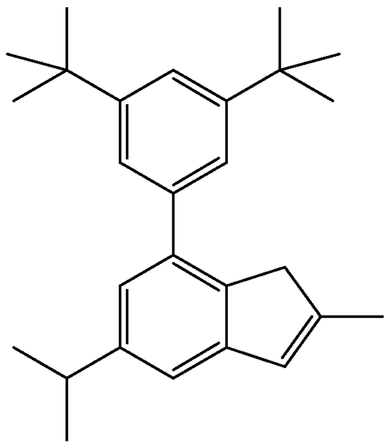

(A')

comprising at least the steps of:

a) reacting a compound of formula (B")

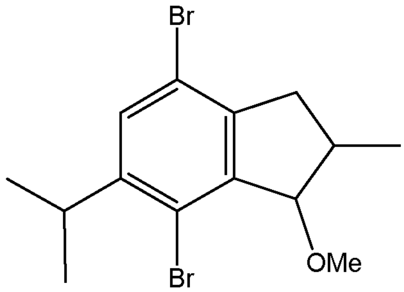

(B'')

with nBuLi to obtain a compound of formula (C")

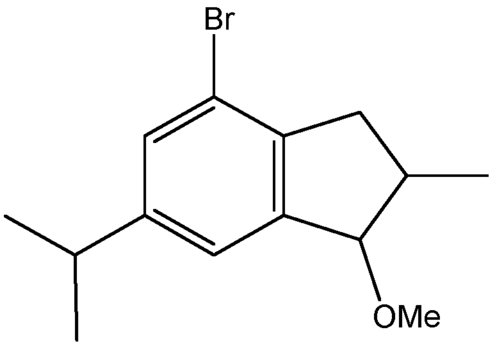

(C'')

b) reacting the compound of formula (C") with a compound (D')

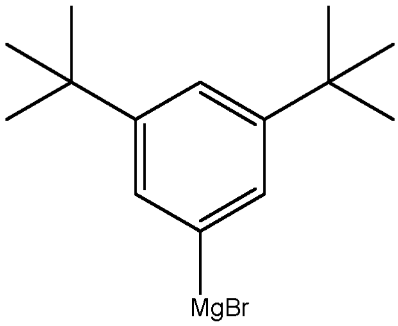

(D')

in the presence of $NiCl_2(PPh_3)$IPr wherein IPr represents 1,3-bis(2,6-diisopropylphenyl)imidazolidin-2-ylidene to form compound (E");

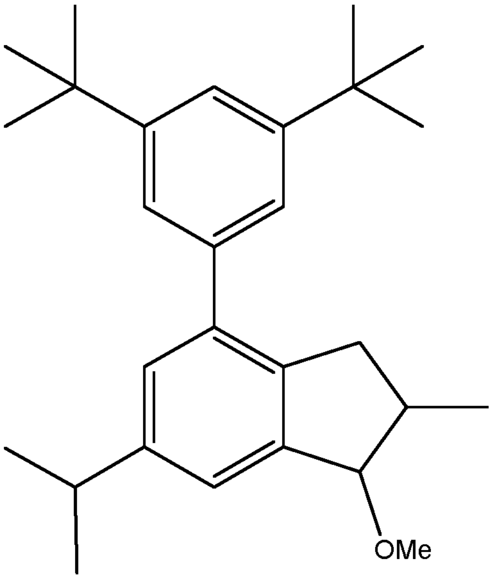

(E'')

and reducing compound (E") in the presence of TsOH.

**Patentansprüche**

**1.** Verfahren zur Herstellung einer Verbindung der Formel (A'):

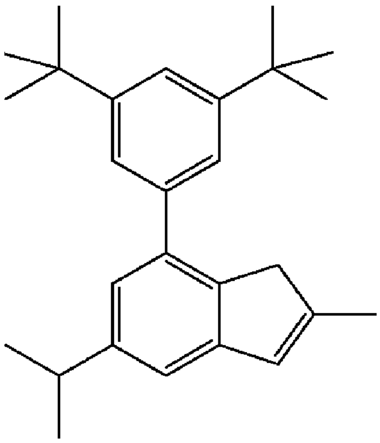

(A')

umfassend mindestens die Schritte des:

a) Umsetzens einer Verbindung der Formel (B")

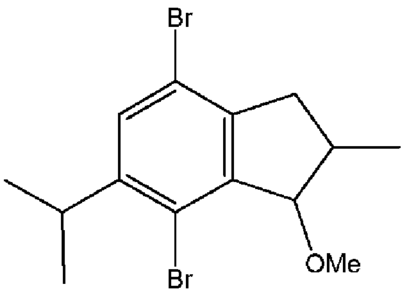

(B'')

mit nBuLi, um eine Verbindung der Formel (C") zu erhalten

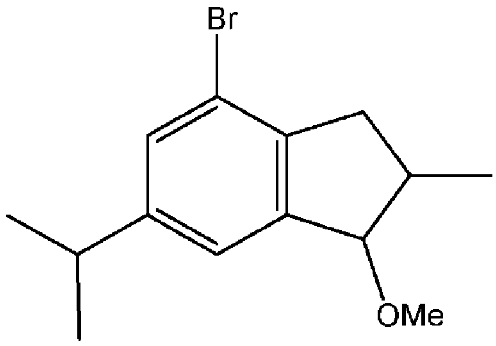

(C")

b) Umsetzens der Verbindung der Formel (C") mit einer Verbindung (D')

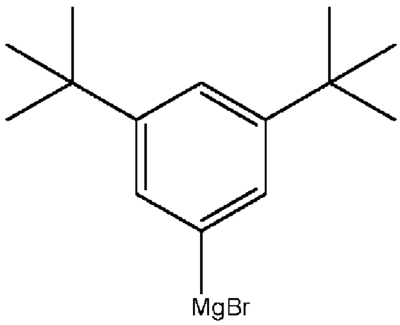

(D')

in Gegenwart von $NiCl_2(PPh_3)$IPr, wobei IPr 1,3-Bis(2,6-diisopropylphenyl)imidazolidin-2-yliden darstellt, um Verbindung (E") zu bilden;

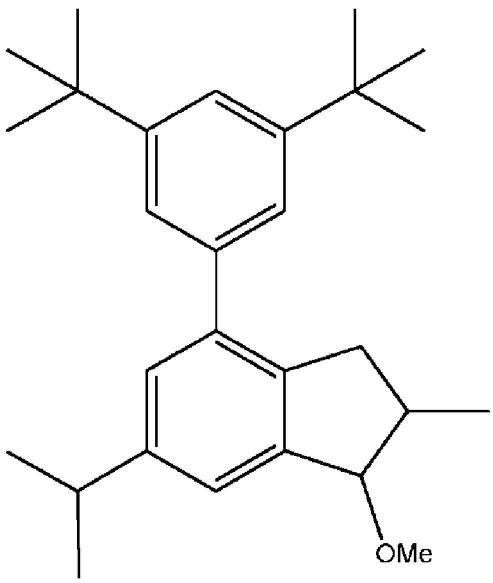

(E")

und Reduzierens der Verbindung (E") in Gegenwart von TsOH.

## Revendications

**1.** Procédé de préparation d'un composé de formule (A') :

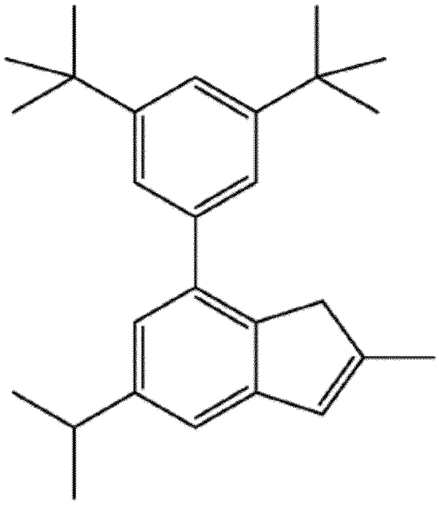

(A')

comprenant au moins les étapes consistant à :

a) faire réagir un composé de formule (B")

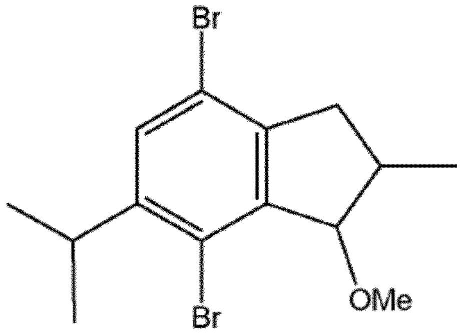

(B'')

avec nBuLi pour obtenir un composé de formule (C")

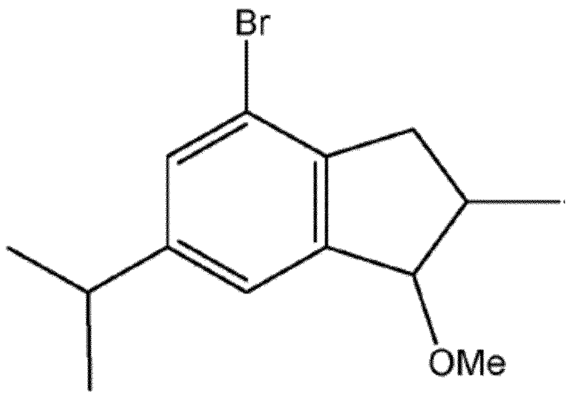

(C'')

b) faire réagir le composé de formule (C") avec un composé (D')

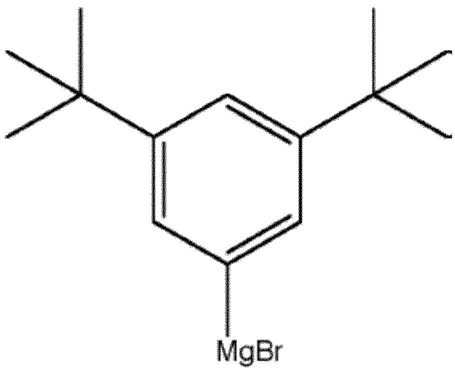

(D')

en présence de $NiCl_2(PPh_3)$IPr dans lequel IPr représente un 1,3-bis(2,6-diisopropylphényl)imidazolidin-2-ylidène pour former un composé (E") ;

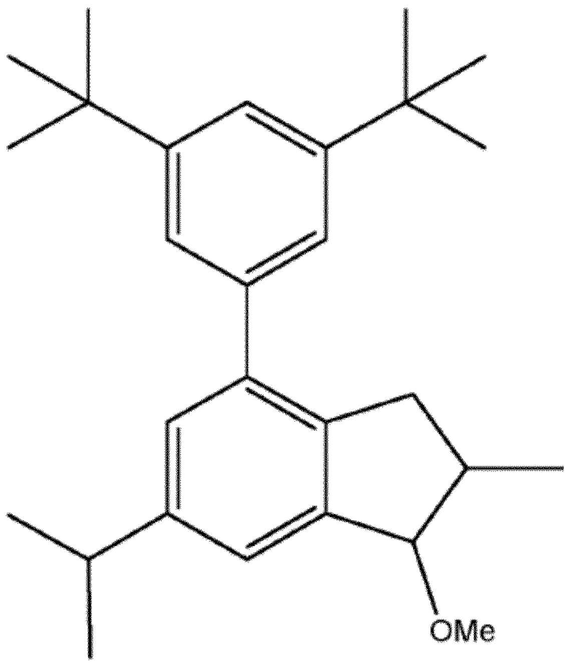

(E'') ;

et réduire le composé (E") en présence de TsOH.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- EP 537686 A
- EP 1070729 A
- US 7405261 B
- WO 2009054831 A
- WO 03051934 A
- WO 0202576 A
- WO 0202575 A
- EP 2532687 A
- EP 12199251


### Non-patent literature cited in the description


- *Deng Macromolecules,* 1996, vol. 29, 6371
- *Spaleck Organometallics,* 1994, vol. 13, 954
- **J. EWEN et al.** *Macromol. Rapid Commun.,* 1998, vol. 19, 71
- **HINTERMANN, L.** *Beilstein J. Org. Chem.,* 2007, vol. 3, 1
- **MATSUBARA, K. ; UENO, K. ; SHIBATA, Y.** *Organometallics,* 2006, vol. 25, 3422
- **IZMER, V.V. ; LEBEDEV, A.Y. ; NIKULIN, M.V. ; RYABOV, A.N. ; ASACHENKO, A.F. ; LYGIN, A.V. ; SOROKIN, D.A. ; VOSKOBOYNIKOV, A.Z.** *Organometallics,* 2006, vol. 25, 1217
- **BUSICO, V. ; CIPULLO, R.** *Prog. Polym. Sci.,* 2001, vol. 26, 443
- **BUSICO, V. ; CIPULLO, R. ; MONACO, G. ; VACATELLO, M. ; SEGRE, A.L.** *Macromolecules,* 1997, vol. 30, 6251
- **ZHOU, Z. ; KUEMMERLE, R. ; QIU, X. ; REDWINE, D. ; CONG, R. ; TAHA, A. ; BAUGH, D ; WINNIFORD, B.** *J. Mag. Reson.,* 2007, vol. 187, 225
- **BUSICO, V. ; CARBONNIERE, P. ; CIPULLO, R. ; PELLECCHIA, R. ; SEVERN, J. ; TALARICO, G.** *Macromol. Rapid Commun.,* 2007, vol. 28, 11289
- **RESCONI, L. ; CAVALLO, L. ; FAIT, A. ; PIEMONTESI, F.** *Chem. Rev.,* 2000, vol. 100, 1253
- *CHEMICAL ABSTRACTS,* 65605-70-1
- *CHEMICAL ABSTRACTS,* 335-27-3
- **MATSUBARA, K. ; UENO, K. ; SHIBATA, Y.** *Organometallics,* 2006, vol. 25, 3422
- **IZMER, V.V. ; LEBEDEV, A.Y. ; NIKULIN, M.V. ; RYABOV, A.N. ; ASACHENKO, A.F. ; LYGIN, A.V. ; SOROKIN, D.A. ; VOSKOBOYNIKOV, A.Z.** *Organometallics,* 2006, vol. 25, 1217
- **ZHOU, Z. ; KUEMMERLE, R. ; QIU, X. ; REDWINE, D. ; CONG, R. ; TAHA, A. ; BAUGH, D. ; WINNIFORD, B.** *J. Mag. Reson.,* 2007, vol. 187, 225